# EUROPEAN PATENT APPLICATION

(11) **EP 4 796 645 A2**
(43) Date of publication of application: **26.08.2026**
(21) Application number: 26188531.3
(22) Date of filing: 08.02.2022
(51) Int. Cl.: C12Q 1/686

(54) **METHODS FOR MANUFACTURING A SYNTHETIC TEMPLATE**

(30) Priority: 08.02.2021 US 202163147173 P
(62) Document number(s) of the earlier application(s) in accordance with Art. 76 EPC: 22750562.5 / 4 288 210
(71) Applicant: Nutcracker Therapeutics, Inc., Emeryville CA 94608 (US)
(72) Inventor: DEUTSCH, Samuel, Emeryville, CA 94608 (US); NATH, Sangeeta, Emeryville, CA 94608 (US); WEN, Ximiao, Emeryville, CA 94608 (US); CAI, Wei, Emeryville, CA 94608 (US); SANII, Babak, Emeryville, CA 94608 (US); ELDRIDGE, Benjamin, Emeryville, CA 94608 (US)
(74) Representative: Simmons & Simmons

(57) **Abstract**

Provided herein is a method of making, comprising transporting reagents to a reactor in a microfluidic path device, wherein the reagents include a synthetic gene of interest, a polymerase, a buffer, a first primer having a first region specific to the synthetic gene of interest, and a second primer, wherein the second primer comprises a poly-T sequence of ≥ 150 base pairs (bp) or a poly-A sequence of ≥ 150 bp and a second region specific to the synthetic gene of interest; controlling a temperature of the first reactor to perform a polymerase chain reaction within the microfluidic path device to amplify the synthetic gene of interest using the first primer and the second primer to form a synthetic product including the poly-A sequence of ≥ 150 bp; and transporting the synthetic product out of the first reactor, wherein the synthetic product comprises a synthetic DNA template for in vitro transcription.

## Description

### CLAIM OF PRIORITY

This patent application claims priority to U.S. provisional patent application no. 63/147,173, titled "METHODS FOR MANUFACTURING A SYNTHETIC TEMPLATE" and filed on Feb. 8, 2021, which is herein incorporated by reference in its entirety.

### INCORPORATION BY REFERENCE

All publications and patent applications mentioned in this specification are herein incorporated by reference in their entirety to the same extent as if each individual publication or patent application was specifically and individually indicated to be incorporated by reference.

### BACKGROUND

Currently available technologies for manufacturing and formulating polynucleotide therapeutics, particularly mRNA therapeutics, often expose the products to contamination and degradation. Currently available centralized production can be too costly, too slow, and susceptible to contamination for use in therapeutic formulations possibly including multiple polynucleotide species. Development of scalable polynucleotide manufacturing, production of single patient dosages, elimination of touchpoints to limit contamination, input and process tracking for meeting clinical manufacturing requirements, and use in Point-of-Care operations can advance the use of these promising therapeutic modalities. Microfluidic instrumentation and processes can provide major advantages against these goals.

### SUMMARY OF THE DISCLOSURE

Described herein are methods and apparatuses (e.g., systems) that for making a variety of therapeutics that may address the needs mentioned above.

Described herein are apparatuses and methods useful for making a wide variety of vaccines and therapeutics. For example, described herein are methods and apparatuses (e.g., systems, devices, etc.) for making personalized therapeutics, including vaccines. In one, nonlimiting example, the methods and apparatuses described herein may be used to produce a therapeutic mRNA vaccine against a cancer-specific antigen active in Cutaneous T-Cell Lymphoma.

This disclosure is related to methods and systems for rapid, high-yield manufacturing of polynucleotide-based therapeutics, and may relate specifically to automated manufacture of therapeutic mRNAs, including vaccines, which may be performed rapidly and efficiently. Such therapeutics may take into account patient specific information and may be produced on-demand and completely or partially at the point-of-care (e.g., hospital, clinic, etc.). Thus, described herein are automated, high-yield manufacturing methods for mRNA therapeutics, optionally deployed at point-of-care.

The methods and apparatuses described herein may include synthetically forming the template for mRNA formation without the use of bacteria. In some examples, these methods and apparatuses may be used without the use of any bacterial components. These methods and apparatuses may use a polymerase, as part of a polymerase chain reaction (PCR) to synthesize the mRNA therapeutic, including mRNA therapeutics having single polynucleotide repeats (e.g., poly-A) tails of greater than about 100 bp - e.g., greater than about 150 bp, greater than about 200 bp, or higher.

For example, described herein are methods of forming (e.g., manufacturing, making, synthesizing, etc.) a therapeutic polynucleotide using a system comprising a plurality of fluid depots configured to be secured in sealed fluid communication with one or more microfluidic path devices, the method comprising: transporting reagents between one or more fluid depots of the plurality of fluid depots and a plurality of reactors on the one or more microfluidic path devices in a sealed and closed fluidic path that is protected from atmospheric contact to perform the operations of: forming a synthetic template, performing *in vitro* transcription from the template to produce a therapeutic polynucleotide, and purifying the therapeutic polynucleotide. These methods may include forming the synthetic template by PCR.

A method of manufacturing a therapeutic mRNA using a system comprising a plurality of fluid depots configured to be secured in sealed fluid communication with one or more microfluidic path plate devices, wherein the one or more microfluidic path plate devices comprise a plurality of reactors, may include: delivering a template precursor material from one or more fluid depots to a first one or more reactor regions of the plurality of reactors and processing the template precursor material (e.g. by PCR) to prepare a template from the template precursor material; transferring the template to a second one or more reactor regions of the plurality of reactors and processing the template by *in vitro* transcription to form a therapeutic mRNA; and transferring the therapeutic mRNA to a third one or more reactor regions of the plurality of reactors and purifying the therapeutic mRNA by one-dimensional (1D) or two-dimensional (2D) purification within the third one or more reactor regions; wherein all of the method steps are performed without exposing the template and therapeutic mRNA to atmospheric contact.

A method of manufacturing a therapeutic mRNA using a system comprising a plurality of fluid depots in sealed fluid communication with one or more microfluidic path plate devices, wherein the one or more microfluidic path plate devices comprise a plurality of reactors, may include: delivering, using fluid power, a template precursor material from one or more fluid depots to a first one or more reactor regions of the plurality of reactors and processing the template precursor material (e.g., by PCR) to prepare a template from the template precursor material; transferring, using fluid power, the template to a second one or more reactor regions of the plurality of reactors and processing the template by *in vitro* transcription to form a therapeutic mRNA; transferring, using fluid power, the therapeutic mRNA to a third one or more reactor regions of the plurality of reactors and purifying the therapeutic mRNA by two-dimensional (2D) purification within the third one or more reactor regions; transferring, using fluid power, the therapeutic mRNA to a fourth one or more reactor regions of the plurality of reactors and encapsulating the therapeutic mRNA with a delivery vehicle to form a therapeutic mRNA composition; and concentrating, using fluid power, the therapeutic mRNA composition in a fifth one or more fluid depots, wherein all of the method steps are performed without exposing the template and the therapeutic mRNA to atmospheric contact.

Described herein are methods of making (e.g., methods of making a synthetic product comprising a synthetic DNA template suitable for in vitro transcription), the method comprising: transporting reagents to a first reactor in a microfluidic path device, wherein the reagents include a synthetic gene of interest, a polymerase, a buffer, a first primer having a first region specific to the synthetic gene of interest, and a second primer, wherein the second primer comprises a poly-T sequence of 150 base pairs (bp) or longer or a poly-A sequence of 150 bp or longer and a second region that is specific to the synthetic gene of interest; controlling a temperature of the first reactor of the microfluidic path device to perform a polymerase chain reaction (PCR) within the microfluidic path device to amplify the synthetic gene of interest using the first primer and the second primer to form a synthetic product including the poly-A sequence of 150 bp or longer; and transporting the synthetic product out of the first reactor, wherein the synthetic product comprises a synthetic DNA template suitable for *in vitro* transcription.

For example, the methods described herein may include automated methods of making a synthetic DNA template for *in vitro* transcription that include: transporting reagents to a first reactor in a microfluidic path device, wherein the reagents include a synthetic gene of interest, a polymerase, a buffer, a first primer having a first region specific to the synthetic gene of interest, and a second primer, wherein the second primer comprises a poly-T sequence of 150 bp or longer or a poly-T sequence of 150 bp or longer and a second region that is specific to the synthetic gene of interest; controlling the temperature of the first reactor of the microfluidic path device to perform a polymerase chain reaction (PCR) within the microfluidic path device to amplify the synthetic gene of interest using the first primer and the second primer to form a synthetic product including the poly-A sequence of 150 bp or longer; and transporting the synthetic product out of the first reactor, wherein the synthetic product comprises the synthetic DNA template.

Any of the methods for making synthetic DNA template as described herein may include one-dimensional (1D) or two-dimensional (2D) purification of the template material on the microfluidic path device (e.g., chip) to remove impurities. For example, any of these methods may include performing purification of the synthesized DNA template in one or more dedicated purification chambers within the microfluidic path device. 1D or 2D purification may include performing a size selection to remove smaller (e.g., 500 bp or less, 400 bp or less, 300 bp or less, 200 bp or less, 100 bp or less, etc.) polynucleotides and/or nucleotides from the solution containing the synthesized DNA template. For example, 2D purification may include passing the synthesized DNA template into a chamber including charge-switch beads followed by Ampure^{™} beads (e.g., AMPure XP, New England Biolabs, U.S.). One-dimensional purification may include passing the synthesized DNA template in solution into a chamber including Ampure^{™} beads.

The first primer may be a forward primer, e.g., including a 5' end that hybridizes to a first region of a polynucleotide that is complimentary to the synthetic gene of interest, or a reverse primer, e.g., including a 3' end that is complimentary to a first region of the synthetic gene of interest. The second primer may be a reverse primer, e.g., including a poly-T sequence of 150 bp or longer and a 5' region that is complimentary to a 5' end of the synthetic gene of interest, or a forward primer, e.g., including a poly-A sequence of 150 bp or longer and a 3' region that that hybridizes to a second region of a polynucleotide that is complimentary to the synthetic gene of interest. In any of the methods described herein, if the first primer is a forward primer, the second primer is a reverse primer; conversely, if the first primer is a reverse primer, the second primer is a forward primer.

For example, the first primer may include an end that is complementary to or includes the sequence of a 3' end region of the synthetic gene of interest. The second region of the second primer may comprise an end region that includes or that is complimentary to a 5' end region of the synthetic gene of interest. The first primer may include a promoter region, such as (but not limited to) a T7 promoter region.

The first primer and the second primer are typically asymmetric in size; the second primer (including the 150 or longer length of poly-A or poly-T) may be about four or more fold the length (e.g., about five or more fold, about six or more fold, about seven or more fold, about eight or more fold, etc.) of the first primer.

Controlling the temperature to amplify the synthetic gene of interest by PCR may include generating about 0.5 µM or more (e.g., about 1 µM or more, about 2 µM or more, about 3 µM or more, about 4 µM or more, about 5 µM or more, about 7.5 µM or more, about 10 µM or more, about 50 µM or more, about 100 µM or more, etc.) of an amplified DNA template. For example, the methods and apparatuses described herein may generate between about 1 and about 200 µg (e.g., between about 1 and about 100 µg, between about 10 and about 100 µg, between about 10 and about 50 µg, between about 10 and about 30 µg, etc.), in about 1 and about 200 µL of volume (e.g., about 1 and about 100 µL, about 10 and about 100 µL, about 10 and about 50 µL, about 10 and about 25 µL, about 10 and about 20 µL, etc.). For example, the methods and apparatuses described herein may yield between about 10 and about 30 µg of the synthetic DNA template in about 10 and about 20 µL of elution volume.

In any of these methods the synthetic DNA template may be free of bacterial DNA and free of endotoxin. For example, any of these methods may include treating the synthetic gene of interest and/or the synthetic product with a methylation sensitive restriction enzyme to remove any bacterial DNA.

The temperature to amplify the synthetic gene of interest by PCR may be controlled in order to amplify between about 20 and about 25 annealing and extension cycles.

Transporting the second primer comprises transportation the second primer comprising a poly-T sequency of 200 bp or longer (if the second primer is a reverse primer) or a poly-A sequence of 200 bp or longer (if the second primer is a forward primer).

The end that is complementary to or includes the sequence of a 3' end region of the synthetic gene of interest is complimentary to or includes between about 20 to about 40 bp (e.g., between about 25 and about 40, between about 25 and about 30, between about 25 and about 35, between about 20 and about 35, between about 20 and about 30, etc.) of the synthetic gene of interest. The end region that includes or that is complimentary to the 5' end region of the synthetic gene of interest may be between about 20 and about 40 bp long (e.g., between about 25 and about 40, between about 25 and about 30, between about 25 and about 35, between about 20 and about 35, between about 20 and about 30, etc.).

Any of these methods may include receiving, e.g., in a controller, optical sensor data from one or more sensors of the closed-path system, wherein the controller controls the operation of the microfluidic path device (e.g., a closed-path system) using at least the optical sensor data. These methods may further include purifying, in the microfluidic device, the synthetic product. For example, purifying may include removing polynucleotides below a minimum length threshold. The minimum length threshold may be below about 800 bp (e.g., below about 700 bp, below about 600 bp, below about 500 bp, below about 450 bp, below about 400 bp, below about 350 bp, etc.).

Transporting may include using one or more fluid power circuits to move the reagents between a plurality of fluid depots into and the microfluidic path device or within the microfluidic path device. Thus, transporting may include using one or more fluid power circuits to move the reagents between a plurality of fluid depots into and the microfluidic path device or within the microfluidic path device.

Any of the methods described herein may include performing an *in vitro* transcription using the synthetic DNA template to form a therapeutic polynucleotide. In some examples the therapeutic polynucleotide may be at least partially encapsulated with a delivery vehicle.

Any of these methods may include determining the yield of the synthetic product using a UV yield detection window (of a UV yield detection chamber) on the microfluidic path device. Yield information may be estimated spectrophotometrically while the sample remains in the device. The yield information may be used by a controller (e.g., of a system). For example, any of these methods may include automatically diluting the synthetic product in the microfluidic path device based on the determined yield.

In any of the method described herein controlling the temperature may comprise added additional enzyme during the polymerase chain reaction within the microfluidic path device.

For example, a method of making (e.g., of making a synthetic product comprising a DNA template) may include: transporting reagents to a first reactor in a microfluidic path device, wherein the reagents include a synthetic gene of interest, a polymerase, a buffer, a forward primer including a 5' end that hybridizes to a first region of a polynucleotide that is complimentary to the synthetic gene of interest, and a reverse primer, wherein the reverse primer comprises a poly-T sequence of 150 bp or longer and a 5' region that is complimentary to a 5' end of the synthetic gene of interest; controlling a temperature of the first reactor of the microfluidic path device to perform a polymerase chain reaction (PCR) within the microfluidic path device to amplify the synthetic gene of interest using the forward primer and the reverse primer to form a synthetic product including a poly-A sequence of 150 bp or longer; and transporting the synthetic product out of the first reactor, wherein the synthetic product comprises the synthetic DNA template.

An automated method of making a synthetic DNA template for *in vitro* transcription may include: transporting reagents to a first reactor in a microfluidic path device, wherein the reagents include a synthetic gene of interest, a polymerase, a buffer, a forward primer including a 5' end that hybridizes to a first region of a polynucleotide that is complimentary to the synthetic gene of interest, and a reverse primer, wherein the reverse primer comprises a poly-T sequency of 150 bp or longer and a 5' region that is complimentary to a 5' end of the synthetic gene of interest; controlling the temperature of the first reactor of the microfluidic path device to perform a polymerase chain reaction (PCR) within the microfluidic path device to amplify the synthetic gene of interest using the forward primer and the reverse primer to form a synthetic product including the poly-A sequence of 150 bp or longer; and transporting the synthetic product out of the first reactor, wherein the synthetic product comprises the synthetic DNA template.

In some examples a method of making (e.g., of making a synthetic product comprising a DNA template) may include: transporting reagents to a first reactor in a microfluidic path device, wherein the reagents include a synthetic gene of interest, a polymerase, a buffer, nucleotides, a reverse primer including a 3' end that is complimentary to a first region of the synthetic gene of interest, and a forward primer, wherein the forward primer comprises a poly-A sequence of 150 bp or longer and a 3' region that that hybridizes to a second region of a polynucleotide that is complimentary to the synthetic gene of interest; controlling a temperature of the first reactor of the microfluidic path device to perform a polymerase chain reaction (PCR) within the microfluidic path device to amplify the synthetic gene of interest using the forward primer and the reverse primer to form a synthetic product including the poly-A sequence of 150 bp or longer; and transporting the synthetic product out of the first reactor, wherein the synthetic product comprises the synthetic DNA template.

For example, an automated method of making a synthetic DNA template for *in vitro* transcription may include: transporting reagents to a first reactor in a microfluidic path device, wherein the reagents include a synthetic gene of interest, a polymerase, a buffer, nucleotides, a reverse primer including a 3' end that is complimentary to a first region of the synthetic gene of interest, and a forward primer, wherein the forward primer comprises a poly-T sequency of 150 bp or longer and a 3' region that that hybridizes to a second region of a polynucleotide that is complimentary to the synthetic gene of interest; controlling the temperature of the first reactor of the microfluidic path device to perform a polymerase chain reaction (PCR) within the microfluidic path device to amplify the synthetic gene of interest using the forward primer and the reverse primer to form a synthetic product including the poly-A sequence of 150 bp or longer; and transporting the synthetic product out of the first reactor, wherein the synthetic product comprises the synthetic DNA template.

Also described herein is a method of making, comprising: transporting reagents to a first reactor in a microfluidic path device, wherein the reagents include a synthetic gene of interest, a polymerase, a buffer, nucleotides, a first primer including an end that is complementary to or that includes a sequence of a 3' end region of the synthetic gene of interest, and a second primer, wherein the second primer comprises a poly-T sequence of 150 bp or longer, or a poly-A sequence of 150 bp or longer, and an end region that includes or that is complimentary to a 5' end region of the synthetic gene of interest; controlling a temperature of the first reactor of the microfluidic path device to thermocycle at least a first fluid reactor of the microfluidic path device to perform a polymerase chain reaction (PCR) within the microfluidic path device to amplify the synthetic gene of interest to generate 1 µM or more of synthetic product using the first primer and the second primer to form a synthetic product including a promoter region and a poly-A sequence of 150 bp or longer; and transporting the synthetic product to a second one or more reactors in the microfluidic path device, wherein the synthetic product comprises the synthetic DNA template.

For example, an automated method of making a synthetic DNA template for *in vitro* transcription, the method comprising: transporting reagents to a first reactor in a microfluidic path device, wherein the reagents include a synthetic gene of interest, a polymerase, a buffer, a first primer including an end that is complementary to or that includes the sequence of a 3' end region of the synthetic gene of interest, and a second primer, wherein the second primer comprises a poly-T sequency of 150 bp or longer and an end region that includes or that is complimentary to a 5' end region of the synthetic gene of interest; controlling the temperature of the first reactor of the microfluidic path device to thermocycle at least the first fluid reactor of the microfluidic path device to perform a polymerase chain reaction (PCR) within the microfluidic path device to amplify the synthetic gene of interest to generate about 0.5 µM or more (e.g., about 1 µM or more, about 2 µM or more, about 3 µM or more, about 4 µM or more, about 5 µM or more, about 7.5 µM or more, about 10 µM or more, about 50 µM or more, about 100 µM or more, etc.) of synthetic product using the first primer and the second primer to form a synthetic product including the promoter region and a poly-A sequence of 150 bp or longer; and transporting the synthetic product to a second one or more reactors in the microfluidic path device, wherein the synthetic product comprises the synthetic DNA template.

Purifying the therapeutic polynucleotide may comprise two-dimensional (2D) purification of the therapeutic polynucleotide within one or more of the plurality of reactors. 2D purification may be performed within the substantially flat microfluidic path devices (e.g., microfluidic path plate devices) described herein, and may include using a material to remove material (e.g., double-stranded RNA, etc.) from the therapeutic polynucleotide. 2D purification of the polynucleotides in the microfluidic path device may be particularly advantageous compared to other techniques, which may involve the use of columns and may involve operations that are difficult or impossible to perform in a closed path environment and/or in small volumes as described herein. In some examples purifying the therapeutic polynucleotide comprises removing double-stranded mRNA using a cellulose material within the one or more reactors.

Any of these methods may include formulating the therapeutic polynucleotide with a delivery vehicle in one or more reactors on the one or more microfluidic path devices to form a therapeutic polynucleotide composition. The therapeutic polynucleotide (e.g., mRNA) may be encapsulated with the delivery vehicle as described herein, and in some examples may include additional mRNAs in addition to the therapeutic mRNA, including adjuvant mRNAs (e.g., mRNA enclosing proteins that enhance the immune response). The delivery vehicle may comprise an amphipathic nanoparticle, e.g., an amino-lipidated peptoid.

The system may automatically and continuously perform the operations of forming the synthetic template, performing *in vitro* transcription from the template, and purifying the therapeutic polynucleotide with optical feedback from one or more sensors of system.

In general, the therapeutic polynucleotide may be an mRNA. For example, the therapeutic polynucleotide may be an mRNA, a circular RNA or a self-replicating RNA, etc.

The methods described herein may be performed locally (e.g., at a site of care) completely or in part, as mentioned above. Advantageously, the methods described herein may permit on-demand manufacture of therapeutic mRNAs without the use of preservatives or additives into the therapeutic mRNA that may decrease efficacy and/or risk complications. It may be particularly beneficial to formulate the delivery vehicles with the therapeutic polynucleotide (e.g., therapeutic mRNA) locally, as the therapeutic composition including the therapeutic mRNA and delivery vehicle may aggregate and cluster over time. Further, these methods may be performed quickly, compared to existing methods. For example, the systems described herein may automatically and continuously perform the operations of forming a synthetic template, performing *in vitro* transcription from the template to produce a therapeutic polynucleotide, and purifying the therapeutic polynucleotide in less than 5 days (e.g., less than 4 days, less than 3 days, etc.).

Any of these methods may include sealing the fluid depots to the one or more microfluidic path devices and pressurizing the fluid depots before transporting the reagents between the fluid depots and the plurality of reactors on the one or more microfluidic path devices. The controller may control pressurizing the fluid depots.

In general, these methods and apparatuses may record the manufacture in part or entirely, and this recording may be optical (e.g., showing the movement of fluid within the microfluidic path device, including movies, video, etc.) and/or non-optical sensor data (pressure readings, temperature readings, etc.). This manufacturing data may be saved, stored and/or transmitted for later review, including for quality control and testing. Thus, any of these methods may include recording movement of fluid within the one or more microfluidic path devices during the performance of the processes in a data structure (e.g., digital file, recording, etc.) associated with the therapeutic polynucleotide that is manufactured.

Any of the methods described herein may be performed automatically or semiautomatically by a system including a computer (e.g. processor) executing software configure to perform all or some of these methods (e.g., a non-transitory, computer readable media encoding these instructions). For example, a non-transitory computer readable medium embodying instructions for manufacturing a therapeutic polynucleotide, that when executed by a controller of a system comprising a plurality of fluid depots configured to be secured in sealed fluid communication with one or more microfluidic path devices, cause the controller to perform the method of: pressurize the plurality of fluid depots in fluid communication with one or more microfluidic path devices; transporting reagents between one or more fluid depots of the plurality of fluid depots and a plurality of reactors on the one or more microfluidic path devices in a sealed and closed fluidic path that is protected from atmospheric contact to perform the operations of: forming a synthetic template, performing *in vitro* transcription from the template to produce a therapeutic polynucleotide, and purifying the therapeutic polynucleotide (e.g., all in one or more microfluidic path devices).

The instructions may further cause the controller to automatically and continuously perform the operations of forming the synthetic template, performing *in vitro* transcription from the template, and purifying the therapeutic polynucleotide based on optical feedback from one or more optical sensors of the system. The instructions may further cause the controller to control purifying of the therapeutic polynucleotide by two-dimensional (2D) purification within one or more of the plurality of reactors, and/or formulate the therapeutic polynucleotide with a delivery vehicle in one or more reactors on the one or more microfluidic path devices to form a therapeutic polynucleotide composition, and/or dialyze and/or concentrate the therapeutic polynucleotide composition in the one or more microfluidic path devices, etc.

Also described herein are automated methods of making a synthetic double-stranded DNA template for an mRNA synthesis using any of the closed-path systems described herein. For example, a method of making a synthetic double-stranded DNA template for an mRNA synthesis using a closed-path system comprising a plurality of fluid depots configured to be secured in sealed fluid communication with one or more microfluidic path devices may include: transporting reagents between one or more fluid depots of the plurality of fluid depots and a plurality of reactors on the one or more microfluidic path devices in a closed fluidic path that is protected from atmospheric contact to combine the reagents; and forming the synthetic double-stranded DNA template for *in vitro* transcription of a therapeutic mRNA.

The synthetic template formed (synthetic double-stranded DNA template) may be free of bacterial DNA and free of endotoxin.

These methods may include receiving, in a controller for the closed-path system, optical sensor data from one or more sensors of the closed-path system, wherein the controller controls the operation of the closed-path system based on the optical sensor data.

The methods may include pressurizing the fluid depots, and/or transporting reagents comprises transporting a synthetic gene of interest and a synthetic *in vitro* transcription facilitator cassette from one or more fluid depots of the plurality of fluid depots to a first one or more reactors in the microfluidic path device, joining the synthetic gene of interest with the synthetic *in vitro* transcription facilitator cassette to create a synthetic product, removing unreacted material away from the synthetic product, and amplifying the synthetic product to generate the synthetic double-stranded DNA template. The one or more microfluidic path devices comprises a microfluidic path plate device seated in the closed-path system.

Advantageously, these methods may include making significant amounts of templates (mM amounts) compared to other systems (which typically produce only femtomolar amounts). The methods and apparatuses described herein may be produce large amounts of template.

An automated method of making a synthetic double-stranded DNA template for *in vitro* transcription using a closed-path system comprising a plurality of fluid depots in sealed fluid communication with a microfluidic path device may include: transporting reagents, including a synthetic gene of interest and a synthetic *in vitro* transcription facilitator cassette, from one or more fluid depots of the plurality of fluid depots to a first one or more reactors in the microfluidic path device in a closed fluidic path that is protected from atmospheric contact; joining the synthetic gene of interest with the synthetic *in vitro* transcription facilitator cassette to create a synthetic product; transporting the synthetic product in the microfluidic path device to remove unreacted synthetic gene of interest and unreacted synthetic *in vitro* transcription facilitator cassette away from the synthetic product; and transporting the synthetic product in the microfluidic path device and amplifying the synthetic product to generate the double-stranded DNA template. As mentioned, amplifying the synthetic product may include generating greater than about 0.5 µM or more (e.g., about 1 µM or more, about 2 µM or more, about 3 µM or more, about 4 µM or more, about 5 µM or more, about 7.5 µM or more, about 10 µM or more, about 50 µM or more, about 100 µM or more, etc.) of an amplified DNA template.

Also described herein are automated methods of making a synthetic double-stranded DNA template for *in vitro* transcription using a closed-path system comprising a plurality of fluid depots in sealed fluid communication with a microfluidic path device, the method comprising: transporting reagents, including a synthetic gene of interest and a synthetic *in vitro* transcription (IVT) facilitator cassette, from one or more fluid depots of the plurality of fluid depots to a first one or more reactors in the microfluidic path device in a closed fluidic path that is protected from atmospheric contact; joining the synthetic gene of interest with the synthetic IVT facilitator cassette in the first one or more reactors to create a synthetic product; transporting the synthetic product to a second one or more reactors in the microfluidic path device to remove unreacted synthetic gene of interest and unreacted synthetic IVT facilitator cassette away from the synthetic product; and transporting the synthetic product to a third one or more reactors in the microfluidic path device and amplifying the synthetic product to generate greater than about 0.5 µM or more (e.g., about 1 µM or more, about 2 µM or more, about 3 µM or more, about 4 µM or more, about 5 µM or more, about 7.5 µM or more, about 10 µM or more, about 50 µM or more, about 100 µM or more, etc.) of an amplified DNA template; and receiving, in a controller for the closed-path system, optical sensor data from one or more sensors of the closed-path system, wherein the controller controls the operation of the closed-path system based on the optical sensor data.

For example, an automated method of making a synthetic double-stranded DNA template for *in vitro* transcription using a closed-path system comprising a plurality of fluid depots in sealed fluid communication with a microfluidic path device may include: transporting, using a first fluid power circuit, a synthetic gene of interest and a synthetic *in vitro* transcription (IVT) facilitator cassette from one or more fluid depots of the plurality of fluid depots into one or more joining reactors of the microfluidic path plate device in a closed fluidic path that is protected from atmospheric contact and joining the synthetic gene of interest to the IVT facilitator cassette to create a synthetic product; removing, using a second fluid power circuit, unreacted synthetic gene of interest and unreacted synthetic *in vitro* transcription facilitator cassette away from the synthetic product in the microfluidic path plate device; transferring, using a third fluid power circuit, the synthetic product into one or more amplification reactors of the microfluidic path device and amplifying the synthetic product to generate greater than about 0.5 µM or more (e.g., about 1 µM or more, about 2 µM or more, about 3 µM or more, about 4 µM or more, about 5 µM or more, about 7.5 µM or more, about 10 µM or more, about 50 µM or more, about 100 µM or more, etc.) of an amplified DNA template; and receiving, in a controller for the closed-path system, optical sensor data from one or more optical sensors of the closed-path system, wherein the controller controls the first, second and third fluid power circuits based on the optical sensor data and maintains plurality of fluid depots and the microfluidic path devices in a closed-path and sealed environment.

In any of these methods, the synthetic double-stranded DNA template is free of bacterial DNA and free of endotoxin.

Any of these methods may include receiving, in a controller for the closed-path system, optical sensor data from one or more sensors of the closed-path system, wherein the controller controls the operation of the closed-path system based on the optical sensor data. The optical sensor data may be data from a camera or other imaging sensor. The methods described herein may use one or more fluid power circuits to move material between the plurality of fluid depots and the microfluidic path device or within the microfluidic path device. The controller may coordinate the operation of the fluid power circuits, including using the optical information to coordinate. For example, the controller may determine that fluid is within one or more parts of the closed-path system (e.g., the depot, fluid lines, and/or region(s) of the microfluidic path sensor).

Any of these methods may include transferring the amplified DNA template to one or more digestion reactors of the microfluidic path device and enzymatically modifying the amplified synthetic product to generate the double-stranded DNA template.

As used herein, joining the synthetic gene of interest with the synthetic *in vitro* transcription facilitator cassette to create a synthetic product may include creating a synthetic linear or circular ligated product. Joining may be via ligation and/or by hybridization and/or annealing and/or primer extension. In some examples amplifying the synthetic product includes generating a linear, branched or circular amplified DNA product, further comprising linearizing the amplified DNA product to generate the double-stranded DNA template. Ligating may include ligating with a DNA ligase or by primer extension. In some examples amplifying comprises multiple displacement amplification (MDA). Alternatively, amplifying may comprise polymerase chain reaction (PCR) amplification.

In some examples, the synthetic *in vitro* transcription facilitator cassette may comprise a double-stranded DNA template comprising a promoter; a 5' UTR; a cleavable linker; a 3' UTR; and a portion encoding a poly-A region comprising at least 200 adenine residues or 200 thymidine residues in a row. The double-stranded DNA template may include a poly-A region is at least 300 bps long at the 3' end of the synthetic gene of interest. In general, the *in vitro* transcription facilitator cassette may be less than 1 kb in length. In some examples, the synthetic *in vitro* transcription facilitator cassette does not encode an antibiotic resistance gene, and/or does not have an origin of replication (ORI).

Also described herein are automated methods and apparatuses for performing *in vitro* transcription (IVT) using a template material (including, but not limited to the template material described above), to form a therapeutic mRNA. For example, described herein are methods and apparatuses for automatically performing an *in vitro* transcription (IVT) reaction using a system comprising a plurality of fluid depots configured to be secured in sealed fluid communication with one or more microfluidic path devices, the method comprising: transporting reagents between one or more fluid depots of the plurality of fluid depots and a plurality of reactors on the one or more microfluidic devices in a closed fluidic path that is protected from atmospheric contact to perform *in vitro* transcription of a therapeutic mRNA from a template in the one or more microfluidic path devices and to purify the therapeutic polynucleotide.

For example, an automated method of performing an *in vitro* transcription (IVT) reaction using a system comprising a plurality of fluid depots configured to be secured in sealed fluid communication with a microfluidic path device, the method comprising: delivering a DNA template, a polymerase, and nucleotides into one or more IVT reactors of the microfluidic path device from one or more of: a fluid depot of the plurality of fluid depots and a site on the microfluidic path device; processing, in the one or more IVT reactors, the DNA template and nucleotides to form a therapeutic mRNA; and transferring the therapeutic mRNA into a one or more purification reactor regions of the microfluidic path device and purifying the therapeutic mRNA by two-dimensional (2D) purification within the one or more purification reactor regions, wherein the microfluidic path device and the plurality of fluid depots form a closed-path and sealed environment preventing atmospheric exposure.

In general, the system may include a controller to perform these methods, including, e.g., performing the operations of transporting reagents by deflecting one or more elastic layers within the microfluidic path device. These methods may include receiving, in a controller for the system, optical sensor data from one or more sensors of the system, wherein the controller controls the operation of the system based on the optical sensor data. The controller may also control pressurizing of the fluid depots. In any of the methods described herein the system may include a microfluidic path device that is seated in the system.

In general, the DNA template may comprise a double-stranded DNA template of a synthetic gene of interest and a synthetic *in vitro* transcription facilitator cassette.

Any of these methods may include delivering and transporting using one or more fluid power circuits, controlled by a controller, to move the DNA template, polymerase, nucleotides, and therapeutic mRNA material between the plurality of fluid depots and the microfluidic path device or within the microfluidic path device. For example, the delivering and transporting operations may be performed, under the control of a controller, by deflecting one or more elastic layers within the microfluidic path device to avoid atmospheric contact during the method.

In some examples an IVT reactor is used; this IVT reactor may comprise a pair of connected chambers, each having a liquid-receiving portion and a pressure-receiving portion, wherein the liquid-receiving portion is separated from the pressure-receiving portion by an elastic layer that may be deflected by the pressure-receiving portion to adjust the volume of the liquid receiving portion. The DNA template may comprise a double-stranded DNA template of a synthetic gene of interest and a synthetic *in vitro* transcription facilitator cassette.

Any of these methods may include sealing the plurality of fluid depots in fluid communication with a plurality of receiving ports on the microfluidic path device.

For example, an automated method of performing an *in vitro* transcription (IVT) reaction using a system comprising a plurality of fluid depots configured to be secured in sealed fluid communication with a microfluidic path device, may include: pressurizing the plurality of fluid depots; delivering, using one or more first fluid power circuits, a DNA template, a polymerase, and nucleotides into one or more IVT reactors of the microfluidic path device in amounts metered with sub-microliter precision from one or more of: a fluid depot of the plurality of fluid depots and a site on the microfluidic path device; processing, in the one or more IVT reactors, the template material and nucleotides to form a therapeutic mRNA; and transferring, using a second fluid power circuit, the therapeutic mRNA into a one or more purification reactor regions of the microfluidic path device and purifying the therapeutic mRNA by two-dimensional (2D) purification within the one or more purification reactor regions, wherein the microfluidic path device and the plurality of fluid depots form a closed-path and sealed environment preventing atmospheric exposure.

As mentioned, also described herein are software and/or firmware configured to perform any of these methods described herein. For example described herein are non-transitory computer readable medium embodying instructions for performing an *in vitro* transcription (IVT) reaction, that when executed by a controller of a system comprising a plurality of fluid depots configured to be secured in sealed fluid communication with one or more microfluidic path devices, cause the controller to perform the method of: pneumatically delivering a template material, a polymerase, and nucleotides into a first reactor of a microfluidic path device from a plurality of fluid depots in amounts metered with sub-microliter precision at any time during the reaction; processing the template material and nucleotides in the first reactor to form a therapeutic mRNA; and pneumatically transferring the therapeutic mRNA through the microfluidic path device away from the first reactor, wherein the first microfluidic path device and the plurality of fluid depots form a closed-path and sealed environment to prevent atmospheric exposure.

As mentioned above, in some examples the methods and apparatuses described herein may be used to formulate (e.g., compound) the therapeutic composition by automatically combining the therapeutic polynucleotide (e.g., therapeutic mRNA) with the delivery vehicle, e.g., to encapsulate the therapeutic mRNA with the delivery vehicle. This may be done within an apparatus as described herein and, in some examples, may include forming the template, and/or therapeutic polynucleotide. For example, a method of manufacturing a therapeutic mRNA composition using a system comprising a plurality of fluid depots in sealed fluid communication with one or more microfluidic path plate devices, wherein the one or more microfluidic path plate devices comprise a plurality of reactors, may include: delivering a template precursor material from one or more fluid depots of the plurality of fluid depots to a first reactor of the plurality of reactors and processing the template precursor material to form a DNA template from the template precursor material; transferring the DNA template to a second reactor of the plurality of reactors and processing the DNA template by *in vitro* transcription to form a therapeutic mRNA; transferring the therapeutic mRNA to a third reactor of the plurality of reactors and processing the therapeutic mRNA to combine it with a delivery vehicle to form the therapeutic mRNA composition; and transferring the therapeutic mRNA composition to a concentrator in fluid communication with the third reactor. Transferring the therapeutic mRNA to a third reactor may include transferring multiple different therapeutic mRNAs with the delivery vehicle to form the mRNA composition. The delivering and transferring operations may be performed by one or more fluid power circuits in the system, controlled by a controller. For example, the controller may control the fluid power circuits by deflecting one or more elastic layers within the one or more microfluidic path plate devices. The method may be performed at a site of care.

Any of these methods (and systems performing them) may be configured to automatically and in the same microfluidic path device, dialyze the therapeutic composition (e.g., of the mRNA encapsulated with the delivery vehicles) to remove material and/or to concentrate the therapeutic composition. For example, any of these methods may include dialyzing the mRNA therapeutic composition in the one or more microfluidic path plate devices to purify the mRNA therapeutic composition. Any appropriate nanoparticle may be used (e.g., an amphipathic nanoparticle such as an amino-lipidated peptoid).

In addition, any of these methods may include two-dimensional (2D) purification within one or more of the plurality of reactors in fluid communication with the second reactor.

The method of manufacturing the therapeutic mRNA composition may be fast, particularly as compared to known techniques and technologies. For example, the methods described herein may take about 5 days or less (e.g., about 4 days or less, about 72 hours or less, etc.) to form the therapeutic composition (therapeutic mRNA and delivery vehicle), including forming the synthetic template (e.g., *de novo* synthesis, without using bacterial precursors).

For example, a method of manufacturing a therapeutic mRNA composition using a system comprising a plurality of fluid depots in sealed fluid communication with one or more microfluidic path plate devices, wherein the one or more microfluidic path plate devices comprise a plurality of reactors, may include: pressurizing the plurality of fluid depots; controlling a first fluid power circuit to deliver a template precursor material from one or more fluid depots of the plurality of fluid depots to a first reactor of the plurality of reactors with sub-microliter precision and without atmospheric contact; processing the template precursor material to form a DNA template from the template precursor material; controlling a second fluid power circuit to transfer the DNA template to a second reactor of the plurality of reactors with sub-microliter precision and without atmospheric contact; processing the DNA template by *in vitro* transcription to form a therapeutic mRNA; controlling a third fluid power circuit to transfer the therapeutic mRNA to a third reactor of the plurality of reactors with sub-microliter precision and without atmospheric contact; processing the therapeutic mRNA to combine it with a delivery vehicle to form the therapeutic mRNA composition; controlling a third fluid power circuit to transfer the therapeutic mRNA composition to a concentrator in fluid communication with the third reactor; and concentrating the therapeutic mRNA composition.

The methods and apparatuses described herein may be used to provide on-demand synthesis of therapeutic polynucleotide compositions. In some examples these methods may include remotely synthesizing some of the components and using the apparatuses to locally synthesize the therapeutic polynucleotide composition that may then be delivered to the patient. For example, a method of producing a therapeutic polynucleotide composition on-demand, the method comprising: receiving, at a local facility, a therapeutic polynucleotide that has been synthesized at a remote facility; formulating the therapeutic polynucleotide composition at the local facility by performing, in an automated system that is protected from atmospheric contact, the operations of: combining the therapeutic polynucleotide with a delivery vehicle in a microfluidic path device held in the system to form the therapeutic polynucleotide composition, dialyzing the therapeutic polynucleotide composition in the microfluidic path device; an providing the therapeutic polynucleotide composition.

Synthesizing the therapeutic polynucleotide may comprise synthesizing the therapeutic polynucleotide using a microfluidic system at the remote facility by performing, in a closed fluidic path apparatus that is protected from atmospheric contact, the operations of: forming a synthetic template, performing *in vitro* transcription from the synthetic template to form the therapeutic polynucleotide; and purifying the therapeutic polynucleotide.

For example, a method of producing a therapeutic mRNA composition on-demand, the method comprising: synthesizing a therapeutic mRNA at a remote facility; transporting the therapeutic mRNA to a local facility; formulating the therapeutic mRNA composition at the local facility by performing, in an automated closed fluidic path apparatus that is protected from atmospheric contact, the operations of: combining the therapeutic mRNA with delivery vehicle in a microfluidic path device to form the therapeutic mRNA composition, dialyzing the therapeutic mRNA composition in the microfluidic path device; and providing the therapeutic mRNA composition. The local facility is a hospital or clinic, and typically includes one or more microfluidic control systems, as described herein. In some examples the remote facility may be a manufacturing facility include one or more (e.g., multiple microfluidic control systems as described herein).

The methods described herein may further include concentrating the therapeutic polynucleotide composition.

Any of these methods may include synthesizing the therapeutic polynucleotide using a system as described herein, then storing (e.g., cold and/or frozen) the transferring them (e.g., shipping them) while stored cold, from the remote facility to the local facility and receiving the therapeutic polynucleotide (e.g., mRNA) at the local facility. For example, the therapeutic polynucleotide composition may comprise an mRNA vaccine. Any of these methods may include formulating the therapeutic polynucleotide using the system, wherein the system comprises a plurality of fluid depots configured to be secured in sealed fluid communication with the microfluidic path device.

A first fluid power circuit may be used to deliver the therapeutic polynucleotide and the delivery vehicle from the plurality of fluid depots to one or more reactors of the microfluidic path device with sub-microliter precision and without atmospheric contact, in order to combine the therapeutic polynucleotide with the delivery vehicle.

As mentioned, any of these therapeutic compositions may include multiple mRNAs (including but not limited to multiple therapeutic mRNAs) encapsulated with the same (or different) delivery vehicles. For example, forming the therapeutic polynucleotide composition at the local facility may further comprise combining one or more additional therapeutic polynucleotides with the therapeutic polynucleotide and the delivery vehicle. Any appropriate delivery vehicle may be used, including those described herein. The therapeutic polynucleotide may be an mRNA, such as a linear mRNA, a circular RNA or a self-replicating RNA, etc.

The therapeutic polynucleotide may be stable at cold (e.g., about 4 degrees, about 0 degrees, about -10 degrees, etc.) temperatures for months (e.g., about 1 month or more, about 2 months or more, about 3 months or more about 6 months or more about 8 months or more, about 9 months are more, about 1 year or more, etc.) and may be stored remotely or locally. For example, these methods may include storing the therapeutic polynucleotide at the local facility prior to formulating the therapeutic composition.

For example, a method of manufacturing an mRNA therapeutic composition using a closed-path system comprising a plurality of storage depots configured to be secured in sealed fluid communication with one or more microfluidic path devices, the method comprising: transporting reagents between one or more storage depots of the plurality of storage depots and a plurality of reactors on the one or more microfluidic devices in a closed fluidic path that is protected from atmospheric contact to perform, in the one or more microfluidic path devices, each of the operations of: forming a DNA template, performing *in vitro* transcription of therapeutic mRNA from the template, purifying the therapeutic mRNA, and combining the mRNA with a delivery vehicle.

A method of manufacturing an mRNA therapeutic composition using a closed-path system comprising a plurality of storage depots configured to be secured in sealed fluid communication with one or more microfluidic path devices (wherein the one or more microfluidic path devices comprise a plurality of reactors) may include: delivering a template precursor material from one or more storage depots to a first reactor region of the plurality of reactors and processing the template precursor material to prepare a template from the template precursor material; transferring the template to a second reactor region of the plurality of reactors and processing the template by *in vitro* transcription to form a therapeutic mRNA; and transferring the therapeutic mRNA to a third reactor region of the plurality of reactors and processing the therapeutic mRNA to combine it with a delivery vehicle to form the mRNA therapeutic composition, wherein materials including the template precursor material and delivery vehicle are delivered from the storage depots into the plurality of reactors without atmospheric contact.

A method of manufacturing an mRNA therapeutic composition using a closed-path system comprising a plurality of storage depots in sealed fluid communication with one or more microfluidic path devices (e.g., wherein the one or more microfluidic path devices comprise a plurality of reactors), may include: inducing fluidic flow to deliver a template precursor material from one or more storage depots to a first reactor region of the plurality of reactors and processing the template precursor material to prepare a template from the template precursor material; transferring the template to a second reactor region of the plurality of reactors and processing the template by *in vitro* transcription to form mRNA; transferring the mRNA to a third reactor region of the plurality of reactors and processing the mRNA to combine it with delivery vehicle to form the mRNA therapeutic composition; and transferring the mRNA product depot of the one or more storage depots, wherein the materials are delivered from the storage depots into the reactors of the microfluidic path device with sub-microliter precision and without atmospheric contact. In any of the methods described herein, any of the operations may be performed pneumatically, e.g., the fluidic flow may be induced pneumatically, the fluid may be transferred pneumatically, etc. Alternatively or additionally, fluid may be driven by mechanically, hydraulically, etc.

In any of these methods (and apparatuses for performing them) the closed-path system may automatically and continuously perform the steps of forming a template, performing *in vitro* transcription of therapeutic mRNA from the template, purifying the therapeutic mRNA, and combining the mRNA with a delivery vehicle. The closed-path system may pneumatically control the performance of the operations of forming a template, performing *in vitro* transcription of therapeutic mRNA from the template, purifying the therapeutic mRNA, and combining the mRNA with a delivery vehicle. In some examples, the closed-path system pneumatically controls the performance of the operations of forming a template, performing *in vitro* transcription of therapeutic mRNA from the template, purifying the therapeutic mRNA, and combining the mRNA with a delivery vehicle by deflecting one or more membranes within the one or more microfluidic path devices.

Any of the methods and apparatuses described herein may be configured to be set up and operate at a site of care, such as a hospital, clinic, etc. This may allow immediate/on-demand, patient-specific therapeutics to be custom manufactured to a particular patient. Alternatively or additionally, therapeutic molecules that are not specific to a particular patient may be formulated with delivery vehicles in a "patient-individualized" way. Because of the methods and apparatuses described herein, any of these methods may be performed very quickly. For example, the closed-path system may automatically and continuously perform the processes of forming a template, performing *in vitro* transcription of therapeutic mRNA from the template, purifying the therapeutic mRNA, and combining the mRNA with a delivery vehicle in less than about 5 days. Alternatively, the system may use a pre-made template as an input and perform the remaining operations in shorter time.

Combining the mRNA with a delivery vehicle (formulating the therapeutic) may further comprise dialyzing the mRNA therapeutic composition in the one or more microfluidic path devices to purify the mRNA therapeutic composition.

Any of these methods may further comprise concentrating the mRNA therapeutic composition on the one or more microfluidic path devices, and/or dialyzing the therapeutic.

Any appropriate delivery vehicle may be used, including, e.g., an amphipathic nanoparticle. For example, the amphipathic nanoparticle may comprise an amino-lipidated peptoid.

Alternatively or additionally in any of the methods and apparatuses described herein, mRNAs may be pre-made and stored (e.g., at about 10 degrees C, about 4 degrees C, about 0 degrees C, about -10 degrees C, etc.) for some time. For example, any of these methods and apparatuses for performing them may include a library of therapeutic mRNAs that may be individually or collectively (e.g., 2, 3, 4, 5, 6, etc. or more individual therapeutic mRNAs may be combined and) compounded to form an mRNA therapeutic composition. As described herein, an mRNA therapeutic composition may therefore be manufactured on demand and may be formulated just-in-time in a single or multiple mRNA therapeutic composition "cocktails".

Also described herein are methods for forming the template (e.g., the DNA template). For example, a method of making a synthetic double stranded DNA template for *in vitro* transcription using a closed-path system comprising a plurality of storage depots in sealed fluid communication with a microfluidic path device, may include: joining a synthetic gene of interest with a synthetic *in vitro* transcription facilitator cassette to create a synthetic linear or circular ligated product; removing unreacted synthetic gene of interest and unreacted synthetic *in vitro* transcription facilitator cassette away from the synthetic linear or circular ligated product; amplifying the circular ligated product to generate linear, branched or circular amplified DNA; and linearizing the amplified DNA ligated product to generate double stranded DNA template, wherein each of the joining, removing, amplifying and linearizing operations are performed in the microfluidic path device by the closed-path system.

For example, a high-efficiency, automated method of making a synthetic double stranded DNA template for *in vitro* transcription, may include: pneumatically delivering each of: a synthetic gene of interest and a synthetic *in vitro* transcription facilitator cassette from one or more storage depots of a plurality of storage depots in fluid communication with a microfluidic path device into a ligation reactor of the microfluidic path device to create a synthetic linear or circular ligated product by joining the synthetic gene of interest with the synthetic *in vitro* transcription facilitator cassette; pneumatically introducing one or more exonuclease agents from one or more storage depots of the plurality of storage depots into the ligation reactor to remove unreacted material by removing unreacted synthetic gene of interest and unreacted synthetic *in vitro* transcription facilitator cassette away from the synthetic linear or circular ligated product; pneumatically delivering the synthetic linear or circular ligated product into an amplification reactor of the microfluidic path device to combine with one or more amplification agents for amplifying the linear or circular ligated product to generate linear, branched or circular amplified DNA; and pneumatically transferring the amplified DNA ligated product to a digestion reactor of the microfluidic path device to generate fully synthetic, double stranded DNA template free of any unreacted input material by linearizing the amplified DNA ligated product, wherein the ligation reactor, amplification reactor and digestion reactor and plurality of storage depots form a closed-path and sealed environment.

A method of making a synthetic double stranded DNA template for an mRNA therapeutic composition (using a closed-path system comprising a plurality of storage depots configured to be secured in sealed fluid communication with one or more microfluidic path devices) may include: transporting reagents between one or more storage depots of the plurality of storage depots and a plurality of reactors on the one or more microfluidic devices in a closed fluidic path that is protected from atmospheric contact to, in the one or more microfluidic path devices: form a template for *in vitro* transcription of a therapeutic mRNA.

In general, the methods and apparatuses described herein may produce double stranded DNA template that may be free of bacterial DNA and/or free of endotoxin. The template generation methods and apparatuses described herein may not involve bacterial culture. In addition, the therapeutic mRNA manufactured as described herein may be synthesized from the template without the use of bacterial polynucleotides. Thus, any of the methods described herein may be methods for producing therapeutic mRNA without the use of bacterial DNA, and/or isolated from endotoxin. In particular, described herein are methods of manufacturing double stranded DNA template that is free of bacterial DNA and/or endotoxin. Any of the methods described herein may be aseptic manufacturing methods.

Any of these methods may include digesting the synthetic *in vitro* transcription template with a type IIS restriction enzyme and or methylation sensitive restriction enzymes. Joining may include ligating with a DNA ligase, or ligation by DNA synthesis or ligation by primer extension. Removing may comprise digesting linear DNA with an exonuclease or by a methylation sensitive restriction enzyme. The exonuclease may comprise exonuclease V. Amplifying may comprise multiple displacement amplification (MDA). Amplifying may comprise amplifying with Φ29 DNA polymerase. Amplifying may comprise generating branched amplified DNA. Amplifying may comprise polymerase chain amplification (PCR). Amplifying may comprise amplifying with thermostable DNA polymerase.

Linearizing may comprise digesting with a type IIs restriction enzyme. Linearizing may comprise digesting with a BsaI restriction enzyme. Digestion of the synthetic *in vitro* transcription template may comprise digesting with a methylation sensitive restriction enzyme such as DpnI. The synthetic gene of interest may be linear. In some examples, the synthetic *in vitro* transcription facilitator cassette comprises double stranded DNA template comprising a promoter; a 5' UTR; a cleavable linker; a 3' UTR; and a portion encoding a poly-A region comprising at least 200 adenine residues or 200 thymidine residues in a row. The synthetic *in vitro* transcription facilitator cassette may be delivered as a single unit or as two or more units. The portion encoding the poly-A region may be at least 300 bps long. In some examples, the portion encoding poly-A region may be at least 350 bps long.

The synthetic gene of interest may comprise at least part of a T-cell receptor. The synthetic gene of interest may comprise a Complementary Determining Region (CDR).

The *in vitro* transcription facilitator cassette may be less than about 2 kb in length. The *in vitro* transcription facilitator cassette maybe less than about 1 kb in length. The *in vitro* transcription facilitator cassette may be less than about 700 basepairs in length. The synthetic *in vitro* transcription facilitator cassette may not encode an antibiotic resistance gene.

The synthetic linear or circular ligated product may not have an origin of replication (ORI). The *in vitro* transcription facilitator cassette may not have an origin of replication (ORI).

As mentioned, the operations of any of the methods described herein may be performed in a closed microfluidic path device. The operations may be performed in a closed microfluidic path device and the joining operation may be performed in a different module (e.g., a different microfluidic path device) from the amplifying operation and the amplifying operation is performed in a different module from the linearizing operation.

Any of these methods may include purifying, in the closed path of the one or more microfluidic path devices, the template.

Also described herein are methods of performing *in vitro* transcription using the closed-path method and apparatuses described herein. For example, a method of performing an *in vitro* transcription (IVT) reaction using a closed-path system (e.g., comprising a plurality of storage depots configured to be secured in sealed fluid communication with one or more microfluidic path devices) may include: transporting reagents between one or more storage depots of the plurality of storage depots and a plurality of reactors on the one or more microfluidic devices in a closed fluidic path that is protected from atmospheric contact to perform *in vitro* transcription of a therapeutic mRNA from a template in the one or more microfluidic path devices.

A method of performing an *in vitro* transcription (IVT) reaction may include: automatically delivering a DNA template through directed fluid flow, a polymerase, and nucleotides into a first reactor of a microfluidic path device from a plurality of storage depots in amounts metered with sub-microliter precision; processing the template material and nucleotides in the first reactor to form a therapeutic mRNA; and pneumatically transferring the therapeutic mRNA through the microfluidic path device away from the first reactor, wherein the first microfluidic path device and the plurality of storage depots form a closed-path and sealed environment to prevent atmospheric exposure.

The closed-path system may operate automatically and continuously. The closed-path system may pneumatically control the performance of the *in vitro* transcription of the therapeutic mRNA from the template.

Any of these methods may also include purifying the therapeutic mRNA in the one or more microfluidic devices. Transporting reagents may comprise transporting the reagents to a first reactor of the microfluidic path device from the plurality of storage depots.

Also described herein are methods of formulating (e.g., combining with delivery vehicle) a therapeutic mRNA. For example, a method of manufacturing an mRNA therapeutic composition (e.g., using a closed-path system comprising a plurality of storage depots configured to be secured in sealed fluid communication with one or more microfluidic path devices) may include: transporting reagents between one or more storage depots of the plurality of storage depots and a plurality of reactors on the one or more microfluidic devices in a closed fluidic path that is protected from atmospheric contact to formulate the mRNA therapeutic composition by combining a therapeutic mRNA with a delivery vehicle in the one or more microfluidic path devices. The closed-path system may automatically and continuously combine the mRNA with a delivery vehicle. The closed-path system may pneumatically control combining the mRNA with a delivery vehicle. For example, the closed-path system may pneumatically control combining the mRNA with a delivery vehicle by deflecting one or more membranes within the one or more microfluidic path devices.

For example, described herein are methods of manufacturing an mRNA using a system comprising a plurality of storage depots configured to be secured in sealed fluid communication with one or more microfluidic path devices. Any of these methods may include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of mRNA from the template, and purifying the mRNA.

A method of manufacturing an therapeutic mRNA composition using a system comprising a plurality of storage depots configured to be secured in sealed fluid communication with one or more microfluidic path devices may include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, and formulating the mRNA with a delivery vehicle.

A method of manufacturing an therapeutic mRNA composition using a system comprising a plurality of storage depots configured to be secured in sealed fluid communication with one or more microfluidic path devices may include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, formulating the mRNA with a delivery vehicle, and performing purifying and concentration of the formulated therapeutic mRNA.

A method of manufacturing an therapeutic mRNA composition using a system comprising a plurality of storage depots configured to be secured in sealed fluid communication with one or more microfluidic path devices may include: following a sequence of operations for forming the therapeutic mRNA composition that are encoded in a non-transitory, computer-readable medium, wherein the operations include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, and combining the mRNA with a delivery vehicle.

Also described herein are methods of manufacturing a therapeutic mRNA composition using a system comprising a plurality of storage depots configured to be secured in sealed fluid communication with a microfluidic path device, the method comprising performing *in vitro* transcription of therapeutic mRNA from a template on the microfluidic path device, and purifying the therapeutic mRNA in one or more fluidically connected reactors on the microfluidic path device.

Also described herein are therapeutics made by any of these methods, including in particular, mRNA therapeutics. For example, described herein are therapeutic mRNAs made using a system comprising a plurality of storage depots configured to be secured in sealed fluid communication with one or more microfluidic path devices, the mRNA made by: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of mRNA from the template, and purifying the mRNA.

For example, described herein are therapeutic mRNAs made using a system comprising a plurality of storage depots configured to be secured in sealed fluid communication with one or more microfluidic path devices, the mRNA made by: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, and formulating the mRNA with a delivery vehicle.

For example, described herein are therapeutic mRNAs made using a system comprising a plurality of storage depots configured to be secured in sealed fluid communication with one or more microfluidic path devices, the mRNA made by transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, formulating the mRNA with a delivery vehicle, and purifying and concentration of the formulated therapeutic mRNA.

Described herein are therapeutic mRNA compositions formed using a system comprising a plurality of storage depots configured to be secured in sealed fluid communication with one or more microfluidic path devices, by: following a sequence of operations for forming the therapeutic mRNA composition that are encoded in a non-transitory, computer-readable medium, wherein the operations include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, and combining the mRNA with a delivery vehicle. For example, a therapeutic mRNA may be a therapeutic mRNA composition formed using a system comprising a plurality of storage depots configured to be secured in sealed fluid communication with a microfluidic path device, the method comprising performing *in vitro* transcription of therapeutic mRNA from a template on the microfluidic path device and purifying the therapeutic mRNA in one or more fluidically connected reactors on the microfluidic path device.

Any of the systems described herein may include controller configured to perform any of these methods. Thus, also described herein is software, firmware or hardware configured to perform any of the methods described herein. For example, described herein are non-transitory computer readable medium embodying instructions for manufacturing an mRNA, that when executed by a controller of a system comprising a plurality of storage depots configured to be secured in sealed fluid communication with one or more microfluidic path devices, cause the controller to perform the method of: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of mRNA from the template, and purifying the mRNA.

For example, described herein are non-transitory computer readable medium embodying instructions for manufacturing an mRNA, including a therapeutic mRNA composition, that when executed by a controller of a system comprising a plurality of storage depots configured to be secured in sealed fluid communication with one or more microfluidic path devices, cause the controller to perform any of the methods described herein.

Also described herein are methods of making a synthetic double stranded DNA template for an mRNA using a closed-path system comprising a plurality of storage depots configured to be secured in sealed fluid communication with one or more microfluidic path devices, that may include: transporting reagents between one or more storage depots of the plurality of storage depots and a plurality of reactors on the one or more microfluidic devices in a closed fluidic path that is protected from atmospheric contact to combine the reagents; and forming a template for *in vitro* transcription of a therapeutic mRNA.

For example, a method of making a synthetic double stranded DNA template for use as the input into an mRNA *in vitro* transcription reaction using a closed-path system may include a plurality of storage depots configured to be secured in sealed fluid communication with one or more microfluidic path devices, the method comprising: transporting reagents between one or more storage depots of the plurality of storage depots and a plurality of reactors on the one or more microfluidic devices in a closed fluidic path that is protected from atmospheric contact; and forming a template for *in vitro* transcription of a therapeutic mRNA.

A method of manufacturing an mRNA composition using a system comprising a plurality of storage depots configured to be secured in sealed fluid communication with one or more microfluidic path devices, wherein the one or more microfluidic path devices comprise a plurality of reactors, may include: delivering a template precursor material from one or more storage depots to a first reactor region of the plurality of reactors and processing the template precursor material to prepare a template from the template precursor material; transferring the template to a second reactor region of the plurality of reactors and processing the template by *in vitro* transcription to form an mRNA; and transferring the mRNA to a third reactor region of the plurality of reactors and processing the mRNA to combine it with a delivery vehicle to form the mRNA composition, wherein materials including the template material and delivery vehicle are delivered from the storage depots into the plurality of reactors without atmospheric contact.

A method of manufacturing an mRNA composition using a system comprising a plurality of storage depots in sealed fluid communication with one or more microfluidic path devices, wherein the one or more microfluidic path devices comprise a plurality of reactors, may include: pneumatically delivering a template precursor material from one or more storage depots to a first reactor region of the plurality of reactors and processing the template precursor material to prepare a template from the template precursor material; pneumatically transferring the template to a second reactor region of the plurality of reactors and processing the template by *in vitro* transcription to form mRNA; pneumatically transferring the mRNA to a third reactor region of the plurality of reactors and processing the mRNA to combine it with delivery vehicle to form the therapeutic mRNA composition; and transferring the mRNA product to one or more storage depots, wherein the materials are delivered from the storage depots into the reactors of the microfluidic path device with sub-microliter precision and without atmospheric contact.

A method of making a synthetic double stranded DNA template for *in vitro* transcription using a closed-path system comprising a plurality of storage depots in sealed fluid communication with a microfluidic path device, may include: joining a synthetic gene of interest with a synthetic *in vitro* transcription facilitator cassette to create a synthetic linear or circular ligated product; removing unreacted synthetic gene of interest and unreacted synthetic *in vitro* transcription facilitator cassette away from the synthetic linear or circular ligated product; amplifying the linear or circular ligated product to generate linear, branched or circular amplified DNA; and linearizing the amplified DNA ligated product to generate double stranded DNA template, wherein each of the joining, removing, amplifying and linearizing operations are performed in the microfluidic path device by the closed-path system.

Any of these methods may be high-efficiency, automated methods, including high-efficiency, automated methods of making a synthetic double stranded DNA template for *in vitro* transcription. For example, a method may include: pneumatically delivering each of: a synthetic gene of interest and a synthetic *in vitro* transcription facilitator cassette from one or more storage depots of a plurality of storage depots in fluid communication with a microfluidic path device into a ligation reactor of the microfluidic path device to create a synthetic linear or circular ligated product by joining the synthetic gene of interest with the synthetic *in vitro* transcription facilitator cassette; pneumatically introducing one or more exonuclease agents from one or more storage depots of the plurality of storage depots into the ligation reactor to remove unreacted material by removing unreacted synthetic gene of interest and unreacted synthetic *in vitro* transcription facilitator cassette away from the synthetic linear or circular ligated product; pneumatically delivering the synthetic linear or circular ligated product into a multiple displacement amplification (MDA) or polymerase chain reaction (PCR) reactor of the microfluidic path device to combine with one or more amplification agents for amplifying the linear or circular ligated product to generate linear, branched or circular amplified DNA; and pneumatically transferring the amplified DNA ligated product to a digestion reactor of the microfluidic path device to generate double stranded DNA template by linearizing amplified DNA ligated product, wherein the ligation reactor, MDA or PCR reactor and digestion reactor and plurality of storage depots form a closed-path and sealed environment.

A method of making a synthetic double stranded DNA template for *in vitro* transcription, the method comprising following a sequence of operations that are encoded in a non-transitory, computer-readable medium, may include: delivering each of: a synthetic gene of interest and a synthetic *in vitro* transcription facilitator cassette from one or more storage depots of a plurality of storage depots in fluid communication with a microfluidic path device into a ligation reactor of the microfluidic path device to create a synthetic linear or circular ligated product by joining the synthetic gene of interest with the synthetic *in vitro* transcription facilitator cassette; introducing one or more exonuclease agents from one or more storage depots of the plurality of storage depots into the ligation reactor to remove unreacted material by removing unreacted synthetic gene of interest and unreacted synthetic *in vitro* transcription facilitator cassette away from the synthetic linear or circular ligated product; delivering the synthetic linear or circular ligated product into a multiple displacement amplification (MDA) or polymerase chain reaction (PCR) reactor of the microfluidic path device to combine with one or more amplification agents for amplifying the linear or circular ligated product to generate linear, branched or circular amplified DNA; and transferring the amplified DNA ligated product to a digestion reactor of the microfluidic path device to generate double stranded DNA template by linearizing amplified DNA ligated product, wherein the ligation reactor, MDA reactor and digestion reactor and plurality of storage depots form a closed-path and sealed environment.

A method of performing an *in vitro* transcription (IVT) reaction using a system comprising a plurality of storage depots configured to be secured in sealed fluid communication with one or more microfluidic path devices, may include: transporting reagents between one or more storage depots of the plurality of storage depots and a plurality of reactors on the one or more microfluidic devices in a closed fluidic path that is protected from atmospheric contact to perform *in vitro* transcription of a therapeutic mRNA from a template in the one or more microfluidic path devices.

Also described herein are methods of performing an *in vitro* transcription (IVT) reaction, the method comprising following a sequence of operations that are encoded in a non-transitory, computer-readable medium, wherein the operations include: pneumatically delivering a template material, a polymerase, and nucleotides into a first reactor of a microfluidic path device from a plurality of storage depots in amounts metered with sub-microliter precision at any time during the reaction; processing the template material and nucleotides in the first reactor to form a therapeutic mRNA; and pneumatically transferring the therapeutic mRNA through the microfluidic path device away from the first reactor, wherein the first microfluidic path device and the plurality of storage depots form a closed-path and sealed environment to prevent atmospheric exposure.

Also described herein are methods of performing an *in vitro* transcription (IVT) reaction, the method comprising following a sequence of operations that are encoded in a non-transitory, computer-readable medium, wherein the operations include: delivering, by induced fluidic flow, a template material, a polymerase, and nucleotides into a microfluidic path device from a plurality of storage depots in amounts controlled by a controller following the sequence of operations; processing the template material and nucleotides in one or more reactors to form a therapeutic mRNA; and transferring the therapeutic mRNA through the microfluidic path device away from the one or more reactors, wherein the first microfluidic path device and the plurality of storage depots form a closed-path and sealed environment to prevent atmospheric exposure.

Also described herein are methods of performing an *in vitro* transcription (IVT) reaction, the method comprising: delivering, by induced fluidic flow, a template material, a polymerase, and nucleotides into a microfluidic path device from a plurality of storage depots in amounts controlled by pre-programmed software commands; processing the template material and nucleotides in a first one or more reactors in the microfluidic path device to form a therapeutic mRNA; and transferring the therapeutic mRNA through the microfluidic path device away from the first one or more reactor, into a second one or more reactors adapted for purification of mRNA, wherein the microfluidic path device and the plurality of storage depots form a closed-path and sealed environment to prevent atmospheric exposure.

Also described herein are methods of performing an *in vitro* transcription (IVT) reaction, the method comprising following a sequence of operations that are encoded in a non-transitory, computer-readable medium, wherein the operations include: delivering, by induced fluidic flow, a template material, a polymerase, and nucleotides into a first one or more reactors of a first microfluidic path device from a plurality of storage depots, in amounts controlled by the sequence of operations; processing the template material and nucleotides in the first one or more reactors to form a therapeutic mRNA; and transferring the therapeutic mRNA through the first microfluidic path device away from the first one or more reactors, into a second one or more reactor adapted for purification of mRNA; and transferring thus purified mRNA for completion of the formulation of the mRNA therapeutic, wherein the first microfluidic path device and the plurality of storage depots form a closed-path and sealed environment to prevent atmospheric exposure.

Also described herein are methods of performing an *in vitro* transcription (IVT) reaction, the method comprising following a sequence of operations that are encoded in a non-transitory, computer-readable medium, wherein the operations include: pneumatically delivering a template material, a polymerase, and nucleotides into a first one or more reactors of a first microfluidic path device from a plurality of storage depots; processing the template material and nucleotides in the first one or more reactors to form a therapeutic mRNA; and transferring the therapeutic mRNA through the first microfluidic path device away from the first one or more reactors, into a second one or more reactors adapted for purification of mRNA; and transferring purified mRNA to a third one or more reactors to combine the purified mRNA with one or more delivery vehicles to form an mRNA therapeutic, wherein the first microfluidic path device and the plurality of storage depots form a closed-path and sealed environment to prevent atmospheric exposure.

For example, also described herein are methods of performing an *in vitro* transcription (IVT) reaction, the method comprising following a sequence of operations that are encoded in a non-transitory, computer-readable medium, wherein the operations include: pneumatically delivering a template material, a polymerase, and nucleotides into a first one or more reactors of a first microfluidic path device from a plurality of storage depots; processing the template material and nucleotides in the first one or more reactors to form a therapeutic mRNA; and transferring the therapeutic mRNA through the first microfluidic path device away from the first one or more reactors, into a second one or more reactors comprising cellulose and adapted for purification of mRNA; and transferring purified mRNA to a third one or more reactors to combine the purified mRNA with one or more delivery vehicles to form an mRNA therapeutic, wherein the first microfluidic path device and the plurality of storage depots form a closed-path and sealed environment to prevent atmospheric exposure.

Also described herein are methods of manufacturing an therapeutic mRNA composition using a system comprising a plurality of storage depots configured to be secured in sealed fluid communication with one or more microfluidic path devices, the method comprising: transporting reagents between one or more storage depots of the plurality of storage depots and a plurality of reactors on the one or more microfluidic devices in a closed fluidic path that is protected from atmospheric contact to formulate the therapeutic mRNA composition by combining one or more therapeutic mRNAs with a delivery vehicle in the one or more microfluidic path devices.

A method of manufacturing an therapeutic mRNA composition on demand using a system comprising a plurality of storage depots configured to be secured in sealed fluid communication with one or more microfluidic path devices, may include: transporting reagents between one or more storage depots of the plurality of storage depots and a plurality of reactors on the one or more microfluidic devices in a closed fluidic path that is protected from atmospheric contact to perform, in the one or more microfluidic path devices, one or more of the operations of: forming a template, performing *in vitro* transcription of therapeutic mRNA from the template, purifying the therapeutic mRNA, and formulating the mRNA with a delivery vehicle.

Any of these methods and apparatuses may be operated (e.g., the therapeutic mRNA is manufactured) at the site of care. Any of these methods and apparatuses may be performed rapidly and continuously, e.g., the therapeutic is manufactured in less than about 72 hours.

As mentioned, in any of these methods and apparatuses the polynucleotide formed (e.g., by IVT) may be purified before and after being combined with a delivery vehicle, within the microfluidic path device, under automated control of the microfluidic control apparatus.

In particular, described herein are method and apparatuses that may include one or more microfluidic path devices adapted for use with a permeable insert material that may remove one or more target materials (e.g., double-stranded RNA, etc.) and/or may add one or more additional materials (e.g., lyophilized materials) to the therapeutic material as part of the fabrication process. The permeable insert material may be configured to be retained within one or more fluid-contacting chambers of the microfluidic path device and may be adapted so that the developing therapeutic solution passes through the permeable insert material. The permeable insert material may be compressible, and/or deformable, and/or elastic, so that it may be manipulated by an elastic membrane within the chamber. The permeable insert may include a cover (e.g., an outer cover) that is permeable and contains a modifying material that is solid, granular, gel, etc. In some examples the permeable insert material is a cellulose material that is configured to selectively remove dsRNA from the solution.

A permeable material may be porous (e.g., may include pores). In some examples, the permeable material may be fibrous, or layered. For example, the permeable material may be fibrous, and may include channels through which fluid can move. In some examples channels may be formed through the permeable material to allow it to be permeable to fluid. For example, the permeable material may have channels formed by a laser, or any other means by which the interior volume may be made accessible to the fluid. In some examples the permeable material may be formed from a plurality of layers that are arranged to allow fluid to pass between or among the layers. Multiple thin layers of material with only surface accessibility, may be stacked together. For example, functionalized graphene may be layered (e.g., in the extreme, to single atomic layers). As another example, slices of aerogels may be treated or otherwise made absorptive and stacked to form the permeable material of the insert.

In any of the permeable materials described herein the material may be a pre-formed material (e.g., a pre-formed insert) that is configured to allow the passage of a fluid, in some cases at a predefined flow rate and/or flow resistance. The permeability of the material may be selected to allow flow through the permeable insert when held within the apparatus chamber or channel at the processing flow rates and fluid pressures described herein. As mentioned above, a pre-formed permeable material may be porous, fibrous, could be stacks of layers; any of these materials may be functionalized to bind a material. As used herein, a functionalized material may include any material for which a surface of the material has been modified by the addition of a compound, agent, or functional group that specifically binds a target material. Materials may also or alternatively be functionalized by surface treatments by which specific atomic molecular groups may be attached to alter specific properties of the material. Functionalization can be performed by various surface modification techniques such as wet chemistry, or vapor, gas, and/or plasma chemistry, and/or microwave assisted chemical techniques, etc. including techniques that utilize surface chemistry to bond desirable materials to surfaces. Similar techniques may be used to modify the material, e.g., to "activate" the material.

The permeable material may be configured to maximize the total surface area for the insert within the apparatus, allowing selective binding of unwanted impurities, and/or target products. Any of these permeable materials may be configured to provide for sufficient penetration of the solution into the interior of the pre-formed material for effective binding to the substances in the solution. In some examples the permeable material may comprises more than one pre-formed material, each pre-formed material may be larger than the channels or cambers within the microfluidic path device, and each of these pre-formed materials may still allow for penetration of a solution inside for maximizing of the exposure of solution to the functionally active materials.

As mentioned above, the permeable implant inserts described herein may be configured to remove an impurity (e.g., an unwanted material) from a solution; alternatively or additionally the permeable implant may be configured to bind releasably to a wanted materials, so that it may be eluted (e.g., after washing, etc.).

As mentioned above, the methods and apparatuses described herein may include the use of a microfluidic path device that includes one or more permeable inserts configured to modify the solution forming the therapeutic. The permeable insert may be adapted for use within the microfluidic path devices described herein.

For example, described herein are permeable inserts that are configured to fit within the fluid-contacting side of a chamber. Thus, the permeable insert may be sized and/or shaped to conform substantially to the volume within all or a portion (e.g., a cross-sectional region) of the fluid-contacting side of the chamber. As mentioned above, the permeable material may be a single pre-formed insert, or it may be a combination of a number of pre-formed materials forming the permeable implant. The permeable implant may generally permit a flow of solution into and through the material. In some examples the permeable implant may also be compressible (e.g., "squeezable") to allow removal of fluid from within the permeable implant when the microfluidic path device compresses the chamber in which the permeable implant is inserted. In some examples the permeable implant is sufficiently elastic to return an expanded shape after being compressed.

The permeable insert and microfluidic path device may be configured so that the fluid, and particularly the material being used to generate the therapeutic composition, necessarily passes through the permeable insert during processing. In some examples the permeable insert is a compressible material and/or an elastically deformable material (e.g., elastic) that may be deformed as the volume of the fluid-contacting chamber is changed by deflecting an elastic material (e.g., elastic layer) separating the fluid-contacting side of the chamber from a pressure-receiving side of the chamber. In some examples the permeable insert is compressible but not necessarily elastically deformable. In some examples the permeable insert is swellable material that, when activated (e.g., by the addition of fluid, such as a buffer, water, etc.) may swell within the fluid-contacting side of the chamber. The permeable insert may be compressed by deflecting the elastic material (layer) between the pressure-receiving side of the chamber and the fluid-contacting side of the chamber. In some examples, processing the therapeutic may include transmitting the solution used to formulate the therapeutic material between multiple (e.g., 2, 3, 4, etc.) chambers including a permeable insert material. In some examples, the method may include driving the solution in and out of a chamber including a permeable insert material.

In any of the methods and apparatuses described herein the therapeutic insert material may be compressed to drive the solution including the therapeutic material (or in which the therapeutic material is being formed) out of the fluid-contacting side of the chamber, e.g., by adjusting the pressure in the pressure-receiving side of the chamber to deflect the elastic membrane separating the chamber into a fluid-contacting side and a pressure-receiving side.

The permeable insert may be any appropriate material that may be used to modify and further process and/or modify the therapeutic material. For example, in some examples the permeable insert may include a cellulose material that is configured to retain dsRNA, in order to remove the dsRNA from the solution as it passes through the permeable material. Alternatively or additionally, the permeable insert may include one or more materials that may be added to the solution from the permeable insert.

For example, any of the permeable inserts described herein may include one or more additional materials adsorbed into or on the permeable insert. In any of these examples, the permeable insert may include a material for release. In some examples, e.g., a permeable insert may include a cellulose insert that may be pre-treated with DNAse to entrap the DNAse in the cellulose. This may allow the insert to simultaneously remove dsRNA (as described herein) and digest a DNA material, such as the DNA template from an *in vitro* transcription operation.

Any of the permeable inserts described herein may be configured as surface-functionalized inserts that include one or more additional agents attached, adsorbed or otherwise included on or in the permeable insert. For example, in some example an additional material such included in or on the permeable inserts may include covalently-tethered materials (e.g., antibodies or aptamers), electrostatically tethered materials, adsorbed enzymes (e.g., in some examples, that may selectively degrade an impurity, such as a DNAse as mentioned above), covalently or non-covalently attached sensors (e.g., to detect a material to be removed, such as double stranded RNA, impurities, etc.). In some examples an additional material or materials may include a poly(dT) sequence to capture the polyadenylated RNA molecules, e.g., bound to the surface(s) of/in the permeable implant (for example, poly(dT) sequences may be used within the permeable implant to isolate mRNA). In some examples an additional material or materials may include small molecules to enhance binding properties (e.g., a dsRNA intercalator such as ethidium bromide may selectively bind dsRNA material without binding ssRNA). In some examples, the permeable insert may be at least partially coated with a material. For example, in some examples the coating may be a carboxylate coating.

In some examples the permeable insert may include a lyophilized material that may be released into the solution immediately or in a timed-release manner. For example, in some examples, as the solution contacts the permeable insert, it may dissolve the lyophilized material into the solution. Examples of lyophilized materials may include one or more buffer materials (e.g., salts, chelators, detergents, polynucleotides, enzymes, proteins, etc.). In some examples the permeable insert may include agents, such as binding agents, for binding to one or more materials in the solution. For example, the permeable insert may include bound immunoagents, e.g., antibodies, or portions thereof, including FAB fragments, etc., that may selectively remove material from the solution.

In general, the permeable insert may be configured to span the fluid-contacting side of the chamber so that fluid passes over and/or through the permeable insert. Permeable insert may be a paper, e.g., sheet of material. The permeable insert may be folded so as to span and/or at least partially fill the fluid-contacting side of the chamber. Folded shapes may therefore span the fluid-contacting portion of the chamber, while being configured for deflecting (including elastically deflecting). The folds may include simple folds (e.g., fan-shaped folds) or more complex folds; in general the folds may include one or more bent, regions that may operate as a hinged (e.g., living hinge) region and//or may be biased to return to an expanded shape after being compressed or otherwise deflected by the movement of the elastic membrane dividing the chamber into the fluid-contacting chamber and the pressure-receiving chamber. In some examples the permeable insert may form a sponge. The permeable insert may be formed as a foamed or puffed material.

In any of the examples of permeable inserts described herein, the size of the passages (e.g., pores, channels, chambers, etc.) within the permeable insert may be configured to pass or exclude a material based on the size. Thus, the permeable inserts descried herein may be configured to perform size-exclusion (e.g., size-exclusion chromatography). For example, a large mRNA molecule that has unreacted mononucleotides may be passed into a chamber containing a nano-porous insert, and unreacted dNTPs may diffuse into the insert and become physically entrapped therein, while the size of the passages (e.g., pores) can exclude the large molecules.

For example, in examples in which the permeable insert includes cellulose (e.g., for removing dsRNA), the cellulose may be in the form of a paper (e.g., filter paper), which may be folded or layered, including folds that are configured to be retained within the fluid-contacting portion of the chamber. In some examples the cellulose may be puffed, or foamed. In some examples the cellulose may be in the form of a sponge.

The permeable insert may generally have pores that are of any appropriate size. Pore size may be uniform or non-uniform; in some examples, the pore size may be distributed within a size range.

In any of the methods and apparatuses described herein, the temperature of the microfluidic path device may be controlled, as described herein. In particular, the temperature of the chamber containing the permeable insert may be controlled. For example, the chamber containing the permeable insert in the microfluidic path device may be maintained at a target temperature when the solution including the therapeutic material (or in which the therapeutic material is being formed) contacts the permeable insert. The temperature may be maintained, e.g., at between about 2 degrees C and about 20 degrees, between about 2 °C and about 5°C, between about 2°C and about 10°C, between about 2°C and about 15°C, between about 5°C and about 10°C, between about 5°C and about 15°C, between about 5°C and about 20°C, between about 10°C and about 15°C, between about 10°C and about 20°C, between about 10°C and about 30°C, between about 10°C and about 25°C, between about 15°C and about 20°C, between about 15°C and about 25°C, between about 15°C and about 30°C, between about 20°C and about 25°C, between about 20°C and about 30°C, between about 25°C and about 30°C, between about 25°C and about 40°C, between about 25°C and about 35°C, between about 30°C and about 35°C, between about 30°C and about 40°C, between about 35°C and about 40°C, between about 30°C and about 50°C, between about 30°C and about 45°C, between about 35°C and about 40°C, between about 35°C and about 45°C, between about 35°C and about 50°C, between about 40°C and about 45°C, between about 40°C and about 50°C, between about 45°C and about 50°C, between about 40°C and about 60°C, between about 40°C and about 55°C, between about 45°C and about 55°C, between about 45°C and about 60°C, between about 50°C and about 55°C, between about 50°C and about 60°C, between about 55°C and about 60°C, between about 50°C and about 70°C, between about 50°C and about 65°C, between about 55°C and about 65°C, between about 55°C and about 70°C, between about 60°C and about 70°C, between about 60°C and about 75°C, between about 65°C and about 70°C, between about 65°C and about 75°C, between about 65°C and about 80°C, between about 70°C and about 80°C, between about 75°C and about 80°C, between about 60°C and about 80°C, between about 65°C and about 75°C, between about 65°C and about 80°C , between about 75°C and about 80°C, between about 70°C and about 90°C, between about 75 °C and about 90°C , between about 80°C and about 90°C , between about 85°C and about 85°C, between about 85°C and about 90°C, etc. The temperature may be constant, or it may be varied (e.g., increased, decreased, etc.) before, during and/or after exposure to the permeable insert.

In some examples the permeable insert may be referred to as a solid permeable insert; the permeable (e.g., solid permeable) insert may be configured so that it remains entirely contained within the fluid-contacting side of the chamber. In some examples, as mentioned above, in some examples the permeable insert may be configured as a permeable package that is enclosed by an outer contained, e.g., a permeable cover, that encloses a material and confines the material within the permeable cover. For example, the permeable cover may enclose a granular material, or a gel (e.g., a hydrogel), or the like. The permeable cover may be formed of a material such as a membrane material that is sufficiently permeable to allow fluid to pass through the cover and into the volume contained by the cover. The permeable insert may therefore form a pillow-like shape that may be compressible and/or elastically deformable.

In general, the permeable insert may be inserted into the fluid-contacting chamber of the microfluidic path device and may be configured to fit within the fluid-contacting chamber as mentioned above. In some examples the permeable insert is configured to fit snuggly in the fluid-contacting side of the chamber; for example, the permeable insert may have a shape that is complementary to the shape of the fluid-contacting side of the chamber (e.g., oval, round, square, rounded square, etc.). As mentioned, the permeable insert may be configured to span and/or fill the volume, and in particular, span the volume in a direction perpendicular to the direction of flow through the volume of the fluid-contacting side, so that fluid passes through the permeable insert.

For example, described herein are microfluidic path devices that may include: a means for inducing fluid flow of a solution within the microfluidic path device; a plurality of chambers; and a permeable insert within a first chamber of the plurality of chambers, wherein the insert is configured to be compressed. The means for inducing fluid flow of a solution may include any appropriate means, in particular, a plurality of pressure ports on the microfluidic path device configured to receive positive or negative pressure to deflect a membrane within the microfluidic path device. For example, described herein are microfluidic path devices comprising: an elastic material sandwiched between a first plate and a second plate; and a plurality of chambers formed between the first plate and the second plate, wherein a portion of the elastic material divides each chamber into a fluid-contacting side and a pressure-receiving side. Any of these microfluidic path devices may include a solid and permeable insert within the fluid-contacting side of a first chamber.

For example, described herein are microfluidic path devices comprising: an elastic material sandwiched between a first plate and a second plate; a plurality of chambers formed between the first plate and the second plate, wherein a portion of the elastic material divides each chamber into a fluid-contacting side and a pressure-receiving side; and a solid and permeable insert within the fluid-contacting side of a first chamber of the plurality of chambers, wherein the insert is configured to be compressed by deflection of the elastic material when pressure is applied to the pressure-receiving side of the first chamber.

In some examples, the microfluidic path device comprises: an elastic material sandwiched between a first plate and a second plate; a plurality of chambers formed between the first plate and the second plate, wherein a portion of the elastic material divides each chamber into a fluid-contacting side and a pressure-receiving side; and a solid and permeable insert within the fluid-contacting side of a first chamber of the plurality of chambers, wherein the insert comprises a cellulose material configured to purify RNA, wherein the elastic material diving the first chamber is configured to be deflected by the application of pressure to the pressure-receiving side to move fluid into or out of the first chamber.

A microfluidic path device may comprise: an elastic material sandwiched between a first plate and a second plate; a plurality of chambers formed between the first plate and the second plate, wherein a portion of the elastic material divides each chamber into a fluid-contacting side and a pressure-receiving side; a plurality of fluid ports configured to be in fluid communication with the fluid-contacting sides of the plurality of chambers; a plurality of pressure ports in fluid communication with the pressure-receiving sides of the plurality of chambers; and a solid and permeable insert within the fluid-contacting side of a first chamber of the plurality of chambers, wherein the insert is configured to be compressed by deflection of the elastic material when pressure is applied to the pressure-receiving side of the first chamber from one or more of the pressure ports.

In some examples, and in particular, examples in which the permeable insert is configured to remove an undesired material (e.g., dsRNA) from the solution, such as examples including cellulose, the chamber may be referred to as a separation chamber.

As mentioned, in any of these apparatuses (e.g., systems, devices, etc.) the solid and permeable insert may include a cellulose material configured to purify RNA. For example, the solid and permeable insert may comprise a sheet of cellulose material. Alternatively or additionally, the solid and permeable insert may comprise a lyophilized material.

The solid and permeable insert may have a profile that matches the profile of the first chamber. As mentioned, the solid and permeable insert may be elastic.

The solid and permeable insert may comprise a permeable outer covering containing a granular material. In some examples, the solid and permeable insert comprises a folded structure.

In some examples, the microfluidic path device may include a second chamber that is fluidly connected to the first chamber. The device may be configured to transfer fluid between the first chamber and second chamber by deflecting the elastic material. In some examples the fluid may be reciprocated between the first chamber and the second chamber.

The microfluidic path device may include a plurality of individually addressable pressure ports extending through the first plate and configured to deliver pressure to the pressure-receiving sides of the plurality of chambers to move fluid in the fluid-receiving side.

Also described herein are methods of using any of the devices described herein. For example, a method of processing a therapeutic material in a fluid (e.g., an RNA sample) may include: coupling a microfluidic path device to a pressure source; applying pressure to transport the sample to a fluid-contacting side of a separation chamber of the microfluidic path device; passing the sample into a solid and permeable insert within the fluid-contacting side of the separation chamber, wherein the sample is modified by the solid and permeable insert; and applying pressure to transport the sample out of the fluid-contacting side of the separation chamber.

For example, a method of removing double-stranded RNA (dsRNA) from an RNA sample containing both dsRNA and single-stranded RNA (ssRNA), may include: coupling a microfluidic path device to a pressure source; applying pressure to transport the RNA sample to a fluid-contacting side of a separation chamber of the microfluidic path device; passing the RNA sample into a solid and permeable insert within the fluid-contacting side of the separation chamber, wherein the solid and permeable insert comprises cellulose, so that dsRNA is retained by the insert; and applying pressure to transport the RNA sample out of the fluid-contacting side of the separation chamber.

A method of removing double-stranded RNA (dsRNA) from an RNA sample containing both dsRNA and single-stranded RNA (ssRNA) may include: coupling a microfluidic path device to a pressure source; applying pressure to transport the RNA sample to a fluid-contacting side of a separation chamber of the microfluidic path device so that the RNA sample passes through a solid and permeable insert comprising cellulose within the fluid-contacting side of the separation chamber, so that the dsRNA is retained by the insert; and applying pressure to a pressure-receiving side of the separation chamber to transport the RNA sample out of the fluid-contacting side of the separation chamber.

The method may include synthesizing the RNA sample by *in vitro* transcription in the microfluidic path device. In some examples the method may include coupling the microfluidic path device to a source of the RNA sample.

Applying pressure to transport the RNA sample out of the fluid-contacting side may include applying pressure to a pressure-receiving side of the separation chamber to deflect an elastic material separating the pressure-receiving side of the separation chamber from the fluid-contacting side of the separation chamber. Applying pressure to the pressure-receiving side of the separation chamber may include transporting the RNA sample out of the fluid-contacting side of the separation chamber and into a fluid-contacting side of a mixing chamber, further comprising applying pressure to a pressure-receiving side of the mixing chamber to transport the RNA sample back into the fluid-contacting side of the separation chamber. Applying pressure to transport the RNA sample out of the fluid-contacting side of the separation chamber may include compressing the solid and permeable insert by an elastic material separating the pressure-receiving side of the separation chamber from the fluid-contacting side of the separation chamber.

Also described herein are microfluidic path devices (e.g., microfluidic path devices for making a product comprising a synthetic DNA template) that include: an elastic layer sandwiched between a first plate region having a first surface and a second plate region having a second surface; a plurality of PCR chambers each having a fixed volume connected by one or more channels, wherein each PCR chamber is formed between the first surface and the second surface, wherein a portion of the elastic layer divides each chamber into a fluid-contacting side in the second surface and a pressure-receiving side in the first surface, wherein the pressure receiving side is further partitioned by one or more fluidly-connected serpentine pathways; a plurality of fluid channels each extending from a fluid port through the first plate region and into the second plate region to fluidly connect with the fluid-contacting side of one or more of the plurality of chambers; a plurality of pressure channels each extending from one or more pressure ports, through the first plate region and elastic layer, into the second plate region, and back through the elastic layer and into the first plate region, wherein each pressure channel of the plurality of pressure channels extends within the first plate region and fluidly connects with one or more pressure-receiving sides of one or more of the plurality of chambers; and a UV yield detection chamber in fluid communication with one or more of the PCR chambers, wherein the UV yield detection chamber comprises a UV yield detection window configured to pass UV light therethrough for quantification of a polynucleotide within the UV yield detection chamber.

In any of the microfluidic path devices described herein the fluid-contacting side of each PCR chamber may have a thickness of 1.5 cm or less (e.g., 1.4 cm or less, 1.3 cm or less, 1.2 cm or less, 1.1 cm or less, 1.0 cm or less, 0.9 cm or less, 0.8 cm or less, 0.7 cm or less, 0.6 mc or less, 0.5 cm or less, etc.).

Any of these microfluidic path devices may include a purification chamber in fluid communication with a purification substrate. The microfluidic path device may be configured as a removable cartridge configured to engage with a fluid depot and pneumatic drive.

Any of the microfluidic path devices descried herein may include a vacuum cap, wherein the vacuum cap comprises a bubble-removing chamber formed between the first surface and the second surface, wherein a gas-permeable elastic layer divides the bubble-removing chamber into a fluid-contacting side of the bubble-removing chamber in the second surface and a vacuum receiving side in the first surface, further wherein the fluid-contacting side of the bubble-removing chamber is in fluid communication with the fluid-contacting sides of at least one of the PCR chambers.

The fluid-contacting side in the second surface and the pressure-receiving side may be concave and configured so that the elastic layer seats flush and without gaps to the fluid-contacting side in the second surface when a positive pressure in the pressure-receiving side drives the elastic layer against the fluid-contacting side. The one or more pressure ports and fluid ports may be disposed adjacent to a periphery of the microfluidic path device on an upper surface of the first plate.

The microfluidic path device may also include a material inserted into the fluid-contacting side of the channel. The material may include a cellulose material configured to selectively absorb double-stranded mRNA.

The first plate and the second plate may be formed from a rigid material, wherein the rigid material is a polymer or glass. The polymer may be a cycloolefin copolymer.

Any of these methods may include pre-wetting the solid and permeable inset.

All of the methods and apparatuses described herein, in any combination, are herein contemplated and can be used to achieve the benefits as described herein.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIG. 1A schematically illustrates one example of a method of manufacturing an mRNA therapeutic.
FIG. 1B schematically illustrates one example of an exemplary process for manufacturing a patient-specific T-cell lymphoma vaccine drug product.
FIG. 2A illustrates one example of a microfluidic path device control system as described herein.
FIG. 2B schematically illustrates one example of a microfluidic path device control system that may be used as described herein.
FIGS. 3A-3C illustrate example of microfluidic path devices as described herein.
FIG. 4 is a section through a portion of one example of a microfluidic path device as described herein.
FIG. 5 is one example of a peptoid delivery vehicle that may be used in any of the methods as described herein.
FIG. 6A shows an example of an *in vitro* transcription facilitator cassette useful for making a double-stranded DNA template.
FIG. 6B shows an example of a double-stranded DNA template generated as described herein.
FIG. 7 illustrates one method of making a synthetic DNA template for *in vitro* transcription by PCR as described herein.
FIG. 8 shows one region of an example of a T-cell receptor useful for making a double-stranded DNA for use in a vaccine or therapeutic.
FIG. 9 shows an overview of one example of an architecture of a microfluidic path device reactor for generating double-stranded DNA.
FIG. 10 schematically illustrates one example of a codon optimization process that may be used in any of the methods and apparatuses described herein.
FIG. 11 schematically illustrates one example of a functional diagram for a microfluidic path device configured to perform IVT as described herein.
FIG. 12 schematically illustrates one example of a functional diagram for a microfluidic path device configured as a formulation microfluidic path device as described herein.
FIG. 13 schematically illustrates another example of a functional diagram for a microfluidic path device configured as a formulation microfluidic path device as described herein.
FIG. 14 schematically illustrates another example of a functional diagram for a formulation microfluidic path device as described herein.
FIG. 15 describes one example of an experiment examining *in vivo* mRNA expression and biodistribution using an exemplary model of an mRNA therapeutic as described herein.
FIGS. 16A-16D are graphs illustrating the therapeutic efficacy of an exemplary therapeutic mRNA vaccine as described herein.
FIG. 17A illustrates the whole-body luciferase expression following injection with stored model mRNA therapeutics manufactured as described herein.
FIG. 17B shows a quantitative example of the expression of the model mRNA therapeutic from FIG. 17A.
FIG. 18 schematically illustrates various times at which filtration may be applied in the methods and apparatuses described herein.
FIG. 19A is a top view of an example of a microfluidic path device including a permeable insert within a fluid-contacting side of a chamber.
FIG. 19B is an example of a section through one region of an example of a microfluidic path device including a permeable insert in one side of a chamber.
FIG. 19C illustrates one example of a portion of a microfluidic path device schematically showing a vacuum cap for bubble removal.
FIG. 19D is a top view of an example of a microfluidic path device ("chip") adapted specifically to synthesize a template for a therapeutic material as described herein.
FIG. 20A schematically illustrates one method of making a synthetic product comprising a synthetic DNA template suitable for *in vitro* transcription, including processing a therapeutic material in a fluid (e.g., an RNA sample) using a microfluidic path device including a permeable insert as described herein.
FIG. 20B schematically illustrates a method of making a synthetic product comprising a synthetic DNA template suitable for *in vitro* transcription, including removing dsRNA from a therapeutic material using a microfluidic path device including a permeable insert as described herein.
FIG. 20C schematically illustrates a method of making a synthetic product comprising a synthetic DNA template suitable for *in vitro* transcription using a microfluidic path device (e.g., "chip") as described herein.
FIG. 21A shows one example of a system including a microfluidic apparatus in a class 5 isolation cabinet within a class 7 space. The system may be configured as a mini-factory.
FIG. 21B illustrates the microfluidic apparatus within the class 5 cabinet.
FIG. 22A is an image of an agarose gel electrophoresis showing the successful generation of a synthetic DNA template for *in vitro* transcription using a PCR based technique as described herein. In this example, the DNA template is a synthetic template for a Luciferase reporter gene including a T7 promoter and a 200 bp poly-A tail.
FIG. 22B is an example of a capillary electrophoresis of the same PCR-based template as in FIG. 22A.

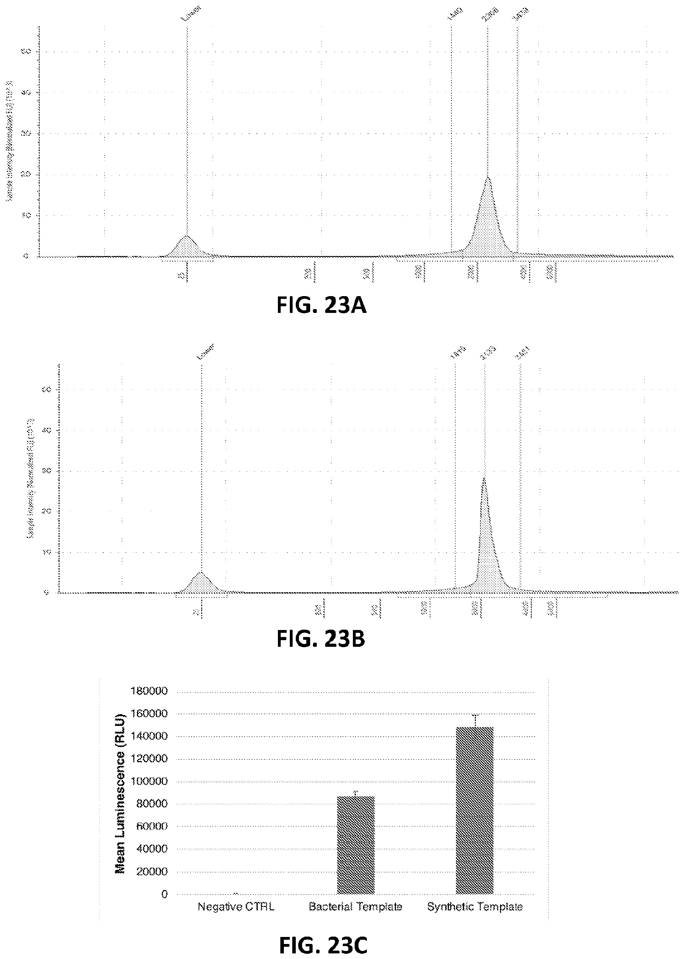

FIGS. 23A-23C illustrate the quality of synthetically produced mRNA template using the PCR-based technique as described herein as compared to bacterially-generated mRNA template. FIG. 23A shows a capillary electrophoresis for mRNA generated using a bacterial templates. FIG. 23B is a capillary electrophoresis for mRNA generated using a synthetic template, as described herein. FIG. 23C shows a comparison of luciferase bioactivity in a mouse dendritic cell line (JAWSII) 6 hours after transfection for the mRNAs shown in FIG. 23A and FIG. 23B, showing a greater bioactivity for the synthetically-generated template.

### DETAILED DESCRIPTION

Described herein are methods and apparatuses for manufacturing therapeutics that may include the use of fully-automated, software-controlled microfluidic apparatuses. In particular, described herein are automated methods and apparatuses for, or including, making a synthetic DNA template for *in vitro* transcription. These methods and apparatuses may include using a polymerase chain reaction (PCR) based method.

These methods and apparatuses may be used for personalized or individualized therapies. Also described herein are apparatuses (e.g., systems, devices, etc.) and methods that include software control of any of the manufacturing operations described herein, including forming the template, *in vitro* transcription, purification of the therapeutic mRNA, concentration of the mRNA, and compounding of the mRNA(s) with one or more delivery vehicle. The software control may allow these methods to be automated so that any, some or all of these operations for manufacturing one or more therapeutic mRNA may be performed rapidly with accuracy and precision. Software control and micro-fluidic precise delivery and transfer of reaction constituents offer the opportunity to increase process control, efficiency and reproducibility whilst substantially reducing or eliminating manual manipulations, reducing facility needs and shortening production cycle times, ultimately leading to lower cost therapies produced just-in-time, if required.

In some of the apparatuses (e.g., systems, devices, etc.) described herein, each batch of therapeutic material may be produced in dedicated, single-use, disposable microfluidic path devices (also referred to herein as biochips), that may be housed inside a microfluidic path device control system (also referred to herein as a control system). The entire production may proceed as a sterile-by-design, closed-path process without contact with the atmosphere. All the production operations may be automated, controlled by the control system to achieve a copy-exact process, regardless of the attributes of the facility housing the system. The production parameters, raw materials and environment data (including a full visual record) may become a part of an extensive, encrypted electronic file secured in the cloud and associated with each production run. In addition, purification operations, as well as a number of QC assays may be performed in-line during the production process in a single fluid flow, allowing anomalies to be detected at an early stage, through process control concepts developed in the semi-conductor industry. By harnessing a fully automated, software controlled approach to manufacturing, personalized and individualized mRNA therapeutics may be manufactured in a cost-effective manner for the benefit of the patients.

These methods and apparatuses may produce mRNA therapeutics synthetically outside of the human body through a synthesis technology known as *in vitro* transcription (IVT). Typically, naked mRNA molecules are large, polyanionic molecules that do not cross the cell membrane, and are rapidly degraded by extracellular nucleases *in vivo.* The methods and apparatuses described herein may produce formulations of mRNA molecules with one or more delivery vehicles, designed to transport the mRNA to a target (tissue, body, region of tissue, etc.). For example, in some examples the delivery vehicle may be a lipid-containing amphipathic delivery vehicle that provides packaging and protection of mRNA cargos during circulation, avoid immune recognition, and may facilitate cellular uptake and release.

In some examples, all or some of the production operations, including template synthesis, IVT, purification, and formulation with delivery vehicles, may be performed in the highly controlled environment of one or more microfluidic path devices, allowing for the optimization of a robust, high-quality and highly reproducible manufacturing process.

### Definitions

As used herein a delivery vehicle may refer to any appropriate nanoparticle. Examples of such nanoparticles may include, but are not limited to, amphipathic nanoparticle such as amino-lipidated peptoids.

As used herein "amplification" may refer to polynucleotide (e.g., DNA) amplification. For example, amplification may be performed entirely within the microfluidic path plate devices described herein. Amplification may include, but is not limited to, multiple displacement amplification (MDA), polymerase chain reaction (PCR) amplification, Loop Mediated Isothermal Amplification. LAMP, Nucleic Acid Sequence Based Amplification, Strand Displacement Amplification, Rolling Circle Amplification, Ligase Chain Reaction, etc.

As used herein, automated and semi-automated may refer to methods and processes that are performed largely without human intervention, and may be under the control of one or more computer processes. Automated methods may be supervised and/or guided by human input.

As used herein, the terms "nucleic acid," "polynucleotide," and "oligonucleotide" may be used interchangeably and refer to deoxyribonuclotides (DNA), ribonucleotides (RNA), and functional analogues thereof, such as complementary DNA (cDNA) in linear or circular conformation. Nucleic acid molecules provided herein can be single stranded or double stranded. Nucleic acid molecules comprise the nucleotide bases adenine (A), guanine (G), thymine (T), cytosine (C). Uracil (U) replaces thymine in RNA molecules. Analogues of the natural nucleotide bases, as well as nucleotide bases that are modified in the base, sugar, and/or phosphate moieties are also provided herein. The symbol "N" can be used to represent any nucleotide base (e.g., A, G, C, T, or U).

As used herein a "cassette" (e.g., a synthetic *in vitro* transcription facilitator cassette) refers to a polynucleotide sequence which may include or be operably linked to one or more expression elements such as an enhancer, a promoter, a leader, an intron, a 5' untranslated region (UTR), a 3' UTR, or a transcription termination sequence. In some examples, a cassette comprises at least a first polynucleotide sequence capable of initiating transcription of an operably linked second polynucleotide sequence and optionally a transcription termination sequence operably linked to the second polynucleotide sequence. The cassette might be provided as a single element or as two or more unlinked elements.

As used herein, "polynucleotide" refers to a nucleic acid molecule containing multiple nucleotides and generally refers both to "oligonucleotides" (a polynucleotide molecule of about 18 and about 25 nucleotides in length) and polynucleotides of about 26 or more nucleotides. Aspects of this disclosure include compositions including oligonucleotides having a length of about 18 and about 25 nucleotides (e. g., about 18-mers, about 19-mers, about 20-mers, about 21-mers, about 22-mers, about 23-mers, about 24-mers, or about 25-mers), or medium-length polynucleotides having a length of about 26 or more nucleotides (e. g., polynucleotides of 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), or long polynucleotides having a length greater than about 300 nucleotides (e. g., polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length. Where a polynucleotide is double-stranded, its length can be similarly described in terms of base pairs.

As used herein, *"in vitro* transcription" or "IVT" refer to the process whereby transcription occurs *in vitro* in a non-cellular system to produce synthetic RNA molecules (synthetic mRNA) for use in various applications, including for therapeutic delivery to a subject. The synthetic RNA molecules (transcription product) generated can combined with a delivery vehicle. Synthetic transcription products include mRNAs, antisense RNA molecules, shRNA, circular RNA molecules, ribozymes, and the like. An IVT reaction may use a purified linear DNA template comprising a promoter sequence and the sequence of the open reading frame of interest, ribonucleotide triphosphates or modified ribonucleotide triphosphates, a buffer system that includes DTT and magnesium ions, and an appropriate phage RNA polymerase.

A "template" or 'double-stranded DNA template" refers to an isolated nucleic acid sequence that comprises the minimal component sequences required for *in vitro* transcription of an inserted open reading frame of interest.

As used herein, "fluid depots" may refer to a storage space for holding a fluid, and may include a vial, a bottle, a bag, a tube, etc. The fluid depot may include a fluid line as an integral portion (e.g., a passage or channel exiting the main body or chamber within the fluid depot).

As used herein fluid power includes both pneumatic and hydraulic. For convenience, the term pneumatic may be include hydraulic and these terms may be used interchangeably.

As used herein a "therapeutic polynucleotide" refers to a polynucleotide (e.g., a therapeutic mRNA) that may be part of a therapeutic polynucleotide composition for delivery to a subject to treat, prevent, improve or otherwise modify the subject's health.

As used herein a "therapeutic polynucleotide composition" may refer to a composition including one or more polynucleotide (e.g., mRNA) encapsulated by a delivery vehicle that may be administered to a subject. An mRNA vaccine is just one example of a therapeutic polynucleotide composition.

As used herein, "free of bacterial DNA" or refers to the absence of bacterial DNA. A material that is substantially free of bacterial DNA may include less than about 0.1%, less than about 0.01%, less than about 0.001%, etc. of bacterial DNA. As used herein "free of endotoxin" refers to the absence of endotoxin. A material that is "substantially free of endotoxin" refers to less than about 0.1%, less than about 0.01%, less than about 0.001%, etc. of endotoxin.

As used herein, "joining" refers to methods such as ligation, synthesis, primer extension, annealing, recombination, or hybridization use to couple one component to another.

As used herein a microfluidic path device or microfluidic path plate device may be equivalently referred to as a chip, cartridge, biochip, microfluidic path plate, etc. and may include a plurality of fluidically interconnected chambers. These chambers may be divided up into fluid-contacting sides and pressure-receiving sides. The pressure-receiving sides may be part of a fluid power circuit, while the fluid-contacting sides may be isolated from the outside atmosphere and may be used to process materials in the microfluidic path device. The microfluidic plate path devices described herein may be generally flat (e.g., having a thickness of less than about 4 cm, less than about 2 cm, less than about 1.5 cm, less than about 1 cm, etc.) and may include a plurality of pressure ports for interfacing with one or more pressure lines to drive and/or control the fluid power circuits in the microfluidic path plate device.

As used herein "on demand" is intended to define when a method or service is performed, and is used in contrast to stored, scheduled, ordered or prepared in advance.

As used herein an optical sensor typically refers to a light-sensing device and may include one or more imaging devices. An optical sensor may include a single lens, camera, stereo cameras, multi-lens cameras, digital still cameras, thermographic camera, CCD, fiber optic, etc.

As used herein a "primer... having a region specific to the synthetic gene of interest" refers to a primer (such as a forward or reverse primer) having a region that hybridizes to the synthetic gene of interest or that hybridizes to a polynucleotide that is complimentary to the synthetic gene of interest. A primer having a region specific to the synthetic gene of interest may be one of a pair of primers used for amplification by PCR.

As used herein "purifying" refers to physical and/or chemical separation of a component (e.g., particles) of other unwanted components (e.g., contaminating substances, fragments, etc.).

As used herein "sealed fluid communication" and "sealed and closed fluidic path" may both refer to the isolation of the material (e.g., a fluid containing a material, such as but not limited to a solution of template, therapeutic polynucleotide, and/or therapeutic polynucleotide composition) from the surrounding atmosphere.

As used herein, a "synthetic gene of interest" refers to a gene of interest that is synthetized *in vitro.* The sequence of the gene may correspond to a patient genetic sequence, such a patient genetic sequence that is derived from a cancerous cell of a patient. The synthetic gene of interest may be synthetized using one or more synthetic techniques. The synthetic techniques may include chemical synthesis (e.g., solid phase synthesis), enzymatic DNA synthesis, etc.

As used herein, "template precursor material" refers to the material necessary to form the template (e.g., a DNA double-stranded template), and may include a synthetic gene of interest, and an *in vitro* transcription facilitator cassette provided as one or more independent elements.

As used herein, 2D purification refers to purification performed within a substantially flat microfluidic path devices (e.g., microfluidic path plate devices) as described herein, and includes using one or more (e.g., two or more, three or more, etc.) type of an absorptive material to remove material (e.g., double-stranded RNA, unreacted nucleotides, unreacted capping reagents, buffer components, etc.), or the like, including materials that are absorptive based on size selection.

Therapies such as mRNA therapeutics may be used for multiple treatment modalities including vaccination, immunotherapies, protein replacement therapies, tissue remodelling/regeneration and treatment of genetic disease by gene editing. In addition to their high potency, mRNA therapeutics also have important advantages related to their rapid development cycle, standardized manufacturing, transient expression and low risk of genomic integration.

In some examples, the mRNA therapeutics described herein may include as an active ingredient in the final drug product an mRNA that encodes an antigen or protein of interest. Robust translation of mRNA requires a functional 5' cap structure. A 5' cap (or 7-methylguanosine cap) consists of a terminal 7-methylguanosine residue that is linked through a 5'-5'-triphosphate bond to the first transcribed nucleotide. Its presence is critical for recognition of the mRNA by the ribosome and protection from RNAses. The poly(A) tail regulates mRNA stability and translational initiation synergistically with the m7G cap by binding the poly(A) binding protein (PABP), which interacts with eukaryotic translation initiation factor eIF4G, and in turn forms a complex with eIF4E. The length of the poly(A) tail may influence the efficiency of the mRNA to protein translation process.

mRNA therapeutics can be broadly divided into at least 5 categories, used for: (i) protein replacement, (ii) vaccines, (iii) Expression of effector proteins, (iv) inducement of loss of function through expression of dominant negative proteins and (v) Gene/genome editing. The methods and apparatuses described herein may provide mRNA therapeutics for any of these categories (or more than one).

The methods and apparatuses described herein may formulate the mRNA therapeutic to provide packaging and protection of mRNA cargos during circulation, avoid immune recognition, localize drug product in desired tissues, and facilitate cellular uptake and release, while avoiding toxicity or immunogenicity concerns which could limit repeated dosing.

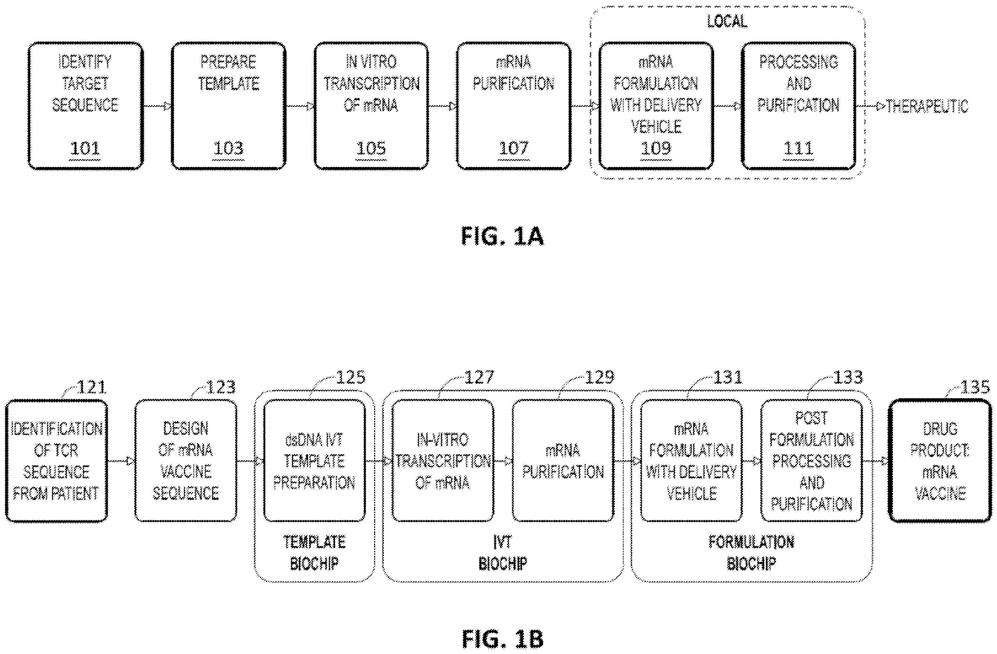

In general, a method of manufacturing an mRNA therapeutic (including, but not limited to a patient-specific T-cell lymphoma vaccine drug product) may involve any or all of the operations that are schematically represented in FIG. 1A, and include identification of the target protein and design of the mRNA sequence 101, preparation a double stranded DNA template as for the target sequence 103. This sequence may be used to generate the mRNA for the *in vitro* transcription (IVT) reaction 105, to synthesize mRNA. This therapeutic mRNA may then be purified to remove process impurities and filtration to generate the drug substance 107. The therapeutic mRNA may then be formulated with the delivery vehicle 109 (including in some example with adjuvant and delivery vehicle components to form amphipathic nanoparticle). The formulation may then be processed and purified to generate drug product 111 that may be used for delivery to the patient. As described above, in some examples some of these operations may be performed remotely (e.g., 101-107) and some locally (e.g., 109, 111); in some examples they may all (e.g., 103, 105, 107, 109, 111) be performed locally.

As a specific example of one example, FIG. 1B shows an exemplary process for manufacturing a patient-specific T-cell lymphoma vaccine drug product. In FIG. 1B, the process may include identification of a clonally expanded TCR sequence (idiotype) expressed by the lymphoma cells 121. The process may also include designing the mRNA vaccine sequence 123, and preparing a double stranded DNA template for the IVT reaction 125. The template may be used for the IVT reaction to synthesize mRNA 127, and this therapeutic mRNA may be purified to remove process impurities and filtration to prepare the therapeutic mRNA as the drug substance 129. The therapeutic mRNA may then be formulated with adjuvant and delivery vehicle components to form amphipathic nanoparticles 131. Post-formulation processing 133 may then be performed to generate drug product, such as a therapeutic mRNA vaccine 135.

Any of these manufacturing operations may be optimized to be performed using an automated microfluidic path device control system as described herein. For example, a DNA template production may take place in one or more microfluidic path devices; in the example shown in FIG. 1B, a template microfluidic path device (e.g., template biochip) may be used. In this same example, the operations of in-vitro transcription of the mRNA and the purification of that material to generate the drug substance may be performed on an IVT microfluidic path device (e.g., an IVT biochip), and drug product formulation operations may be done on a formulation microfluidic path device (e.g., formulations biochip). These microfluidic path devices may contain the input ports, metering valves, reaction chambers, and purification structures required to perform each operation in the manufacturing process.

### Apparatus

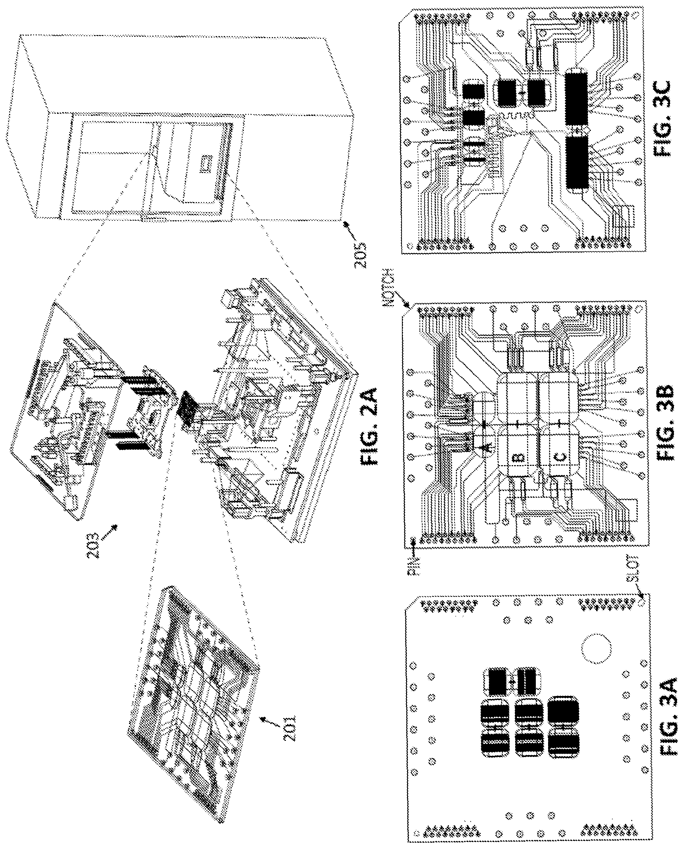

The methods described herein may generally be performed using an apparatus that may be used with and/or may include one or more microfluidic path devices (e.g., biochips), and a system (e.g., a microfluidic control system) that is configured to control operations in the microfluidic path device. These apparatuses may be referred to herein as microfluidic apparatuses, microfluidic control apparatuses, microfluidic path device control system, microfluidic control systems, or microfluidic systems. A microfluidic path devices (also referred to as a microfluidic path plate device) may be placed within the microfluidic control system and may operate in a closed path manner that prevents exposure of the component parts of some, or more preferably nearly all or all of the manufacturing components within the system to the atmosphere. In particular, the portions of the apparatus that contact the fluid(s) within the system are prevented from exposure to atmosphere. FIG. 2A shows one example of a microfluidic path device control system that includes: a microfluidic path device management system 203 (including hardware for holding the microfluidic path device, applying positive/negative pressure to operate microfluidic operations in the microfluidic path device, heating/cooling all or regions of the microfluidic path device, detecting one or more features from the microfluidic path device and/or recording operations performed on the one or more microfluidic path device), a controller (not shown), and a refrigerated container 205 (e.g., an ISO class 5 cabinet). This system may be used or may include one or more microfluidic path devices 201.

A microfluidic apparatuses may be microfluidic apparatuses for forming a therapeutic polynucleotide (e.g., an mRNA therapeutic). The apparatus may include: a seating mount for removably holding a microfluidic path plate device, a plurality of pressure lines; a plurality of fluid vials, wherein each fluid vial either comprises a fluidic line or is configured to couple with the fluidic line, wherein each fluidic line and at least a subset of the pressure lines are configured to be biased against the microfluidic path plate device held in the seating mount to form a closed fluid path; and a controller configured to control the application of pressure through the pressure lines to drive fluidic movement in the microfluidic path plate device when the microfluidic path plate device is held in the seating mount, wherein the controller is configured to direct the synthesis of a synthetic template, direct an *in vitro* transcription (IVT) reaction using the template to form a therapeutic polynucleotide, and direct purification of the therapeutic polynucleotide in one or more microfluidic path plate devices held in the seating mount.

A microfluidic apparatus (e.g., a microfluidic apparatus for forming a therapeutic polynucleotide, such as a therapeutic mRNA) may include: a seating mount for removably holding a microfluidic path plate device; a plurality of pressure lines; a plurality of fluid vials, wherein each fluid vial either comprises a fluidic line or is configured to couple with the fluidic line, wherein each fluidic line and at least a subset of the pressure lines are configured to be biased against the microfluidic path plate device held in the seating mount to form a closed fluid path; and a controller configured to control the application of pressure through the pressure lines to drive fluidic movement in the microfluidic path plate device when the microfluidic path plate device is held in the seating mount, wherein the controller is configured to determine the contents of the fluid vials, transfer sub-microliter amounts of material from the fluid vials to one or more reactors in the microfluidic path plate device held in the seating mount, direct the synthesis of a synthetic template, direct an *in vitro* transcription (IVT) reaction using the template to form a therapeutic polynucleotide, and direct purification of the therapeutic polynucleotide in one or more microfluidic path devices held in the seating mount.

The controller may be configured to perform any of the method described herein, an in particular may be configured to receive inputs (e.g., optical input, pressure input, temperature/thermal input, etc.) and process the input to control movement of fluid in the microfluidic path device, temperature (including thermocycling) of various regions of the microfluidic path device, rinsing/combining, opening/closing of valve of the microfluidic device, detection of the microfluidic device, etc. The controller may include one or more microprocessors, communication circuitry, memory, etc. The controller may comprise firmware, hardware and/or software.

Any of these apparatuses may include a one or more (e.g., a plurality) of optical sensors arranged around the seating mount and reagent storage frame to monitor fluid levels within the reagent storage frame and fluidic movement in the microfluidic path device when the microfluidic path device is seated in the seating mount. Alternatively or additionally, the optical sensor(s) may be present on the bottom of the apparatus (e.g., beneath the seating mount) and may be directed upwards to detect fluid amounts, movement, etc.).

The methods and apparatuses described generally include one or more fluid power circuits to move material (liquid material) between the fluid chambers (depots, fluid-contacting sides, reactors, etc.) and channels of the microfluidic path device or within the microfluidic path device, and in some cases between the microfluidic path device and the fluid depots (vails, bottles, containers, etc.) within the apparatus. A fluid power circuit may be a hydraulic or pneumatic circuit that may include the microfluidic device, and in particular one or more pressure channels and pressure-receiving sides of the chambers in a microfluidic device. The fluid power circuits may also be referred to as microfluidic power circuits. A single microfluidic chip may include multiple fluid power circuits; the fluid power circuits may also include one or more pressure lines and the interface between the pressure lines of the microfluidic control apparatus and the one or more microfluidic chips within the microfluidic path device. One or more fluid power circuits may share components (valves, pressure lines, vacuum caps, etc.) with other, overlapping fluid power circuits. Furthermore, for the same of convenience, it should be understood that where the term "pneumatic" is used, a general fluid power circuit (e.g., hydraulic and/or pneumatic) may be used instead or additionally. The fluid material being driven by the fluid power line may be any appropriate fluid (e.g., gas or liquid, such as air, water, oil, etc.).

Also described herein are microfluidic path devices for processing therapeutic polynucleotides in a closed path (e.g., closed-path microfluidic path devices). As mentioned, these microfluidic path devices may be referred to herein as microfluidic chips, microfluidic path plate, process chip, biochip, process plate, etc. In general, the microfluidic path device may be microfluidic path plate devices, which may be substantially flat plate-like structures; these structures may be relatively thin (e.g., less than a few mm thick, e.g., between about 0.5 and about 20 mm thick, between about 0.5 and about 15 mm thick, between about 0.5 and about 10 mm thick, etc.). The microfluidic path devices described herein may generally be at least partially transparent, and in particular, may be transparent on the top of the microfluidic path device, so that one or more optical sensors (cameras, CCD, fiber optics, etc.) may be used to sense, detect, monitor, record, etc. action, including fluid movement and/or movement of the elastic layer, with the microfluidic path device as it is used by the microfluidic apparatuses described herein.

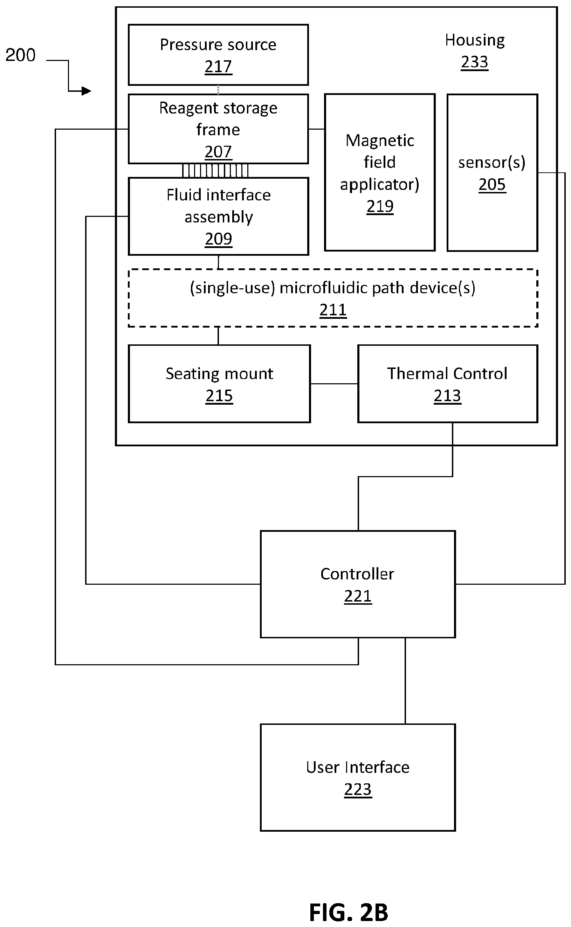

FIG. 2B is a schematic illustration of one example of a microfluidic path device control system that may be used as described herein. In this example, the apparatus includes a housing 233 enclosing a seating mount 215 which can hold one or more microfluidic path devices 211, which may be single use devices. The housing may be a chamber, enclosure, or the like, which may include a lid or opening; when closed it may be sealed. The housing may enclose a thermal regulator and/or may be configured to be enclosed in a thermally-regulated environment (such as a refrigeration unit, etc.). The housing may form an aseptic barrier. In some examples the housing may form a humidified or humidity-controlled environment.

The seating mount 215 may be configured to secure the microfluidic path device using one or more pins or other components configured to hold the microfluidic path device in a fixed and predefined orientation.

In some examples, a thermal control 213 may be located adjacent to the seating mount 215, to modulate temperature to the one or more microfluidic path devices 211. The thermal control may include a thermoelectric component (e.g. Peltier device) and/or one or more heat sinks for controlling the temperature of all or a portion of the microfluidic path device. In some examples, more than one thermal control may be included, for separately regulating the temperature of one or more regions of the microfluidic path device. The thermal control may include one or more thermal sensors (e.g., thermocouples, etc.) that may be used for feedback control of the microfluidic path device and/or thermal control.

In FIG. 2B, a fluidic interface assembly 209 couples the liquid reagents and/or pressure (e.g., gas) with a microfluidic path device 211 held in the seating mount 215, and may assist in delivery of fluidic materials as well as positive /negative gaseous pressure, from the pressure source 217, to the interior of the microfluidic path device 211. The fluid interface assembly may optionally assist in securing the microfluidic path device(s), as described in greater detail below. The fluid interface assembly may be removable coupled to the apparatus (and may be removed or a portion may be removed) for sterilization between uses.

A reagent storage frame 207 may be configured to contain a plurality of fluid sample holders, each of which may hold a fluid vial configured to hold a reagent (e.g., nucleotides, solvent, water, etc.) for delivery to the microfluidic device 211 or, alternatively, a fluid vial may be configured to receive a product from the interior of the microfluidic path device 211. The reagent storage frame may be referred to as a reagent rack. In some examples, the reagent rack includes a plurality of pressure lines and/or a manifold configured to divide one or more pressure sources 217 into a plurality of pressure lines that may be applied to the microfluidic path device and may be independently or collectively (in sub-combinations) controlled. Alternatively, the fluid depots (vials, etc.) may be configured to directly secure and seal against the microfluidic path device(s).

The fluid interface assembly may include a plurality of fluid lines and/or pressure lines and may include a biased (e.g., spring-loaded) holder or tip that individually and independently drives each fluid and/or pressure line to the microfluidic path device when it is held in the seating mount 215 (or, as mentioned, alternatively the device may directly be spring-mounted). The tubing, e.g., the fluid lines and/or the pressure lines, may be part of the fluid interface assembly and or may connect to the fluid interface assembly. In some examples the fluid lines comprise a flexible tubing that connects between the reagent storage frame, via a connector that couples the vial to the tubing in a locking engagement (e.g., ferrule) and the microfluidic path device. The ends of the fluid paths, in some examples the ends of the fluid lines/pressure lines, may be configured to seal against the microfluidic path device, e.g., at a sealing port formed in the microfluidic path device, as described herein. For example, the ends of the fluid lines may cut or formed to be flat (perpendicular inside view). The vials may be pressurized (e.g., > about 1 atm pressure, such as about 2 atm, about 3 atm, about 5 atm, etc.) to via the connector which may also connect to the pressure source. For example, the fluid vials may be pressurized to between about 1 and about 20 psig (e.g., about 5 psig, about 10 psig, about 20 psig, etc.). Negative or positive pressure may be applied; for example, a vacuum (e.g., about - 7 psig or about 7 psia) may be applied to draw fluids back into the vials (e.g., the depots) at the end of the process. In general the fluid vials may be driven at lower pressure than the pneumatic valves, which may prevent or reduce leakage. In some examples the difference in pressure between the fluid and pneumatic valves may be between about 5 psi (e.g., about 7 psi, about 10 psi, about 12 psi, about 15 psi, about 20 psi, etc.).

Each vial may be coded (e.g., by an identifier that may be read by one or more sensors, as described below). The controller may monitor the fluid level and therefore the amount of each material in the fluid interface assembly.

The apparatus may also include a magnetic field applicator 219, which may be configured to create a magnetic field at a region of the microfluidic path device 211. One or more sensors 205, which may be optical sensors, may be part of the apparatus, and may sense one or more of a barcode, a fluid level within a fluid vial held within the reagent storage frame, and fluidic movement within the microfluidic path device 211 when the device is mounted within the seating mount 215.

The sensors may make measurements of the process on the device, e.g., by measuring an optical indicator. In some examples visual/optical markers may be used to estimate yield. For example, fluorescence may be used to detect process yield or residual material by tagging with fluorophores. Alternatively or in addition, dynamic light scattering may be used to measure particle size distributions within a portion of the microfluidic path device (e.g., such as a mixing portion). In some examples, the sensor measurements may be done using one or two optical fibers to convey light (e.g., laser light) in and detect an optical signal coming out. An instrument package may be mounted remotely from the device. Such non-contact sensing may be preferred.

In any of the methods and apparatuses described herein, the sensors (e.g., video sensors) may record all activity on the microfluidic path device (e.g., chip or cartridge). For example, an entire run for synthesizing and/or processing a material (such as a therapeutic RNA) may be recorded by one or more video sensors, including a video sensor that may visualize the microfluidic path device, e.g., from above. Processing on the microfluidic path device may be visually tracked and this record may be retained for later quality control and/or processing. Thus, the video record of the processing may be saved, stored and/or transmitted for subsequent review and/or analysis.

The internal portion of the apparatus, e.g., within the housing 233, may be further configured to be sterilizable. In particular, portions of the apparatus may be removed and individually sterilized. Sterilization may be performed, e.g., by UV irradiation, or any other method of sterilization that may be required to limit contamination or to meet regulatory requirements. The apparatus including the housing may be housed within a High Efficiency Particulate Air (HEPA) filtered environment. The apparatus including the housing may be housed within a temperature controlled enclosure. In addition, the apparatus itself may include one or more regions that are temperature controlled. In any of the apparatuses described herein, the apparatus may include (e.g., within the housing) a temperature controlled region for storing reagents and/or for storing mRNAs (e.g., therapeutic mRNAs), e.g., at a storage temperature (e.g., a temperature between about -10 degrees C and about 20 degrees C, such as about 10 degrees C, about 4 degrees C, about -10 degrees C, etc.). Any of these apparatuses may include a library of manufactured mRNAs that may be compounded individually or in combination with one or more additional mRNAs and a delivery vehicle.

As mentioned above, the microfluidic path device controller system may be controlled by controller 221, including to apply pressure through the microfluidic path device 211 to at least drive fluidic movement. The controller may be completely or partially outside of the housing. The controller may be configured to include user inputs/outputs. For example, the user interface 223 of the system may permit easy operation and direction of the apparatus and microfluidic path device(s).

Any of the apparatuses described herein may include all or some of the components shown in FIG. 2B; not all components may be necessary. In FIG. 2B, only some of the connections between components are shown; additional (or alternative) connections may be used.

A microfluidic path device control system may support all the production activities inside the microfluidic path device such as supply of reagents, fluid control, temperature control, mixing, purification and process monitoring. Manufacturing activities on the microfluidic path device control system may be accessed and controlled through application software.

The microfluidic path devices may be configured to include one or more reactors for the manufacturing operations which are performed to precisely prepare a therapeutic (e.g., a therapeutic mRNA) material. The same microfluidic path device may operate on one or more microfluidic path devices, either in series and/or in parallel, and without interrupting the continuous-path nature of the microfluidic path device control system. For example, when manufacturing a therapeutic using multiple processing operations performed in multiple reactors using multiple microfluidic path devices, the fluid product(s), including partial products from one microfluidic path device may be transferred to one or more additional microfluidic path device in a closed-path manner by the apparatus, including by moving fluid containing the microfluidic path device product(s) into a storage depot portion of the microfluidic path device control device.

Each microfluidic path device may be configured to include one or more reactors for processing during the manufacturing processes. For example, FIGS. 3A-3C illustrate three examples of microfluidic path devices. These examples illustrate three distinct types of microfluidic path device: a template microfluidic path device (FIG. 3A), an *in vitro* transcription (IVT) microfluidic path device (FIG. 3B) and a formulation microfluidic path device (FIG. 3C). Each of these microfluidic path device examples may be configured to include features to perform a set of unit operations in a controlled and highly reproducible manner.

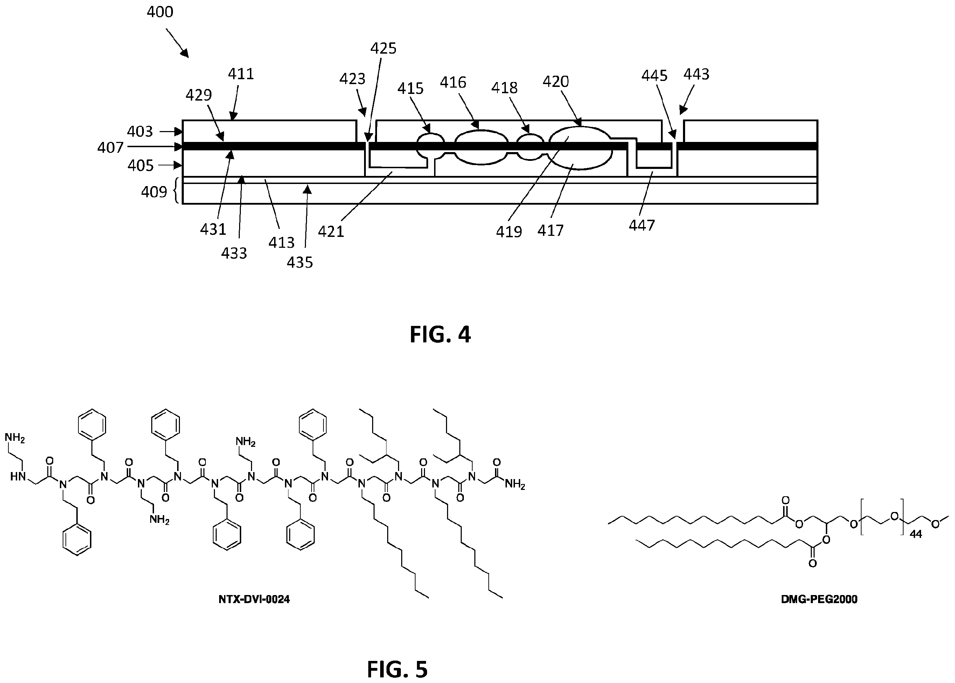

In some examples, a microfluidic path device may be configured as multilayered structure composed of two more rigid layers with a flexible membrane sandwiched between the two ridged layers. FIG. 4 illustrates a sectional view (transverse to the plane of the microfluidic path device) through one example of a microfluidic path device having multiple layers that form the reactors for processing the therapeutic as described herein. The reactors may include seals, channels, valves, and chambers, including pumping chambers formed from the multiple layers. For example, a microfluidic path device may be formed of two or more rigid or semi-rigid plates 403, 405 and at least one elastic layer 407. The elastic layer 407 may be a sheet of elastic material that is liquid-impermeable. The elastic layer maybe somewhat gas permeable, or may be treated to be more or less gas permeable, including in various regions. Although a single continuous sheet of elastic material may be used, in some examples multiple elastic materials sheets may be used, or the 'sheet' may be formed of sections of multiple sheets. The layers and the elastic sheet may be laminated together. In general, chambers for holding, valving and/or pumping fluid may be formed in the plates on either side of the elastic layer so that the elastic layer bisects the chambers into a liquid containing side and a pressure (e.g., gas) applying side. The overall volume of chamber(s) may be constant, and may be formed into both the first (e.g., upper) plate and the second (e.g., lower) plate, but this volume may be divided into the pressure side and the liquid side. By applying positive or negative pressure into the pressure side, the elastic sheet may be deformed to make reduce (down to zero, closing the chamber off) the volume of the liquid containing side or to increase the volume of the liquid containing side (to a predetermined maximum). The pressure applying side of the chamber may be connected, e.g., via a pressure port 443 in the upper plate 403 connecting to a pressure channel 447, for applying negative or positive pressure to the pressure-receiving side 419 of one or more chambers. The liquid containing side 417 opposite the pressure-applying side of each chamber may be connected via a fluid channel 421 to a fluid port 423. Both the fluid port and the pressure port may be formed by an opening into the upper plate 403 and the elastic layer 407, allowing a sealed connection that is isolated from the atmosphere even when there are multiple different input lines as the pressure line is pushed into the elastic layer 407 that is supported on the underside of the port by the opposite rigid or semi-rigid layer(s), 405, 409.

In FIG. 4, the microfluidic path device 400 includes a first (e.g., upper) plate 403 having a first (e.g., top or upper) surface 411 and a second (bottom or lower) surface 429 and a thickness between the two. The first surface 411 may form an exposed outer surface. The microfluidic path device also includes a second plate 405 having a first (e.g., upper or top) surface 431 and a second (e.g., lower or bottom) surface 433 and a thickness therebetween. An elastic layer 407 is sandwiched between the second surface 429 of the first plate 403 and the first surface 431 of the second plate 405. A third plate 409 is coupled to the second plate on the second surface 433 of the second plate, either directly or indirectly. The third plate 409 also has a first (e.g., upper or top) surface and a second (lower or bottom) surface and a thickness therebetween. The second surface of the third plate may form a bottom surface of the microfluidic path device. Any of the plates may be formed of multiple layers, which may be laminated or otherwise connected together. For example, in FIG. 4, the third plate 409 includes an optional second elastic layer 413 which may help couple the third plate to the second plate; the second elastic layer 413 in this example forms the first surface 435 of the third plate 409. The layers and plates shown in FIG. 4 may not be to scale (e.g., the elastic layer 407 may be thinner relative to the plates).

The microfluidic path device 400 shown in FIG. 4 may also include a plurality of chambers 415, 416, 418, 420 each having a fixed volume. These chambers are formed by cut-out regions (e.g., rounded/curved cuts) into the second (bottom) surface 429 of the first plate 403 and the first (upper) surface 431 of the second plate 405; the elastic layer 407 bifurcates these chambers 415 so that each includes a liquid containing side 417 and a pressure (e.g., gas containing) side 419. The microfluidic path device 400 may also include multiple liquid (e.g., fluid) channels. In FIG. 4, a single fluid channel 421 is shown extending from a fluid port 423 passing through the thickness of the first plate 403, to a fluid channel opening 425 through the elastic layer 407 and through much of the thickness of the second plate 405 down to the bottom surface 433 of the second plate where a length of the liquid channel 421 running parallel to the bottom surface of the third plate is formed in the bottom surface 433 of the second plate, and bounded by the upper surface of the third plate 409.

In regard to the fluid port 423, the diameter of the opening into the first plate 403 forming the fluid port 423, which extends through the thickness of the first plate, may be larger than the diameter of the fluid channel opening 425 which extends through the elastic layer 407 and into the liquid (e.g., fluid) channel 421. The fluid channel opening 425 may be centered relative to the bottom of the fluid port opening and may be offset from the walls of the fluid port opening by at least the expected wall thickness of the fluid line or fluid line coupling interface that will connect to the fluid port.

The fluid channel 421 connects to the liquid containing side 417 of a first chamber 415. This first chamber may be configured as a valve, which has a relatively low retaining volume (fixed volume), but can be fully opened or closed by the movement of the elastic layer 407.

The microfluidic path device 400 also includes a plurality of pressure channels that may be independently controlled to apply positive and/or negative pressure. In FIG. 4, a single pressure port 443 is shown, connected to the fourth chamber 420, although each of the chambers 415, 416, 418 may be connected to a separate pressure port and pressure channel for independently operating and controlling the movement of the portion of the elastic layer 407 bifurcating these chambers, to valve, and/or pump each chamber independently. In some examples the pressure ports may be shared between multiple chambers. In FIG. 4 the pressure (e.g., gas) port 443 is similar to the fluid (e.g., liquid) port 425, and includes an opening completely through the first plate 403, down to the exposed elastic layer 407, to an opening through the elastic layer forming a pressure (e.g., gas) channel opening 445. The pressure channel opening 445 is continuous with a pressure (e.g., gas) channel 447 that extends from the pressure port 443, passing through much of the thickness of the first plate 403, and in a cut-out channel along the bottom of the second plate (or alternatively into a cut-out region in the top of the third plate) and back up through the second plate and the elastic layer 407, to a region of the pressure channel within the first plate that connects to the pressure (e.g., gas) containing portion 419 of the fourth chamber 420. As described for the similar fluid (e.g., liquid) port, the diameter of the pressure port 443 passing through the thickness of the first plate 403 may be larger than the diameter of the pressure channel opening 445 through the elastic layer 407, and may be centered or offset by greater than the wall thickness of a pressure line or pressure line coupling interface that will connect to the pressure port.

In the section through a microfluidic path device 400 shown in FIG. 4, there are multiple connections to other fluid (e.g., liquid) lines, fluid ports, pressure lines and pressure ports that are not shown, as they may be out of the plane shown. For example, in FIG. 4 the liquid containing side or portion 417 of the fourth chamber may be connected to additional valves (chambers) and/or channels, including, e.g., an exit channel extending from the liquid containing side 417. An additional chamber (e.g. configured as a valve), no shown may be formed as described above. In some examples, an exit channel may deliver fluid from the one or more chamber through another fluid port (not shown) to a fluid receiving depot, e.g., a vial, tube, etc. This receiving depot may be held in the reagent storage frame.

In general, this configuration of the microfluidic path device and the microfluidic apparatus is configured so that multiple, complex operations may be executed by the apparatus on the microfluidic path device in a fully enclosed (sealed and protected from atmosphere) manner, without requiring manual intervention. Fluid may be metered using the fixed-volume chambers and moved, mixed, filtered, etc. by applying pneumatic pressure to deflect regions of the elastic layer.

In some examples, the chambers within the microfluidic path device may be configured as mixing chambers, for mixing fluid within the microfluidic path device. In some examples the chamber(s) may be configured as purification chambers, which may include a filter material. In some examples one or more chambers may be configured as a concentrator for concentrating the therapeutic material(s).

Although the various microfluidic path devices may have different arrangements of channels, ports and chambers, they may also share a similar basic architecture and a number of functional elements that can be used in different configurations to carry out different protocols. Functional elements include input ports, metering valves, pumps, reaction chambers, mixing structures and purification structures, as described above.

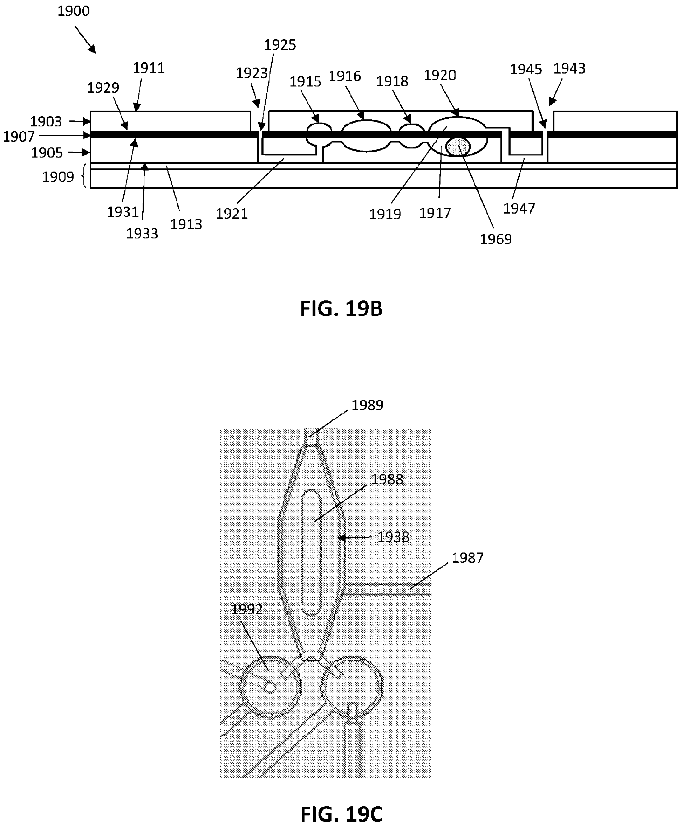

Any of these microfluidic path apparatuses may include one or more bubble removal chambers, or any of the chambers of the fluid-contacting side of the chamber may be configured as a bubble removal chamber, in which bubbles within the fluid of the fluid-containing side may be removed. A bubble removal chamber may be referred to as a vacuum cap, and may generally be configured to apply negative pressure on the opposite side of the membrane while fluid is held within the fluid-contacting side of the chamber. The membrane may be at least partially gas-permeable, as mentioned. FIG. 19C shows an example of a bubble removal chamber. All, or more preferably a portion 1988 (e.g., just a cap region), of the membrane dividing the chamber may be in contact with the vacuum through a vacuum line 1987, e.g., in the upper surface or upper plate of the device, as shown in FIG. 19C. In operation, the vacuum cap 1938, may remove or reduce a bubble within the line by holding fluid within the fluid-contacting side of the chamber and applying a negative pressure on the upper (pressure receiving) side of the chamber. The membrane dividing the chamber into the fluid-contacting side and the pressure-receiving side may be gas permeable, so that the negative pressure may remove gas from the liquid (fluidic) side by drawing gas (e.g., air, nitrogen, etc.) through the membrane overlying the fluid path. For example, the membrane (or the region of the membrane in the vacuum cap may be, e.g., PolyDiMethylSilicone (PDMS) elastomer film that is sufficiently gas permeable to allow remove gas from the liquid side of the membrane. Fluid chambers having a fixed volume (e.g., formed between the first plate and the second plate) as described herein may include or be coupled to one or more bubble removal chambers (vacuum caps) and/or may be configured as bubble removal chambers. In some examples the portion of the elastic layer disposed between the first and the second surfaces forming the chamber, which divides the fluid-contacting side, e.g., in the second surface (and/or second plate) and a pressure-receiving side in the first surface (and/or first plate) may be only minimally (or not at all) deflected. For example, the upper, pressure-receiving side, may be minimally spaced, and/or nearly flush with the relaxed membrane (e.g., flat), while the fluid-contacting side is concave and extends into the second surface (second plate). A controller may hold fluid within the vacuum cap region, e.g., by blocking valves on either or both sides (entrance and exit) of the vacuum cap, e.g., by applying positive pressure to the pressure-receiving side of the valve, and may apply negative pressure to the pressure-receiving side of the vacuum cap. The absolute amount of negative pressure applied (e.g., the magnitude of the negative pressure) may be less than that applied to deflect the membrane (e.g., less than the absolute value of the positive pressure applied to close the valve, and/or pump). Alternatively, in some examples the membrane may be configured to be deflected (e.g., deflected up), against the first surface and/or plate, e.g., to draw fluid into the enlarged fluid-contacting side of the chamber from an input 1989. The membrane may be held by the applied negative pressure against the first, upper surface, allowing gas bubbles (e.g., air bubbles) to be removed. The controller may hold fluid in the vacuum chamber for a period sufficient to remove all or some gas (e.g., about 1 second or more, about 5 seconds or more, about 10 seconds or more, about 20 seconds or more, about 30 seconds or more, about 1 minute or more, about 1.5 minutes or more, about 2 minutes or more, about 5 minutes or more, between about 1 second and about 5 minutes, between about 2 seconds and about 5 minutes, between about 5 seconds and about 5 minutes, etc.). In FIG. 19C, the pressure may be applied through the pressure line 1987 in communication with the pressure-receiving sides of the chamber formed between the first and second surface (e.g., first and second plate) of the device. The vacuum cap 1938 may be valved by one or more valves 1992. Fluid may exit the fluid-contacting side from a fluid line 1989 at an opposite side of the vacuum cap.

The fluid-contacting side of the chamber of the pressure cap (as with the valves and reactors described herein) may be in fluid communication with a fluid port that fluidly connect with the fluid-contacting side of each of the chambers via one or more fluid channels, which may be in the second surface and/or plate. The pressure-receiving side of the vacuum cap may be in fluid communication with a pressure port extending through the first surface/plate (e.g., and into the surface/plate) to fluidly connect with the pressure-receiving port or side via a pressure channel extending through the second plate and along the first plate, as described herein.

Any of the microfluidic path devices described herein may be microfluidic path plate devices, in which the device is substantially thin, as described above. Thus processing in/on the plate may be performed in substantially two dimensions (2D), including purification of any polynucleotides (e.g., mRNA). Purification of the polynucleotides in 2D is particularly advantageous compared to prior art techniques, which may require the use of columns and may involve operations that are difficult or impossible to perform in a closed path environment and/or in small volumes as described herein.

In addition, as illustrated in FIG. 4, the fluid-contacting sides (and/or the pressure-receiving side) of each chamber may be configured to so that the elastic layer seats flush and without gaps to the fluid-contacting side in the second surface when a positive pressure in the pressure-receiving side drives the elastic layer against the fluid-contacting side. In some examples the fluid contacting sides and/or the pressure-receiving sides may be concave. The concavity may have a somewhat shallow, oval cross-section to permit the elastic layer to readily seat flush against the wall of the fluid contacting side (and/or pressure-receiving side). The elastic layer may push (e.g., seat) against the wall of the chamber so that there is no dead retention portion of the chamber (e.g., of the fluid-contacting side).

The microfluidic path devices may interface with the microfluidic path device control system through a set of spring-loaded connections for both the reagents, as well as pneumatic lines used for managing fluid movement and valve control. The reagent and gas lines may be sealed by pressure against an elastomeric layer embedded in the microfluidic path device that creates a completely sealed path from reagent vials into the microfluidic path device and from the microfluidic path device to the export vials. The sealed path may be maintained through all of the reactions inside the microfluidic path device(s), effectively precluding any contact with the atmosphere and minimizing the risk of contamination.

The microfluidic path device control systems described herein may provide an, aseptic controlled environment, and may include an interface for loading reagents and retrieving outputs. In any of the apparatuses (e.g., systems) described herein, the apparatus may include an enclosure that provide a controlled environment; this enclosure may also be placed within a controlled environment. For example, the enclosed apparatus may be a class 5 environment that may be placed within a class 7 environment.

The microfluidic path device control systems of the microfluidic path device(s) and may provide a single-step connection to all the actuators. These control systems may also scan all the reagent and microfluidic path device identifiers (e.g., barcodes), and may monitor fluid levels. In general, these microfluidic path device control systems may automate all or some of the microfluidic path device functions and may generate a visual recording of all process operations that may be monitored (such as for optical quality control analysis, e.g., of intermediate process outputs), stored, transmitted, or later reviewed.

As mentioned above, a microfluidic path device control system may include a microfluidic path device management system that includes the hardware, such as a nest (microfluidic path device holder) that may be engineered such that microfluidic path devices are correctly aligned can only be inserted in a single orientation. This may be managed, e.g., through two pins and/or a notch in the nest that is matched by the shape of the microfluidic path device. The microfluidic path device management system (control system) also includes vial racks to hold the reagent and export vials, a downward looking camera that records all liquid and valve movements, and product export. Side cameras on rails to capture barcodes and detect fluid levels, and a robotic arm with magnets for bead manipulation. The microfluidic path device is held in place with a vacuum chuck which ensures good contact with a Peltier device for temperature management. Once the microfluidic path device is in place, mating with all the connectors is achieved in a single operation by lowering the top part of the microfluidic path device management system through a dowel pin guided system.

As mentioned, the microfluidic path device control system may include a control panel, which may be an interface for all electronic devices (CPU, Ethernet RIO device controller, etc.) as well as the valves and manifolds for pneumatic control, and pressure regulators. Any of these systems may also include a refrigerated cabinet or chamber (e.g., an ISO class 5 safety cabinet) that behaves like a biosafety hood providing a microbiologically safe enclosure through HEPA air filtering and air flow management. In addition, this may ensure that all reagents are kept at the correct temperature through the manufacturing process. The cabinet may also be equipped with UV lamps for sterilization of the microfluidic path device and all the internal microfluidic path device management system components. The microfluidic path device control system may reside inside an environment (e.g., a 6 ft x 6 ft ISO class 5 mini environment) that is itself in an ISO class 7 room. Operator and system interactions, including loading reagent vials and microfluidic path device(s) may all be performed following aseptic best practices.

### Delivery Vehicle

The methods and apparatuses described herein are compatible with a broad array mRNA delivery vehicles. For example, the delivery vehicle may be compatible with electroporation and gene gun, viral delivery through adenovirus (AV) or adeno-associated virus (AAV), exosomes and liposomes, encapsulation by cationic polymers and formulation with lipid nanoparticles (LNPs).

### Manufacture of Therapeutics

Described herein are methods, and apparatuses (e.g., devices, and systems) for making therapeutics. These methods and apparatuses may be used to manufacture patient-specific therapeutics, in a very rapid time period. In particular, these methods and apparatuses may be used to manufacture therapeutics based on polynucleotides, such as mRNA, as described above. As part of this process, the methods and apparatuses may perform some or all of the operations described above, including producing the IVT DNA template, performing the IVT reaction to produce a therapeutic mRNA, purifying the therapeutic mRNA, formulating the mRNA with a delivery vehicle to form the therapeutic composition and post-formulation processing of the final drug product.

### Producing the IVT template

The methods for making DNA templates and in particular for making synthetic DNA templates described herein may be especially useful for making better, more scalable, faster and safer vaccines and therapeutics. The use of synthetic template for mRNA synthesis by IVT is beneficial in numerous ways, including preventing potential microbial contamination. Solutions containing the synthetic DNA templates are generally free from contaminating cells, free from cell extracts, and free from endotoxins from cells. These solutions may be especially well suited to be part of a vaccine for injecting into a patient with virtually no risk of toxicity due to contaminating cells, cell extracts, or endotoxins.

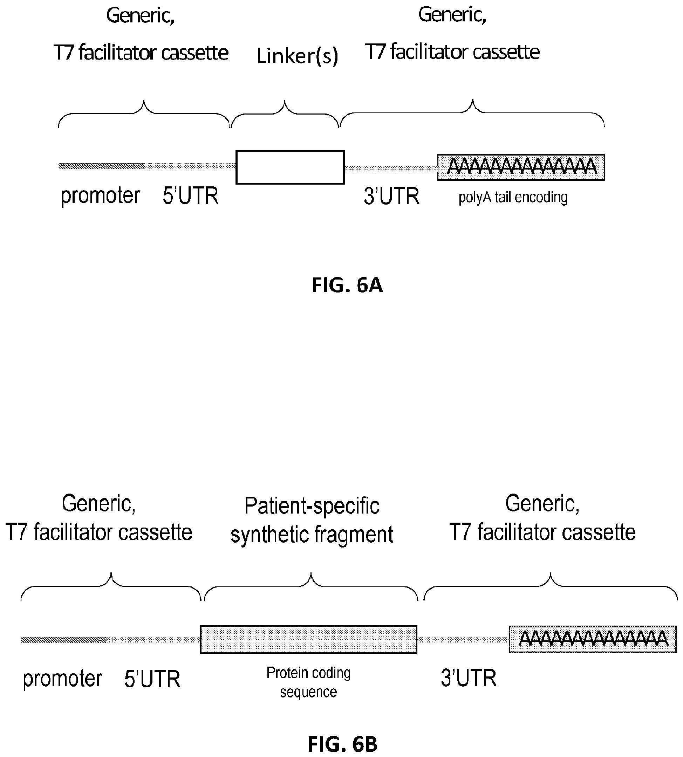

In some examples, the *in vitro* transcription facilitator cassette (IFC) as described herein is an *in vitro* transcription capable double-stranded DNA. FIG. 6A shows an example *in vitro* transcription facilitator cassette useful for making a double stranded DNA template. The *in vitro* transcription facilitator cassette includes functional elements configured to facilitate effective *in vitro* transcription (e.g., from an inserted gene of interest), such as a promoter, a portion encoding a 5' untranslated region, (5'UTR), a portion encoding a 3' untranslated region (3'UTR), and a portion encoding for a poly-A tail. The *in vitro* transcription facilitator cassette also includes one or more linkers useful for cloning a gene of interest into the *in vitro* transcription facilitator cassette for expression of the gene of interest and restriction sites to ensure template linearization.

An *in vitro* transcription facilitator cassette can be manufactured synthetically or non-synthetically but in general will be manufactured synthetically. In some examples, methods of manufacturing synthetic *in vitro* transcription facilitator cassette include using a commercially available DNA synthesizer such as those available from Twist Bioscience (San Francisco, CA) or ThermoFisher Scientific (Waltham, MA). Further, the *in vitro* transcription facilitator cassette can be assembled from separate pieces of DNA, or it may be synthesized as one piece. In some examples, the *in vitro* transcription facilitator cassette is linear and may include compatible ends that can be ligated together. In some examples, the *in vitro* transcription facilitator cassette is circular. In some examples, the circular *in vitro* transcription facilitator cassette includes a site (e.g., a restriction endonuclease site) between the portion encoding a poly-A region and the promoter configured for generating a linear DNA containing, in order, a promoter, a 5' UTR, linker region, a 3'UTR, and a portion encoding a poly-A region upon application of the appropriate restriction endonuclease. In general, the *in vitro* transcription facilitator cassette does not encode an antibiotic resistance gene. For example, a synthetically synthesized *in vitro* transcription facilitator cassette does not need an antibiotic resistance gene as it is not grown in a biological (e.g., bacterial) cell and does not require antibiotic selection. In general, the *in vitro* transcription facilitator cassette does not have an origin of replication (ori) or related control elements for facilitating DNA replication. For example, a synthetically synthesized *in vitro* transcription facilitator cassette does not need an ori as it is not grown in a biological (e.g., bacterial) cell and does not need an ori for replication. The total length of the *in vitro* transcription facilitator cassette can be smaller than many plasmids. The *in vitro* transcription facilitator cassette can be less than about 2kb in length, less than about 1.5 kb in length, less than about 1.0 kb in length, less than about 900 bps in length, less than about 800 bps in length, less than about 700 bps in length, or less than about 600 bps in length.

As indicated above, *in vitro* transcription facilitator cassette includes a promoter. The enzyme RNA polymerase binds to the promoter and initiates transcription of RNA from a gene of interest (e.g., after a double stranded DNA template has been assembled from the cassette and the gene of interest). Examples of promoters useful for transcription in the cassette include natural or modified T7 promoters, natural or modified T3 promoter, or natural or modified SP6 promoters.

The *in vitro* transcription facilitator cassette also includes a portion encoding an exchangeable 5' untranslated region (5' UTR) and a portion encoding an exchangeable 3' untranslated region (3' UTR). These regions, which do not themselves get translated into protein or peptide, help regulate translation of an mRNA into a protein or peptide. The *in vitro* transcription facilitator cassette also includes a portion encoding a poly-A tail. A poly-A tail in an mRNA is a long chain of tens or hundreds of repeated adenine residues. A poly-A tail on an mRNA is believed to serve several functions such as increasing the stability of the mRNA in the cytoplasm of a cell and aiding in translation of the mRNA into protein. Unlike the rest of the sequence of an mRNA which is encoded directly by the DNA in a template in mRNAs, the poly-A tail is not normally directly encoded by the DNA (e.g., in nature). Rather, naturally occurring DNAs contains a shorthand signal, called a polyadenylation signal (e.g., AATAAA), that along with other DNA sequences, signals the transcription machinery in a cell to add a poly-A tail to an mRNA that is being synthesized. In other words, the length of the poly-A tail in naturally occurring mRNAs is determined by the cell that makes mRNA. As seen in FIG. 6A, the *in vitro* transcription facilitator cassette as described herein includes a region of DNA that directly encodes for the poly-A tail (e.g., the entire tail). The length of the poly-A tail is determined by the length (e.g., the number of adenines or poly-As or the number of thymidines or poly-Ts) in the region of DNA that directly encodes for the poly-A tail. The region of DNA that direct encodes for the poly-A tail can be at least about 100 bp long, at least about 200 bp long, at least about 300 bp long, at least about 400 bp long, or at least about 500 bp long and can be anything in between these sizes (such as about 350 base pairs long). A poly-A tail can be added to an mRNA made using the cassette as a template using the same process as used to generate the rest of the mRNA. One advantage of this is that the process for generating the entire mRNA, including the poly-A tail, is greatly simplified. Living cells and complex extracts from cells containing cell DNA, cell RNA, cell membrane proteins, and other components are not needed in order to generate mRNA. Instead, well-defined transcription mixtures can be used to generate the entire mRNA, including the poly-A tail, from a double-stranded DNA template as described herein, making a transcription mixture generally free from toxic side products that may otherwise be found in transcription mixtures that are made using cells or cell extracts. The well-defined mixture may be safely delivered to a patient with only minimal clean-up required. When the double-stranded DNA template is also generated from a well-defined mixture substantially free from toxic side products, the transcription product made from the double-stranded DNA template is suitable for direct injection into a patient with only minimal clean-up necessary. Described herein is a double-stranded DNA template that is generated from a well-defined mixture substantially free from toxic side products.

The *in vitro* transcription facilitator cassette also includes one or more linker regions. The linker region is between the 5' UTR and the 3' UTR. The linker region includes at least one cleavable site and generally two cleavable sites. If two or more cleavable sites are present, they may have the same sequence or different sequences. The one or more cleavable restriction sites are useful for inserting a gene of interest (GOI) into the *in vitro* transcription facilitator cassette to generate a synthetic linear or circular ligated product. The gene of interest is generally inserted between the 5'UTR and 3'UTR in the *in vitro* transcription facilitator cassette though in some cases 5'UTR or 3'UTR sequences could be included with the gene of interest and inserted into the *in vitro* transcription facilitator cassette along with the gene of interest. The cleavable site(s) may be a restriction endonuclease site, such as a Type II (type IIG, type IIS) restriction endonuclease, such as BsaI, BbsI, AarI, HhaI, HindIII, NotI, BbvCI, EcoRI, BglII, FokI, AlwI, AcuI, or BcgI available from New England Biolabs (NEB; Ipswich, MA); Promega Corporation (Madison, WI); or ThermoFisher Scientific (Waltham, MA).

The gene of interest (GOI) as described herein is a short piece of DNA that generally encodes for a functional product molecule (RNA or protein). The gene of interest may encode a particular protein, a part of a protein, or a particular function. In some cases it may contain instructions for generating an RNA that does not encode for a particular protein or part of a protein (e.g., it may encode a functional RNA that does not get translated).

A gene of interest useful for inserting into an *in vitro* transcription facilitator cassette can be manufactured synthetically or non-synthetically, but in general will be manufactured synthetically. Methods of manufacturing synthetic genes of interest include by using a commercially available DNA synthesizer and method such as those available from Twist Bioscience (San Francisco, CA) or ThermoFisher Scientific (Waltham, MA). Further, although the genes of interest can be assembled from separate pieces of DNA, in general it is synthesized as one piece. A gene of interest may be manufactured as a linear piece of DNA or a circular piece. A circular gene of interest may be digested with a restriction enzyme to form a linearized gene of interest. The manufactured gene of interest may be purified (e.g., by column, electrophoretic separation, etc.).

A gene of interest in general will be cleaved prior to combining it with an *in vitro* transcription facilitator cassette. In particular, a gene of interest may be cleaved with the same restriction endonuclease(s) as used to cleave the *in vitro* transcription facilitator cassette, but may also be generated through enzymatic amplification. In general, the gene of interest does not encode an antibiotic resistance gene. For example, a synthetically synthesized gene of interest does not need an antibiotic resistance gene as it is not grown in a biological (e.g., bacterial) cell and does not require antibiotic selection. In general, the gene of interest does not have an origin of replication (ori) or related control elements for facilitating DNA replication. For example, a synthetically synthesized gene of interest does not need an ori as it is not grown in a biological (e.g., bacterial) cell and does not need an ori for replication. In some examples, total length of the gene of interest can be smaller than many plasmids. The gene of interest can be less than about 2kb in length, less than about 1.5 kb in length, less than about 1.0 kb in length, less than about 900 bps in length, less than about 800 bps in length, less than about 700 bps in length, or less than about 600 bps in length, less than about 500 bps in length, less than about 400 bps in length, or less than about 300 bps in length, less than about 200 bps in length, less than about 100 bps in length.

In some examples, the gene of interest is a T-cell receptor (TCR) or a portion of a T-cell receptor, such as for treating a CTCL or other disease or condition mediated by a T-cell receptor (TCR) or a portion of a T-cell receptor. For example, in T-cell development, cells must rearrange the T-cell receptor (TCR) genes to create and express a novel TCR molecule. Because TCR rearrangement occurs early in T-cell development and prior to the development of mature T-cell lymphomas such as CTCL, every malignant CTCL cell expresses an identical, clonal TCR, composed of unique TCR alpha and TCR beta subunits. This TCR is unique to the lymphoma cells, making it otherwise foreign to the immune system and therefore an excellent target for therapy.

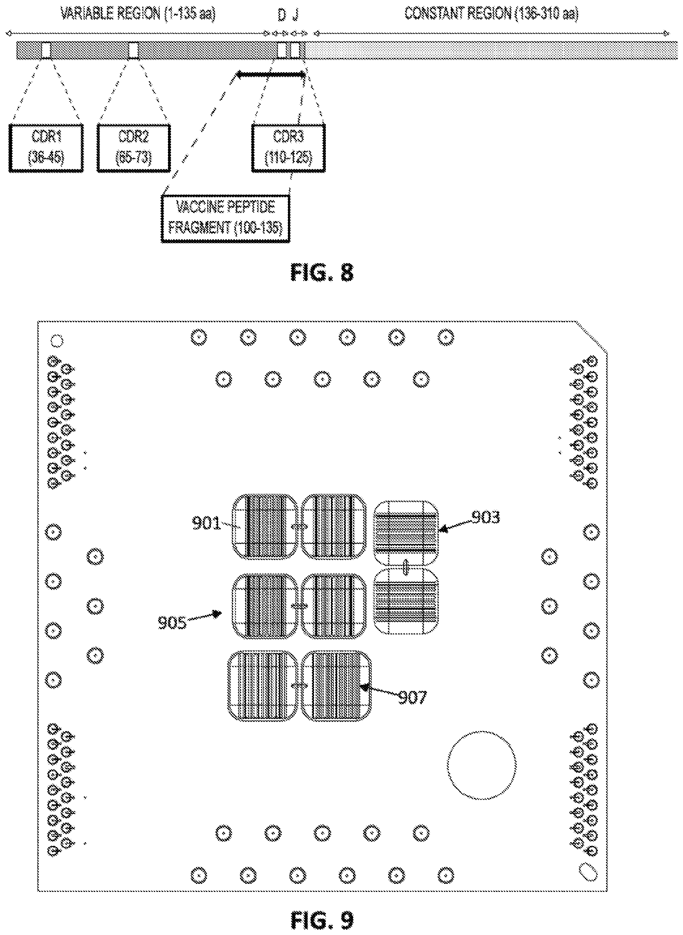

The gene of interest may be part or all of a complementary determining region (CDR) region. CDRs are the highly variable portions of the TCR sequences and mediate binding of the T cell to an antigen-major histocompatibility complex (MHC). FIG. 8 shows one region of a T-cell receptor useful for making a double-stranded DNA for use in a vaccine or therapeutic. In particular, the CDR3 region which spans the junctions between the V(D)J and C regions have the highest variability and represent a truly unique protein fragment that should only be found in the lymphoma cells. Thus, the CDR3 region extended by 10 amino acids both at the C and N termini constitutes the vaccine peptide fragment. In some methods, the unique sequence of a gene, such as T-cell receptor (TCR) or a portion of a T-cell receptor, is determined from an individual and the gene of interest is manufactured to be the same as the T-cell receptor (TCR) or a portion of a T-cell receptor from the individual. Although in some cases, the sequence may be controllably modified in a specific, known way, such as for codon optimization or optimized RNA stability or expression, the sequence of the gene of interest is nonetheless based on the sequence obtained from the individual. In some examples, the sequence of the gene of interest comprises a T-cell receptor having a DNA sequence identical to a DNA sequence from a patient or controllably modified in a known way relative to the DNA sequence from the patient.

Described herein are methods of making a double stranded DNA template, and in particular methods of making a synthetic double stranded DNA template. The double stranded DNA template may be especially useful for performing *in vitro* transcription to generate mRNA such as for use in a vaccine or other therapeutic for injection or another mode of delivery to a patient.

Existing DNA templates for performing *in vitro* transcription and mixtures for performing *in vitro* transcription commonly include crude or semi-purified cellular extracts (e.g., bacterial, other microbial, or other extracts) and may be complex and undefined. Such extracts may include bacterial, other microbial, or other DNA, endotoxin, and/or other undesirable components. When used for generating DNA templates or performing *in vitro* transcription as part of a process for vaccine or therapeutic use, undesirable components can increase the risk of serious side effects. For example, endotoxin is a large molecule of lipopolysaccharide in the exterior cell wall of Gram-negative bacteria, a commonly used source for generating cellular extracts for use in *in vitro* transcription reactions. Endotoxin in the bloodstream, such as by injection, can cause a variety of problems in humans and other animals, such as inflammation and sepsis and poses a significant health risk. The methods described herein may be especially useful for making a double-stranded DNA template free of biological contaminants (bacterial, other microbial or other contaminants), free of bacterial (or other microbial or unwanted) DNA, and/or free of endotoxin. The methods described herein may include using defined or synthetic components for making a gene of interest, making an *in vitro* transcription facilitator cassette, and/or making a double-stranded DNA template (or making any intermediaries used for making these materials). The defined or synthetic components may be made from defined or synthetic ingredients such as DNA synthesizers, purified nucleotides and purified enzymes. The defined or synthetic components may be essentially free of bacterial, other microbial, or other DNA, endotoxin, and/or other undesirable components. By avoiding the use of biologically based components, biological contaminants such as DNA and endotoxin do not contaminate the DNA template (or other components) in the first place. Double-stranded DNA templates and downstream materials are safer without the need for difficult or troublesome purification operations. This and other methods herein may include operations of joining a synthetic gene of interest with a synthetic *in vitro* transcription facilitator cassette to create a synthetic linear or circular ligated product; removing unreacted synthetic gene of interest and unreacted synthetic *in vitro* transcription facilitator cassette; amplifying the circular ligated product to generate a linear, circular or branched amplified DNA; and linearizing the amplified DNA ligated product to generate double stranded DNA template.

As indicated above, joining a gene of interest with an *in vitro* transcription facilitator cassette to create a synthetic linear or circular ligated product may include inserting the gene of interest into an *in vitro* transcription facilitator cassette. FIG. 6B shows a double-stranded DNA template generated as described herein. The gene of interest and *in vitro* transcription facilitator cassette may have the same restriction endonuclease site(s) as described elsewhere herein and the method may include digesting the gene of interest and the *in vitro* transcription facilitator cassette with the restriction endonuclease for the restriction endonuclease site, creating compatible ends, and ligating the gene of interest into the cassette. The method may include combining a gene of interest, an *in vitro* transcription facilitator cassette, a restriction endonuclease buffer, a source of energy, one or more restriction endonuclease enzyme(s), a ligase buffer, and a ligase and incubating the mixture for an appropriate amount of time. The buffer(s) may be suitable for or optimized for the particular restriction endonuclease and/or ligase and may be one buffer or may be two (or more) buffers. Endonuclease and/or ligase buffers may be commercially available buffers (e.g., NEB, Promega) and/or may include Tris, potassium, magnesium, sodium chloride, and dithiothreitol, such as Tris-acetate (e.g., about 6mM - 90 mM), potassium acetate (about 50 mM - 100 mM), magnesium acetate (about 5mM - 10mM), bovine serum albumin (BSA; about 50 ug/ml -200 ug/ml) dithiothreitol (1mM) at a pH from about 7.4 to about 9.0. The ligase may be a commercially available (e.g., New England Biolabs, Promega, Thermo Fisher Scientific) or other ligase such as T3 DNA ligase, T4 DNA ligase, or T7 DNA ligase. Digesting may take place from about 10 minutes to about 4 hours, or any amount of time in between (e.g., about 30 min, about 1 hour, about 2 hours, etc.) The ligating operation may take place from about 10 minutes to about 4 hours, or any amount of time in between (e.g., about 30 min, about 1 hour, about 2 hours, etc.). The digesting and ligating operations may be performed simultaneously or sequentially. A source of energy may be adenosine 5' - triphosphate (ATP) (e.g., from about 0.1 mM to about 5 mM). Additional quantities of any of the components such as restriction endonuclease and ligase may be added over time and incubation may continue. In some examples, only materials certified to be animal origin free (AOF) will be used for therapeutic manufacturing to reduce the risk of transmitting infectious agents. Some of these methods in which the *in vitro* transcription facilitator cassette is not circular includes the operation of ligating the ends of the *in vitro* transcription facilitator cassette and generating a circular *in vitro* transcription facilitator cassette. Alternatively, other methods for ligating the gene of interest and the *in vitro* transcription facilitator cassette such as chew back methods can be used. Alternatively or additionally, ligation between the *in vitro* transcription facilitator cassette and the gene of interest can be performed using primer extension to generate linear molecules prior to exponential amplification methods.

This or other methods described herein may include the operation of removing unreacted synthetic gene of interest and unreacted synthetic *in vitro* transcription facilitator cassette away from the synthetic linear or circular ligated product or purifying the double-stranded DNA away from the unreacted synthetic gene of interest and unreacted synthetic *in vitro* transcription facilitator. Removing unreacted synthetic gene of interest and unreacted synthetic *in vitro* transcription facilitator cassette away from the synthetic circulated ligated product may be done using an enzyme such as an exonuclease (such as exonuclease V) in an appropriate exonuclease buffer (NEB; Promega, Thermo Fisher). The method may include digesting synthetic gene of interest and unreacted synthetic *in vitro* transcription facilitator. The method may include passing the digested mixture through a resin or column, such as an ion exchange resin or size exclusion resin, and holding either the unreacted synthetic gene of interest and unreacted synthetic *in vitro* transcription facilitator cassette in the column or holding the double-stranded DNA template in the column and allowing the double-stranded DNA template or the unreacted synthetic gene of interest and unreacted synthetic *in vitro* transcription facilitator cassette to pass through the resin or column. Some examples may also include holding digested nucleotides within the resin or column or allowing digested nucleotides to pass through the resin or column. Some examples include washing and/or eluting the resin or column. Some examples may also include holding digested nucleotides within the resin or column. Some examples include binding unreacted synthetic gene of interest and unreacted synthetic *in vitro* transcription facilitator cassette to beads or binding double-stranded DNA to the beads, holding the beads with a magnet and removing either the double-stranded DNA or unreacted synthetic gene of interest and unreacted synthetic *in vitro* transcription facilitator cassette from the double-stranded DNA. Resins, columns, and magnetic beads are available from places such as Bangs Laboratories, Inc., (Fishers, IN), Beckman Coulter (Brea, CA), Millipore (Burlington MA), Thermo Fisher, VWR (Radnor, PA). Some examples may include the use of methylation sensitive restriction enzymes.

This and other methods described herein may include amplifying the linear or circular ligated product to generate amplified DNA. Some methods include amplifying the linear or circular ligated product to generate a linear amplified DNA. Some methods include amplifying the linear or circular ligated product to generate a linear, branched or circular amplified DNA. The amplified product may be amplified using helicase-dependent amplification (HAD), loop-mediated isothermal amplification (LAMP), multiple displacement amplification (MDA), nucleic acid sequence based amplification (NASBA), polymerase chain reaction (PCR), rolling circle amplification (RCA), self-sustained sequence replication (3 SR), or strand displacement amplification (SDA). The appropriate buffer, deoxyribonucleotide triphosphates (dNTPs), enzyme (DNA polymerase), and primers for the reaction are added as needed. Temperature and timing of the amplification is controlled. The method may include the operations of heating the linear or circular ligated product (e.g., at or above about 70°C to about 100°C) to denature the DNA and then cooling the DNA. The method may include the step of adding a denaturation buffer configured for denaturing the DNA to the linear or circular ligated product to denature the DNA and then adding a neutralization buffer to the denatured DNA mixture to neutralize the denaturation buffer and leave denatured DNA. The method may include the operation of adding an enzyme such as a DNA polymerase enzyme for amplifying or extending the denatured DNA (e.g., Bst DNA polymerase, Φ29 DNA (Phi29) polymerase, Taq DNA polymerase) and amplifying or extending the DNA with the enzyme to generate amplified DNA (e.g., branched, circular, or linear amplified DNA).

Some methods include purifying the amplified or extended DNA away from the buffer, enzyme, nucleotides, and other unwanted components. The method may include passing amplified or extended DNA using through beads, resin or a column, such as an ion exchange resin, magnetic beads, or size exclusion resin, and either holding the amplified or extended DNA or allowing the amplified or extended DNA to pass through the beads, resin or column and holding the unwanted enzyme and other components in the beads, resin or column. Some examples include washing and/or eluting and/or drying and/or rehydrating the resin or column. Some examples include repeating one or more of these operations. Some examples include a two or more (a plurality) of depots of beads, resin, or columns and repeating one or more of the washing/eluting/drying/and/or rehydrating operations. Some examples include binding DNA to beads, holding the beads with a magnet and removing (washing) unwanted components and contaminants away from the DNA and beads. Resins, columns, and magnetic beads suitable for use are available from Bangs Laboratories, Inc., (Fishers, IN), Beckman Coulter (Brea, CA), Millipore (Burlington MA), Thermo Fisher, and VWR (Radnor, PA).

In some examples, amplified DNA is not linear; it may be branched or circular. Some methods include linearizing DNA and generating linearized template DNA. Some methods may include adding a restriction endonuclease (in an appropriate buffer) to purified amplified or extended DNA, incubating the DNA with the restriction endonuclease, and linearizing the DNA. The restriction enzyme is chosen to cut outside of the 5'UTR, gene of interest, 3'UTR, and the portion encoding the poly-A region. In some examples, the restriction enzyme cuts between the 3'UTR of one extended or amplified DNA and the 5'UTR of an adjoining (and downstream) extended or amplified DNA. The restriction enzyme can be any restriction enzyme, such as a type IIs restriction enzyme as indicated above with regards to restriction enzyme digestion for joining a synthetic gene of interest with a synthetic *in vitro* transcription facilitator cassette to create a synthetic linear or circular ligated product. In some examples, the restriction enzyme is at least one of BsaI, BbsI, AarI, HhaI, HindIII, NotI, BbvCI, EcoRI, BglII, FokI, AlwI, AcuI, or BcgI available from New England Biolabs (NEB; Ipswich, MA); Promega Corporation (Madison, WI); or ThermoFisher Scientific (Waltham, MA). In some examples, the restriction endonuclease is/are the same restriction endonuclease(s) as used for inserting the synthetic gene of interest into the *in vitro* transcription facilitator cassette. In some examples, the restriction endonuclease is/are different from the restriction endonuclease(s) used for inserting the synthetic gene of interest into the *in vitro* transcription facilitator cassette. Also described herein is microfluidic path device reactor for making double-stranded DNA as described herein.

FIG. 9 shows an example of one example of an architecture of a microfluidic biochip reactor for generating double-stranded DNA. This and other methods described herein may include generating double-stranded DNA from the gene of interest and the *in vitro* transcription facilitator cassette in a sterile, closed biochip in which all components are sterilely maintained during generation. The sterile, closed biochip is closed to the atmosphere. FIG. 9 shows the microfluidic biochip reactor with 4 interconnecting reactors (e.g., modules or chambers) through which DNA precursors at different stages along the pathway to becoming a double-stranded DNA template move. For example, in FIG. 9 a ligation reactor (ligation reaction chamber 901), a pre-mixing chamber 903, an amplification reactor (amplification reaction chamber 905) and a digestion reactor (digestion reaction chamber) 907 may be included (connectors and valves are not shown in this example. Different operations of the methods described herein are carried out in different modules or chambers. The gene of interest and the *in vitro* transcription facilitator cassette are mixed together in the pre-mixing chamber. The gene of interest and the *in vitro* transcription facilitator cassette are joined together to create a ligated product in the ligation reaction chamber. The ligated product is amplified to generate amplified DNA in the amplification chamber. The amplified DNA is further processed, such as being digested in the digestion reaction chamber to remove unwanted DNA or to separate different copies of the amplified gene of interest.

### Template formation using PCR

In general, described herein are methods and apparatuses using PCR based, animal-free techniques for forming the synthetic polynucleotide (e.g., DNA) template including a short primer and a long (greater than about 150 bp, greater than about 200 bp, etc.) primer for forming mRNA template having a long poly-A tail.

The template for mRNA formation described herein may be completely synthetic and formed without the use of bacteria in a microfluidic apparatus. Template formed using bacterial processes may provide an opportunity for contamination and possible cross-reactivity issues (e.g., patient reactions) when used as part of a therapeutic. The methods and apparatuses described herein may form a template starting with a fragment (e.g., synthetic fragment) that may be combined with a promotor and a poly-A tail, and then amplified, as described above. For example, a target sequence, e.g., a patient-specific sequence (such as a gene of interest or portion of gene of interest or UTR) may be synthesize and made competent for amplification and in vitro transcription by adding the promotor, e.g., T7 promotor, that can bind for IVT at the 5' end and also add the long poly-A tail at the 3' end. The poly A tail may advantageously be quite long (e.g., > about 150 mer, greater then about 200 mer, greater than about 250 mer, etc.).

In some examples the method for forming the template may use PCR and an asymmetric (e.g., one relatively short and one much longer) set of primers. The promotor side of the synthetic fragment may include a region specific to anchor a primer (for amplification). In some examples the tail end may be non-specific. In any of these methods, a long poly-T tail primer (e.g., about 150 mer or greater, about 200 mer or greater, etc.), which may also include a specific sequence region (anchor region, e.g., of about 20 and about 40 bp long, e.g., about 25 bp long) at one end for annealing/hybridization. The PCR process may then be used to form and amplify the template, and may be run for about 20 cycles or more. A methylation sensitive cutter (such as, e.g., DPN1) may be used to get rid of any potential trace of bacterial DNA. These methods and apparatuses may include a purification operation to wash/remove anything below about a threshold, e.g., of less than about 800 bp, less than about 700 bp, less than about 600 bp, less than about 500 bp, etc. Any of these methods may be performed by and integrated into the apparatuses (e.g., microfluidic devices, chips, etc.) described herein.

In some examples the patient-specific sequence (e.g., the target, gene of interest sequence) may be provided by a commercial provider and may originate from, e.g., a circular plasmid. The circular plasmid may be linearized before performing the PCR formation and amplification and the DPN1 treatment. Alternatively, in some examples the entire starting (target or gene of interest) sequence could be synthesized (e.g., chemically synthesized, etc.).

In some examples the starting material is about 1 ng/uL or less, e.g., about 0.5 ng/uL or less, about 0.1 ng per ul or less, about 0.09 ng/uL or less, about 0.07 ng/uL or less, about 0.05 ng/uL or less, etc. For example, these methods and apparatuses may be configured to perform a 50 ul reaction, starting with about 5 ng of input material. The method or apparatus may include cycling for about 20 cycles (e.g., about 21 cycles, about 22 cycles, about 23 cycles, about 24 cycles, etc.). The output may be matched to the input for the IVT for which the template is to be sued. For example, a 50 uL reaction using 3 ug of template may be used with about 20 cycles or more (e.g., about 21 cycles, about 22, cycles, about 23 cycles, about 24 cycles, etc.). In some examples, this may result in about 4 and about 5 ug being produced after purification (some of which may be used for quality control testing and/or verification).

Surprisingly, the methods for forming template described herein with a very long (greater than about 150 bp) primer may cleanly produce a significant amount of template, without any substantial shortened forms or transcription errors without the need for significant downstream purification and/or without the use of multiple restriction enzymes, which may make the template formation process both overly complex may result in possible contamination (e.g., from bacterially sourced enzymes). Although it is known that a poly(A) tail may confer stability to an mRNA and enhance translation efficiency, it is widely believed that the use of a PCR/primer-based method for adding a poly(A) tail has an upper limit on the length of the polyA region of approximately 120-mer, beyond which amplification abnormalities will prevent reliable use. For example, US9943612B2 to Scharenberg explicitly addresses the issue of generating really long (>200 bp) polyA tails for use in IVT, and concludes that "[a]lthough the addition of a poly (A) tail to the mRNA by PCR avoids the extra enzymatic operation, PCR methodologies can also bring additional problems, such as length variation, as well as amplification abnormalities, which can stem from specific genes that are not readily amplified by PCR. PCR is also restricted by the number of adenine residues that can be contained in a primer (to approximately 120). More importantly, it is also limited to genes that can be amplified by PCR."

In contrast, the method of formation of IVT template for manufacturing therapeutic mRNAs described herein may instead use a very long (e.g., about 150 mer or greater, about 200 mer or greater, about 250 mer or greater, etc.) poly-A containing primers.

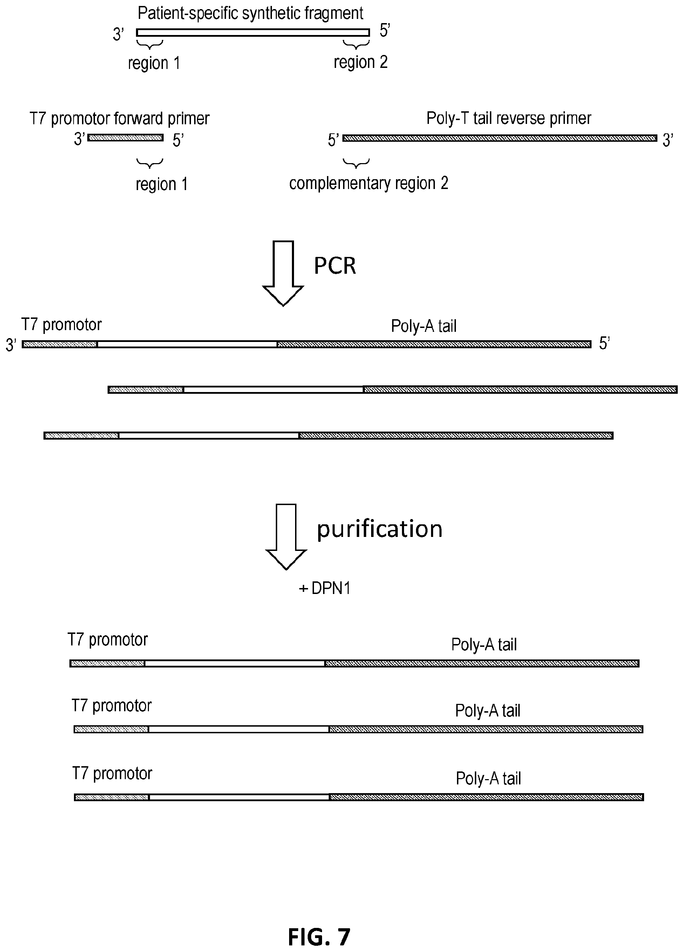

The methods (and apparatuses for performing them) described herein may use a synthesized length of the target gene (or region of a gene) of interest that may be modified using a PCR-based method to add a long (> about 150 mer) poly-A tail, and in some examples a promoter (e.g., T7 promoter) in a manner that is relatively free of bacterial products and therefore cross-contamination. For example, a target gene of interest may be provided in ng amounts. In some examples the target gene (or region of a gene) of interest may be synthesized by a commercial synthesis company or technique and may be presented in a plasmid that contains the gene; this plasmid does not include the poly-A region, and in some examples does not include the promoter for use in IVT. Thus, the target gene, or region of a gene, may therefore be referred to as a synthetic gene of interest. The synthetic gene of interest may be a patient-specific synthetic fragment. As illustrated in FIG. 7, the method may therefore include the use of this synthetic gene of interest and a pair of primers. In FIG. 7, the first primer ("T7 promoter forward primer") is a forward primer that include the desired promoter (e.g., T7 promoter) and a region in the 5' end of the primer that is identical to a region in the 3' end of the patient-specific synthetic fragment. A second, reverse, primer ("Poly-T tail reverse primer") includes a region that is complementary to a second region of the 5' end of the patient-specific synthetic fragment (docking region) at the 5' end of the Poly-T tail reverse primer. The remainder of the Poly-T tail reverse primer may include a stretch of about 150 or more thymine (a poly-T region).

In FIG. 7, the method may include combining the synthetic gene of interest (patient-specific synthetic fragment) with the T7 promoter forward primer and the Poly-T tail reverse primer in a chamber of the microfluidic device. This chamber may then be temperature controlled using the apparatus to thermocycle to form and amplify the patient-specific synthetic fragment to which the poly-A tail has been added, forming template as shown. Following amplification, the synthesized template may then be purified in the same microfluidic path device (e.g., chip). For example, in some examples an enzyme, such as DPN1 may be added to the chamber and/or the material may be moved to a second chamber to include the DPNI. The material may then be further processed, including removing smaller (e.g., less than about 600 bp, less than about 500 bp, less than about 400 bp, less than about 300 bp, etc.). The final template may then be stored and/or used immediately (or withing a few days, hours, minutes) to form a therapeutic mRNA, using an IVT procedure as described herein.

FIG. 7 shows a first example of a first primer and a second primer. Alternatively, in some examples the first primer may instead be a reverse primer (e.g., "T7 promoter reverse primer") that includes a region that is complimentary to the first region at or near the 3' end of the patient-specific synthetic fragment. The second primer may be a forward primer (e.g., "Poly-A tail forward primer") that includes a region at or near the 3' end of the second primer that is approximately the same sequence as the second region at or near the 5' end of the patient-specific synthetic fragment, and a long length (e.g., about 150 mer or more) of adenosine, forming the poly-A region.

In some examples the first primer, including the promoter (e.g., T7 promoter) region, hybridizes to the synthetic gene of interest or a polynucleotide that is complimentary to the synthetic gene of interest.

In some examples, the synthetic gene of interest ("patient specific synthetic fragment) may be provided in a vector that may include either or both the promoter (e.g., T7 promoter) and/or a, typically short (< 50 bp) poly-A tail. Thus, in some examples the second primer may be configured to hybridize to a portion of the poly-A tail that is part of the vector.

In operation, the apparatus may control the temperature of the chamber(s) including the reagents (e.g., the synthetic gene of interest, the forward and reverse primers, the polymerase, and a buffer, including dNTPs). The microfluidic apparatus may therefore be configured to rapidly modify the temperature and maintain the temperature to control denaturation, annealing, extension, etc. The polymerase may be a polymerase having a low error rate, which is free from bacterial contamination, such as the Q5 ("High Fidelity polymerase" from New England Biolabs).

In some examples the thermocycling may be performed in a chamber or in multiple chambers of the microfluidic apparatus. The chamber may be prepared to prevent contact or surface interference, for example, by pretreating or coating the chamber with a material prior to adding the reagents. For example, a coating such as a synthetic (non-bacterial) material (e.g., protein material) may be included in order to reduce or prevent nonspecific binding to the walls of the chamber, which may be polymeric plastic material. In some examples the chamber may be pretreated with a recombinant (e.g., synthetic) albumen (e.g., a recombinant albumin that is molecular biology grade). The material may be passivated by pretreatment with a synthetic material, such as synthetic albumen.

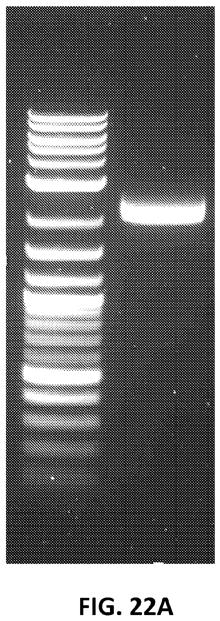

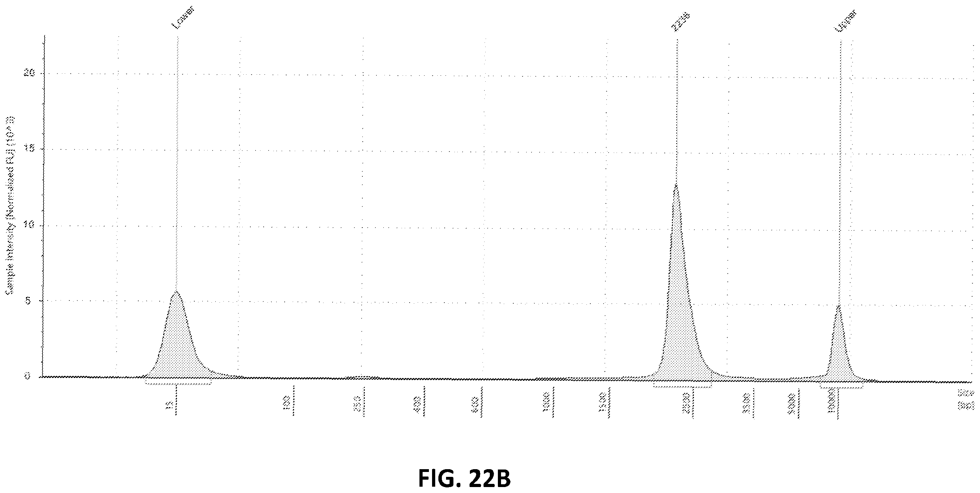

FIGS. 22A and 22B illustrate one non-limiting working example of a synthetic template that was formed as described above. For example, FIG. 22A shows a gel electrophoresis of a template sample, in this example the template is a Luciferase reporter gene to which a T7 promoter and a 200 bp poly-A tail have been added by the method shown in FIG. 7. As illustrated in FIG. 22A, the right hand column shows a single clean band at the expected size for the polynucleotide template. The starting material was synthetically generated Luciferase reporter gene. The T7 promoter and the 200-mer poly-A tail was added by the use of a first (T7 promoter forward primer) and a second (Poly-T tail reverse primer). FIG. 22B shows a capillary electrophoresis of the same template shown in FIG. 22A.

The method of making a synthetic DNA template described above may result in a significantly higher yield and more uniform template as compared to more traditional bacterially synthesized template. This is illustrated in FIGS. 23A-23C, which shows a comparison between synthetically produced template mRNA and bacterially produced template mRNA, showing that synthetically-produced template mRNA results in a better size distribution and higher bioactivity. In FIGS. 23A-23C the template is a Luciferase reporter gene, similar to that described above in FIG. 22A-22B. FIG. 23A shows a capillary electrophoresis result for a Luciferase reporter gene mRNA template formed using a bacterial synthesis method, e.g., by cloning into a vector having the promoter and poly-A tail. For comparison, FIG. 23B shows a capillary electrophoresis result for a Luciferase reporter gene mRNA template formed using a synthetic PCR technique in which the T7 promoter and poly-A tail (e.g., 200-mer poly-A tail) were added by the use of primers as described above in relation to FIG. 7). In FIG. 23B the resulting template has a much narrower distribution and demonstrated a more uniform template with a comparable or higher yield.

FIG. 23C shows a comparison between the luciferase bioactivity in a mouse dendritic cell line (JAWSII) six hours after transfection of the two mRNAs synthetized from the templates shown in FIGS. 23A and 23B. As shown in FIG. 23C, the synthetic template (shown in FIG. 23B) has much higher expression (mean luminescence) as compared to the bacterial template.

### IVT Reaction

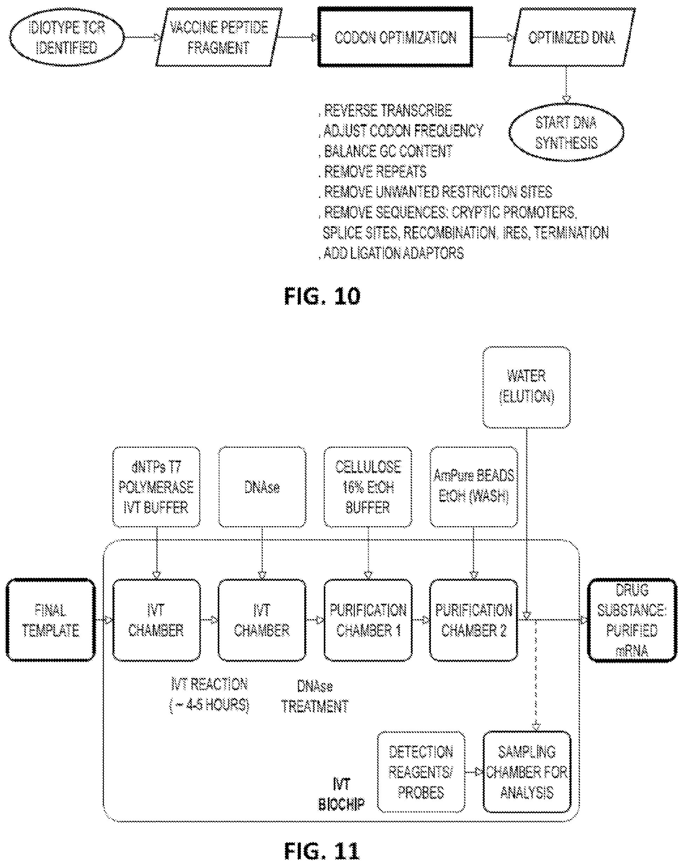

The next step in the process may be the IVT reaction that produces the mRNA. This process may be conducted inside the same or a different microfluidic path device (e.g., in some examples in an IVT microfluidic path device) which may be housed in the microfluidic path device control system as previously described. The high-level mRNA manufacturing process illustrating the main operations and sub-compartments within the IVT microfluidic path device are described in FIG. 11.

The IVT reaction may involve combining the DNA template with T7 polymerase enzyme, nucleotides and capping reagents and incubating the reaction under controlled conditions to produce capped mRNA molecules. The IVT reactions may take place inside a reaction chamber of a microfluidic path device (e.g., an IVT microfluidic path device) and process parameters such as temperature, mixing and reagent additions (both at the beginning and throughout the reaction) may be controlled to optimize levels. The process may be driven by the controller, as described above. The buffers and solutions may be delivered via an array of microvalves and volume may be controlled using a pre-set programs that may be specific to the protocol optimized for each mRNA drug substance.

Following the IVT reaction, a DNAse treatment may be performed to degrade the template DNA. This operation may be performed inside the IVT reaction chamber (part of the IVT reactor), and parameters such as dilution rate, enzyme/buffer concentration, temperature and mixing may be controlled to optimized levels. This procedure may be executed autonomously and recorded by a monitoring camera.

### IVT Purification

The DNAse treated mRNA may be purified to remove impurities and side products. In particular, degraded template, any unreacted nucleotides, enzymes (T7 polymerase and DNAse) and dsRNA affect the quality and immunogenicity of the drug substance. For purification, a 2-step solid-phase reversible immobilization procedure, using supports with different surface chemistries may be used. The first step may involve using a cellulose membrane to selectively capture dsRNA under precisely controlled binding conditions and eluting the non-bound fraction into a second purification chamber. The second purification operation may use about 1 and about 2 □m carboxyl-coated paramagnetic beads that selectively capture mRNA greater than about 500 bp in length. A number of washes may then be performed to remove unbound material that includes nucleotides, enzymes and degraded template. The pure mRNA can then be eluted in USP grade water. In- line microfluidics based purification enables a fully integrated workflow, without exposing materials to the atmosphere, avoids the use of toxic mobile-phases used with traditional HPLC-based methods and significantly reduces manual intervention.

As mentioned above, in general, these methods and apparatuses are aseptic methods and apparatuses that permit the manufacture of therapeutic mRNA, or any or all of the components for manufacturing therapeutic mRNAs without exposure to outside atmosphere, and/or to possible sources of RNAse and/or contaminating components that may otherwise be necessary. For example, as described herein these methods may be performed without the addition of bacterial sources of polynucleotides (e.g., in the template DNA), and/or without the addition of components, such as plasticizers, that may be present when purifying via HPLC or other traditional techniques. Described herein are apparatuses and methods for purifying within the microfluidic path device (e.g., using pure cellulose).

The purified mRNA may be quantified using A260 nm UV absorption, or fluorescence using an mRNA specific fluorescent dye. Additional mRNA QC operations may be performed to confirm purity and identity. The entire mRNA manufacturing process may be conducted inside the microfluidic path device control system and reagent addition and export may be performed via the closed-path microfluidic path device control system described above, e.g., using aseptic techniques. Finally, a filtration, e.g., through a 0.22 □m filter may be performed. The final product may be considered low bioburden drug substance and released for drug product formulation if it meets the acceptance criteria for: yield (e.g., by UV vis/Fluorometry, > about 6.5 ug mRNA per ul of starting IVT), identity (e.g., by sequencing, 100% consensus homology to target), integrity (e.g., sequencing, < 1% mutation rate), purity (e.g., CE, > about 95% of product in single band), capping efficiency (HPLC, > about 95% capped mRNA), residual dsRNA (e.g., FRET/Immunoblot, < about 0.02% (1 ng)), bacterial components (e.g., HCP ELISA (for DNA & protein), < X), bacterial components (e.g., HC-DNA, < X), endotoxin (e.g., LAL test, < about 0.2 EU/ml), bioburden (e.g., microbial limits testing (MLT)), etc.

### Formulation of mRNA into ANPs

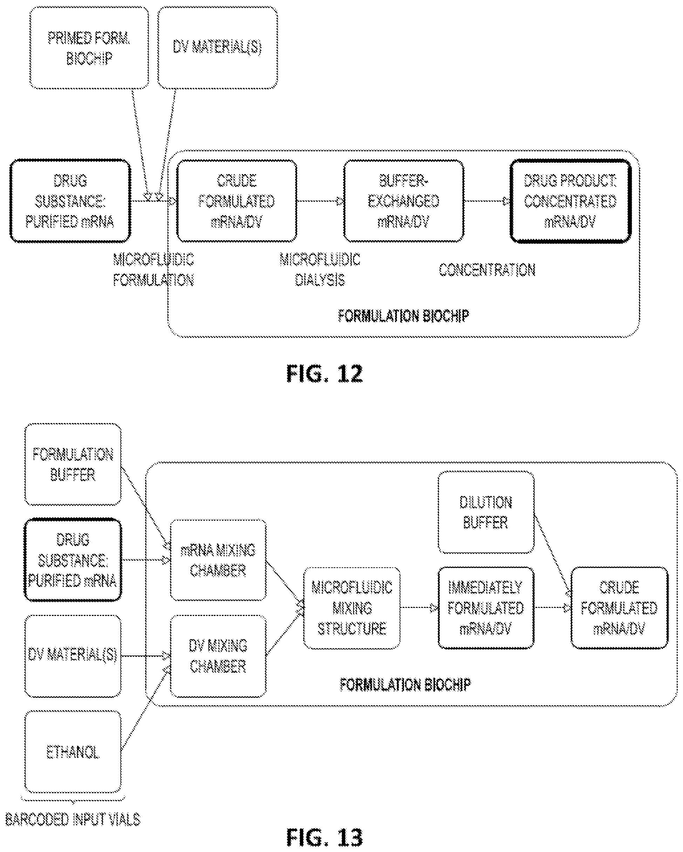

The purified mRNA may be combined with delivery components to form a nanoparticle formulation. This process is depicted in FIG. 12. For example, an aqueous solution of the mRNA cargo (therapeutic mRNA, also referred to herein as drug substance) may be combined with an ethanolic solution of delivery vehicle in a microfluidic mixing structure within a formulation microfluidic path device. The material may then undergo two post-formulation processing operations involving first an on-chip purification process to exchange buffer components in the formulated product, followed by a concentration operation to reduce the volume of the drug product to match specifications. The implementation of these processes onto a microfluidic path device-based manufacturing device may result in a high degree of control over the formulation process without the need for human intervention and with minimal possibility for human error.

In general, the component parts of the manufacturing methods described herein, including, e.g., synthesizing the template, performing the IVT to generate the mRNA, purifying the mRNA, combining the mRNA with a delivery vehicle to form a therapeutic composition, dialyzing the therapeutic composition, and/or concentrating the therapeutic composition may be performed on a single microfluidic path device and/or multiple microfluidic path devices, as shown in FIGS. 3A-3C, described above. Thus, the fluidic path may be continuous or partially continuous (e.g., continuous over the component portion of the manufacturing process, such as one or more of: template formation, IVT, purifying the mRNA, combining the mRNA with a delivery vehicle to form a therapeutic composition, dialyzing the therapeutic composition, and/or concentrating the therapeutic composition). In all cases, the same controller apparatus may be used, or different controller apparatuses may be used. The product of each of these component portions may be stored in a fluid vial (e.g., depot) in the controller apparatus and transferred to a new or subsequent microfluidic path device. Thus, in any of these methods and apparatuses, the product may be protected from exposure to the atmosphere.

As mentioned above in some examples a peptoid-based lipid formulations may be used as the drug vehicle, which may incorporate both cationic groups and lipid moieties onto an N-substituted peptide (i.e. peptoid) backbone. The delivery vehicle components may be monodisperse, fully-characterizable chemical entities which can be sourced through conventional means.

A controlled and consistent formulation process may be crucial to maintaining small, uniform particle sizes in mRNA ANP formulations. Delivery vehicle components are rapidly mixed with mRNA in a controlled ratio by the methods and apparatuses described herein. Exposure of DV components to aqueous solution and interaction between cationic (+) lipids and anionic (-) mRNA may trigger particle formation. This process can be carried out (to control particle size and uniformity) by using the microfluidic path devices described herein. The mRNA may be dissolved in an acidic buffer (pH 3-5) which may help ensure full protonation of basic functional groups (such as amines) on the delivery vehicle which are responsible for its cationic charge. The delivery vehicle may be dissolved in an aqueous-miscible organic solvent (typically ethanol) which facilitates the formation of nano-sized particles upon exposure to the aqueous cargo solution. Immediately after mixing, the solution pH may be stabilized by a neutral buffer. The resulting formulation can be stored at about 4 °C for weeks with no apparent loss of function. Alternatively, the formulation process can be performed just-in-time and at the point-of-care.

A formulation microfluidic path device as described herein may be designed to accomplish these formulation tasks. FIG. 13 illustrates a schematic of a general architecture of such a microfluidic path device that may be used. The first portion of the formulation microfluidic path device may include pre-dilution of both the mRNA and the DV components into separate staging chambers. The input materials may be advanced from sterile, barcoded vials into these pre-mixing chambers. The mRNA material(s) may be pre-diluted in acidic formulation buffer, and the delivery vehicle components are diluted in ethanol. At this stage, the concentrations of both materials may be adjusted to match required specifications for target DV/mRNA ratio, and the volume ratio that matches, e.g., a 3:1 aqueous:ethanol ratio that has previously shown to achieve good mixing behavior.

A microfluidic path device including a mixing structure may control, with precision, the mixing rate of the material. Faster or slower mixing may be provided, and controlled (e.g., by a controller). For example, a microfluidic path device including a mixing structure may provide for a significantly increased DV/mRNA mixing rate. At the start of the mixing process, equal pressure may be applied to both mixing chambers which forces fluid through the microfluidic structure at, e.g., about 0.5 mL/min. The geometry of this structure may be determined by the rapid mixing time of roughly about 3 ms. Under these conditions, amphipathic nanoparticles (ANPs) may be formed as water-insoluble lipid domains on the peptoid molecule are exposed to the aqueous mRNA solution.

Immediately following mixing, ANPs may be diluted with an in-line addition of 1:1 neutral PBS. This neutralizes the acidic formulation buffer and may prepare the formulation for purification and concentration. All of these processes may be controlled through the microfluidic path device control system to maintain highly-reproducible particle sizes and formulation properties.

The microfluidic device allows for the formulation of a personalized therapeutic at the point of care. In some examples, the therapeutic is a T-cell receptor (TCR) or a portion of a T-cell receptor, such as for treating a CTCL or other disease or condition mediated by a T-cell receptor (TCR) or a portion of a T-cell receptor. Personalized therapeutics may base the therapeutic composition on a specific patient's genetics (e.g., genotype), including generating a specific mRNA composition based on the patient's own sequence). The methods and apparatuses described herein may also or alternatively permit individualized therapeutics. Individualized therapeutics may be based on the patient's phenotype, e.g., based on the category a patient falls into, such as risk factor categories. Individualized therapeutics may therefore adapt specific therapeutics to a patient based on the patient's category. For example, a microfluidic formulation device may allow for multiple mRNAs to be mixed, for example, to generate from a sub-set of mRNAs from a larger library a therapeutic composition that is individualized to a patient based on the components and rations (amounts) of each component which may be determined from phenotype data on the patient. Any of these compositions may be compounded at the point-of-care to generate an optimized treatment for an individual.

### Post-formulation processing to generate drug product

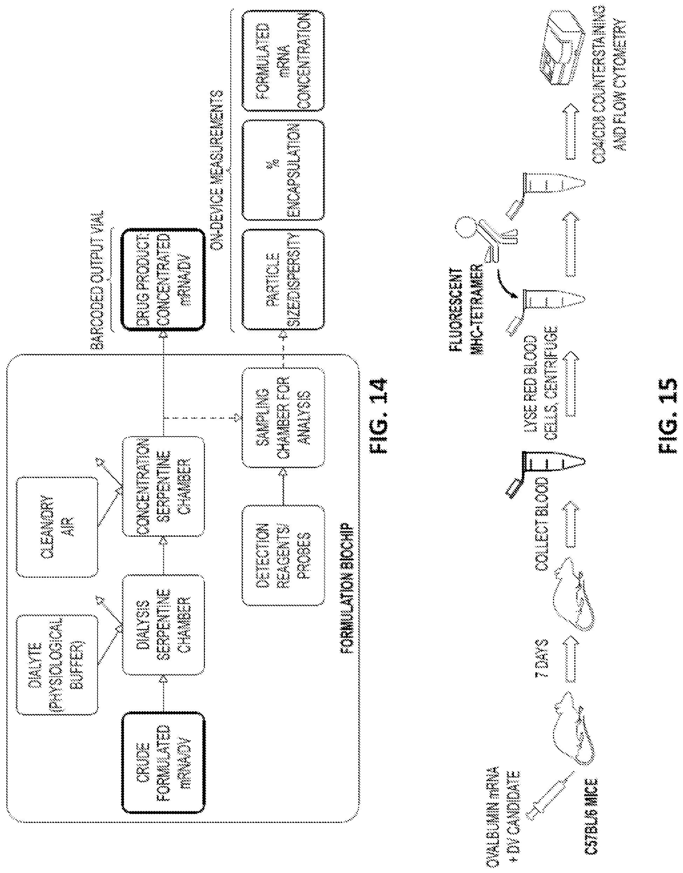

Once ANPs are formed during the formulation process, several post-processing operations may be completed on the formulation microfluidic path device. These may include purification (e.g., for ethanol removal), followed by evaporative concentration to reduce volume for dosing. See, e.g., FIG. 14.

The resulting nanoparticles may be analyzed on the microfluidic path device (e.g., by the microfluidic path device control system) for size distribution using, e.g., Dynamic Light Scattering (DLS) and % mRNA encapsulation using fluorescence. Analysis may be completed on a small aliquot of the final formulated material that is diverted from the main fluid path into an optically-transparent sampling chamber. Within this chamber, a fiber-optic light source may be used for the light scattering measurement to determine particle size and dispersity. Next, a fluorescent mRNA-specific probe is used to determine RNA concentration before and after particle disruption by addition of a detergent. This assay may elucidate the mRNA concentration for dosing information and the percentage of mRNA encapsulated in the ANPs versus free in solution. For example, analytical methods that may be used to test the formulated mRNA drug Product may include: Optical clarity (e.g., by visual inspection, no visible, aggregates, clear solution), characterizing lipid composition (e.g., by HPLC), size (e.g., DLS, about 80 - 300 nm), % Encapsulation (e.g., by fluorometry, > about 95% encapsulation), dispersity (e.g., DLS, PDI < about 0.25), endotoxin (e.g., LAL test, < about 0.2 EU/ml), sterility (e.g., culture (USP), < X cfu), pH (e.g., USP , pH 7.4 +/-0.2), potency (e.g., bioassay/ELISA, X EC₅₀).

### Examples

As mentioned above, the methods and apparatuses described herein may be used to manufacture mRNA therapies, including, for example, treatments for Cutaneous T-Cell Lymphoma (CTCL). Mature T-cell express a unique TCR which is formed by a combination of two proteins, the alpha and beta chains in αβ T-cells or the delta and gamma chains in δγ T-cells. Each TCR chain is formed by a unique recombination event by which any one of many possible exons encoding the V, (D) and J regions of the genes are brought together by a process called V(D)J recombination. These V(D)J recombination events are quasi-random and can produce a large number of combinations resulting in a large diversity of TCRs. In addition, during the V(D)J recombination process, random additions or deletions of nucleotides can occur at exon junctions, resulting in the generation of additional TCR diversity which together generate an individual's TCR repertoire. Healthy individuals sequenced deeply by next-generation technologies have been estimated to harbor in the order of 1-5 x 10⁶ different TCRs in peripheral blood at any given time point, and in the absence of infection no single TCR usually accounts for more than 5% of the total population. T-cell lymphomas arise from the clonal expansion of a single malignant T-cell leading to tumor development either in lymphoid tissues (spleen or lymph nodes) or other tissues such as skin, liver or gastro-intestinal track.

Alternative methods have been developed to sequence an individual's TCR repertoire which also serves to diagnose and identify the clonally expanded TCR in T-cell lymphoma patients. One commonly used method is to sequence TCRβ or δ genomic re-arrangements using carefully developed panels of PCR primers, where amplification bias is controlled. Thus, a multiplex PCR can be performed for target enrichment followed by next-generation sequencing. Such methods, for example the Immunoseq assay from Adaptive biotechnologies have been validated and are used in the clinic as a diagnostic tool and for minimal residual disease quantification. An alternative approach is to deeply sequence cDNA directly with no target enrichment to identify significantly overrepresented TCR chains. Identification of the Lymphoma TCR, is usually referred to as the lymphoma idiotype or clonotype.

For the determination of the Idiotype, biopsies may be collected and samples may be sequenced to determine lymphoma idiotype identification. The digital data concerning the patient-specific idiotype may be used for patient-specific vaccine design.

### Design of the mRNA vaccine

Production of an mRNA-based patient specific cancer vaccine may start with the design of a DNA sequence corresponding to a personalized target peptide capable of generating a specific and immunological effective epitope presentation by antigen presenting cells (APCs). To accomplish this, the first operation may include extracting the Complementary-Determining Regions (CDRs) from the idiotype TCR chains (αβ or δγ). The CDR3 regions can be extracted by performing a sequence alignment to a canonical TCR. CDRs are the highly variable portions of the TCR sequences which mediate binding to the antigen-MHC complex. In particular, the CDR3 region which spans the junctions between the V(D)J and C regions have the highest variability and represent a truly unique protein fragment that should only be found in the lymphoma cells. Thus, for example, a CDR3 region extended by 10 amino acids both at the C and N termini constitutes the vaccine peptide fragment, as described above for FIG. 8.

Since the TCR has two chains (α & β) there are 2 CDR3s per patient, although in a minority of cases only one CDR3 will be identified.

Once the final vaccine peptide amino acid sequence is identified the design process may include codon optimization to derive a DNA sequence that can be: (i) highly transcribed and highly translated, leading to good protein expression, (ii) amenable to DNA synthesis, (iii) includes adaptor sequences required for the template generation operation, and (iv) excludes sequence motifs such as restriction enzymes that would otherwise interfere with the template generation process. Codon optimization may be performed, e.g., to balance the sequence GC, and remove sequence repeats, internal promoter sequences, termination sequences, splice sequences, recombination sequences, and internal ribosomal entry sites (IRES). In addition, the codon usage may be adapted to that observed in highly expressed human genes. A schematic view of one example of a codon optimization process that may be used is shown in FIG. 10.

Once the sequence design process is completed, the optimized sequence may be synthesized as a linear DNA molecule. A template for IVT may be prepared as described above. For example, prior to mRNA synthesis via IVT, an IVT-capable double-stranded DNA template may be generated. The DNA template may comprise (i) a protein coding sequence (or CDS), defined as the set of codons corresponding to the target patient specific peptide to be produced, (ii) non-coding sequences that include the 5' Untranslated region (5'UTR) and 3'UTR, (iii) a poly-A sequence that protects mRNA from exonuclease activity and (iv) a promoter sequence that recruits the RNA polymerase enzyme that transcribes the DNA template into mRNA, as described in FIG. 6A, above.

Thus, a patient-specific peptide encoding sequence that is a synthetic linear DNA (e.g., from a DNA synthesis vendor) may be paired with the generic functional elements required for IVT, as a template generation process.

The microfluidic path device-based methods and described herein may include template generation and may be much faster and more efficient than currently practiced bacterial culture-based methods which may take ~ 4 days or longer, may result in variable length poly-A tails (due to bacterial recombination processes) and may have a risk of carry-over of bacterial proteins, bacterial DNA and endotoxins. In contrast, the methods and apparatuses described herein can be performed in a single day (~12 hours), and may result in consistent poly A tails (greater than 300 bp), and may not involve any contact with host nucleic acids or host cell proteins. The final double-stranded DNA may be made from chemically produced nucleotides and can thus be considered of synthetic origin.

As described above, these methods may include four steps: (i) ligation of the sGOI with the TIFC, and removal of non-ligated material by an exonuclease treatment, (ii) circular amplification of the ligated product via a technology called multiple displacement amplification (MDA), (iii) linearization of the amplified product using digestion with a type IIs restriction enzyme, (iv) on-chip purification procedure to remove impurities. As a final operation, the purified template may be filtered through a 0.22 □m filter. To ensure the quality of the resulting material prior to use in the IVT reaction a number of analytical tests may be performed, including tests for yield (e.g., > about 50 ug at 1 ug/ul, 260/280 nm ratio > about 1.8), identity (e.g., 100% consensus homology to target), integrity (e.g., < about 1% mutation rate), purity (e.g., > about 95% of product in a single band by CE), and endotoxin (e.g., < about 0.2 EU/ml).

Thus, the drug products may include an mRNA encoding patient-specific TCR peptides together with CpG as an adjuvant mixed in 1:1 ratio. The nucleic acid mix may be encapsulated into 200 nm ANPs that serve to protect the mRNA from degradation by RNases and also act as facilitators for its cellular uptake and cytoplasmic release. Intact mRNA bioavailability in the cytoplasm where the translation process takes place may be required for the active ingredient's mechanism of action. The ANPs are composed of the nucleic acid components, the cationic amine-functionalized peptoid NTX-DV-0024 and 2 wt% PEG-lipid, at an overall ratio of mRNA:DV of 5:1 w/w. The size distribution of the ANPs may be unimodal with a Z-average particle size of approximately 200 nm. ANPs may be suspended in phosphate buffered saline (0.144 mg/mL potassium phosphate monobasic, 9.0 mg/mL sodium chloride, and 0.795 mg/mL sodium phosphate dibasic) at a target pH of 7.4. All formulation excipients may generally be recognized as safe. The final product may be sterile and physiologically isotonic with an osmolality of 295 ± 20 mOsm/kg.

Although mRNA itself has an attractive safety profile, levels of sub-visible particles, host cell proteins (HCP), host cell DNA, process-related impurities, bioburden, bacterial endotoxin levels, sterility, and leachables and extractables, for example, are predominantly safety-related and must be minimized and controlled according to established safety profiles and industry standards. In addition, eliminating or minimizing the presence of residual template DNA, double stranded RNA, and enzymes used to manufacture IVT mRNA may ensure a safe and efficacious product.

As mentioned, the methods and apparatuses described herein may include quantitative analysis of particulate matter, e.g., by one or more procedures, such as a light obscuration particle count test, and/or a microscopic particle count test. It may be necessary to test some preparations by the light obscuration particle count test followed by the microscopic particle count test to reach a final conclusion on conformance to requirements. Since nanoparticle preparations are intrinsically opaque due to light scattering by droplets and/or particle assemblies present in the injection, filtration and subsequent microscopic analysis of the filter may be used for particulate matter analysis. A light microscope adjusted to 100 ± 10 magnifications may be used which allows visualization of particles as small as approximately 1 µm and the nominal pore size of the filter used in the method can be up to about 1.0 µm, drug product nanoparticles in the 100 nm to 250 nm range will not interfere with particulate matter detection.

The methods for template generation described herein do not involve the use of bacteria or any other live microorganisms but relies on the use of enzymatic reaction and chemically manufactured nucleotides, thus the template and mRNA products are fully synthetic.

With respect to residual host cell DNA in the finished drug product, the methods and apparatuses described herein may have less than about 10 ng/dose and about 200 base pairs in the final product dose. In addition to minimizing the presence of process-related impurities, product-related impurities may be controlled through the manufacturing process, formulation development and optimization, and the identification of proper storage conditions described herein. Although the IVT mRNA product are intended to be manufactured and administered as soon as possible, the stability profile may meet defined acceptance criteria until administration at a minimum. The duration for maintaining adequate stability for the therapeutics described herein may be at least about 30 days under refrigerated conditions.

Once IVT mRNA has entered the cytoplasm, its pharmacology is governed by the same cellular mechanisms that regulate the stability and translation of native mRNA. Thus, IVT mRNA potency will largely be dependent on cytoplasmic bioavailability and focus should be on developing the product such that cellular uptake is maximized.

The storage containers (e.g., depots) described herein may generally protect the product from the external environment (including oxygen ingress and protection from photodegradation, if applicable), be sterilizable and ensure that sterility is maintained throughout the shelf-life, be compatible with the product formulation, and contribute minimal little to no leachable chemicals to the product during storage. For example, the depots may include Type I borosilicate glass vials with halobutyl rubber stoppers confer proper product protection and ensure that sterility, safety, and efficacy are maintained throughout the product's shelf-life.

As a non-limiting working example, a preliminary screening maximizing mRNA expression, minimizing impacts on cellular viability, and achieving favorable biodistribution profiles was performed. For these experiments, a bioluminescence assay based on firefly luciferase (Fluc) expression was selected. This assay allows for the quantitative measurement of gene expression resulting from mRNA uptake by each delivery vehicle candidate in a high-throughput fashion. For initial evaluation, thirty-six amino-lipidated peptoids were synthesized for initial evaluation by solid phase peptoid synthesis and isolated by lyophilization and or precipitation. These candidate materials contained structural examples in both their cationic and lipid domains. These thirty-six materials were combined with Fluc mRNA at different ratios along with 2% (w/w) of a lipid-anchored PEG (1,2-dimyristoyl-rac-glycero-3-methoxypolyethylene glycol-2000). Several cell lines were treated with the resulting formulations including HeLa, HepG2, and JAWSII dendritic cells, leading to the down-selection of 6 lead candidates.

*In vivo* mRNA expression and biodistribution of delivery vehicle candidates were quantified by Fluc expression in Balb/c mice following intravenous (IV), subcutaneous (SC), and intramuscular (IM) injections. Based on expression (*in vitro* and *in vivo*) and biodistribution (in-vivo), NTX-DV-0024 was selected as a candidate. mRNA coding for Ovalbumin was synthesized and evaluated as a model vaccine. Ovalbumin is extremely well-studied in the context of vaccination, and reagents are commercially available to track epitope presentation and T-cell responses which make it an ideal candidate for proof-of-concept studies. Initial evaluation of OVA mRNA produced as described herein was conducted on an *in vitro* model using JAWSII murine (C57BL/6) dendritic cells. Briefly, JAWSII cells were transfected with OVA mRNA candidates using commercial transfection reagents (ex. Lipofectamine2000^{™}) for 24 hours, after which time cells were stained using a fluorescent antibody for MHC-I bound to the SIINFEKL epitope. The mean fluorescence intensity (MFI) of the stained population represents a measure of overall antigen presentation. This is described schematically in FIG. 15.

Using this assay, mRNA produced as described herein was evaluated against commercially-purchased material. In this case the produced mRNA resulted in 42% higher levels of SIINFEKL presentation on MHC-I compared to the commercial control. The reproducibility of the on-device mRNA synthesis was also demonstrated, with 5 batches of OVA mRNA (NTX-RNA-0184) resulting in similar levels of SIINFEKL+ JAWSII cells.

mRNA candidates were similarly evaluated as vaccine candidates in a murine *in vivo* experiment. C57BL/6 mice were injected (IV) with commercial or produced mRNA (produced by the microfluidics path devices described herein) and a delivery vehicle. Seven days post-injection, peripheral blood was isolated and stained using a fluorescent MHC-I tetramer specific for T-cells recognizing the OVA epitope. The fraction of OVA-specific CD8+ T-cells was then quantified by flow cytometry. In this experiment, as before, produced mRNA resulted in 50% increase in the fraction of OVA-specific T-cells in peripheral blood relative to the commercial control, indicating the strength of these molecules as vaccination candidates.

The first demonstration the *in vivo* efficacy of an mRNA-based vaccine (produced as described herein) was in murine, OVA-expressing, EG.7, syngeneic T-cell lymphoma model. This model is a physiologically relevant animal model for the indication lymphoma and relevant to the immunotherapeutic mechanism of action of NTX-0565. Syngeneic mouse models are grafts of immortalized mouse cancer cell lines in murine hosts from the same inbred background strain (homograft). The syngeneic host murine background enables immunotherapy-based drugs to recruit a functional anti-tumor immune response from the host, which is necessary for investigating immunotherapies. Syngeneic models feature fully competent murine immunity, diversity of immune cell infiltration into the tumor, comprehensive mouse tumor, immune cells and stroma interface, and ease of tumor synchronization for pharmacology studies including genetic and protein expression histories.

The murine E.G7-OVA Lymphoma tumor model was used. The E.G7-OVA tumor cell line is an EL-4 lymphoma derivative line engineered to carry 1 genomic copy of OVA antigen expressed consistently and robustly. This method enables a highly specific immune reaction to exogenous OVA-based antigens, making these tumor cells ideal for studying cancer vaccines. Tumor growth inhibition has been demonstrated in the literature using DNA, cell-based, and siRNA vaccines. For a first efficacy study, the vaccine component was an mRNA encoding the OVA antigen, and study design was modeled after previous literature on this animal model with an mRNA-based vaccine. A randomized concurrent negative control was run parallel using test article delivery vehicle - phosphate buffered saline. Animals were age and sex-matched and group assignment were randomized to ensure average distribution of tumor size and body weight. Test articles were blinded so that the *in vivo* study director was removed from bias. Sample analysis was blinded again to remove analyst bias. Inclusion and exclusion criteria for acceptance into the study were predefined in the proposal prior to initiation. Endpoints, observation frequency and schedule were predefined in the study proposal prior to study initiation. Euthanasia criteria and animal care interventions were predefined.

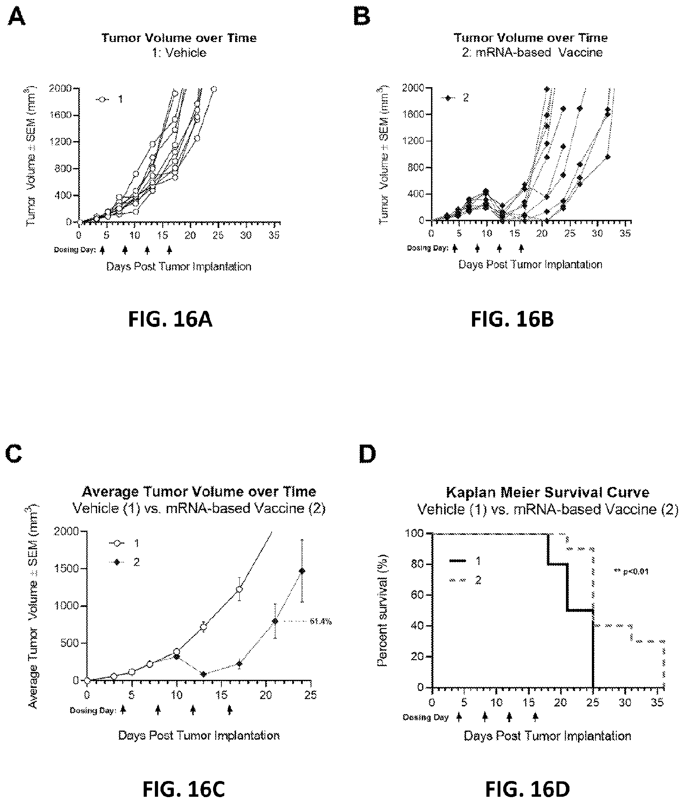

This initial study demonstrated that the mRNA vaccine described herein had statistically highly significant therapeutic efficacy (FIGS. 16A-D). When the OVA mRNA vaccine was delivered intravenously to group 2, the group began showing statistically extreme significance on day 14 post tumor implantation (***, p<0.0005, relative to Neg. Control via Multiple Dunnett's Comparison Test on day 14). On day 21, tumor volumes were 797 mm³ in group 2 versus 2000 mm³ in group 1 control (**, p<0.005 as measured by Multiple Dunnett's Comparisons Test on day 21). This translated into a tumor growth inhibition of 61.42% when animals received the mRNA vaccination manufactured as described herein (IV). Tumor growth inhibition (TGI%) was calculated on day 21 post implantation when the negative control group (Group 1) reached the predetermined endpoint tumor volume (2,000 mm³). Tumor growth inhibition (%) was defined by the following formula: TGI (%) = (TVcontrol group - TVtreated group)/TVcontrol x 100 and is relative to the negative control on day 21 post tumor implantation, when all control animals had reached terminal tumor volume. This tumor growth inhibition translated into a highly statistically significant increase in survival for the mRNA-vaccinated group with Group 2: mRNA-vaccinated animals having a time to endpoint of 25 days versus Group 1: Vehicle-treated animals having a median time to endpoint of 23 days (** p <0.01 as measured by Log Rank Test Against Control). No observable toxicity at the given doses was observed based on weight and laboratory test results. In FIG. 16A-16C a manufactured mRNA-based vaccine shows *in vivo* efficacy in a murine lymphoma E.G7 syngeneic model. Tumor growth was inhibited by 61.4% as seen in the individual animal curves (FIGS. 16A, 16B) and in the average tumor volume for each group (FIG. 16C) (*p<0.01). This tumor growth inhibition translated into a statistically significant increase in survival for the mRNA-vaccinated group (FIG. 16D) (**, p<0.01).

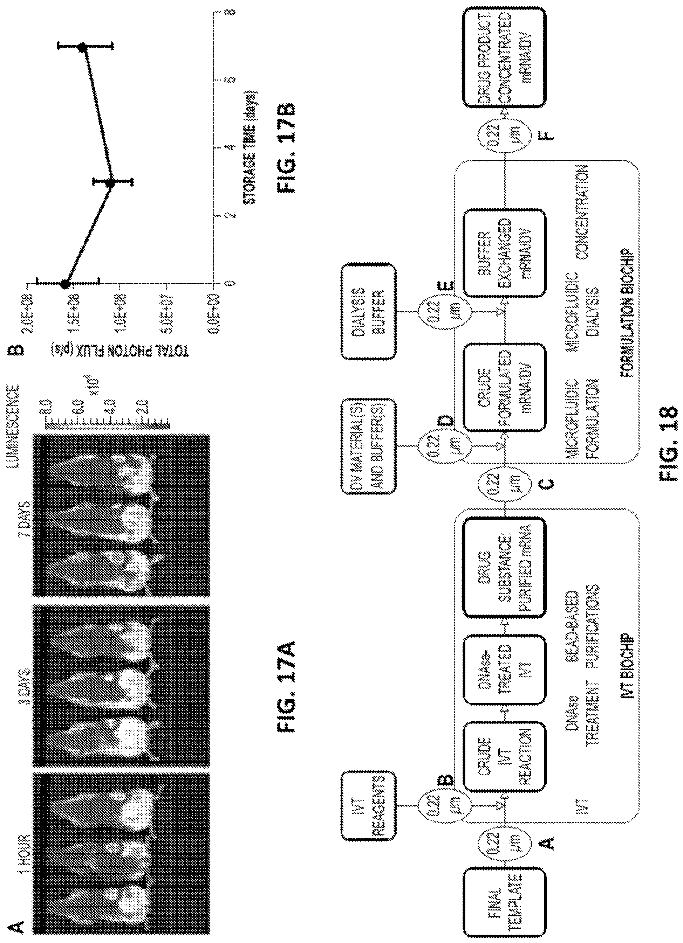

*In vivo* testing of stored drug product formulations using reporter gene expression to supplement the physical measurements above was also performed over a one week time period. In this experiment, formulations of firefly luciferase mRNA and NTX-DV-0028 were prepared 1) 7 days prior to injection, 2) 3 days prior to injection, and 3) 1 hour prior to injection. Post-formulation, products were stored at 4 °C until administration. All three materials were then administered to Balb/c mice at a dose of 0.25 mg/kg via a tail vein injection. 8 hours post-injection, whole-body bioluminescence was measured, and the resulting images are shown in FIG. 17A, and quantified in FIG. 17B (showing quantified photon flux of whole-body luciferase expression following injection with stored mRNA formulations). Over the course of this 1-week storage experiment, there was no substantial loss in measured bioluminescence. Both the 3-day and 7-day stored materials are within error of the material formulated immediately prior to injection. This functional stability data supports the particle size stability data above to strongly indicate that the mRNA formulations described herein are stable to storage at 4 °C for at least 1 week.

In general, an mRNA drug substance formulated with a delivery vehicle molecule into an ANP as described herein may have an approximately 200 nm size, precluding the use of a 0.2 micron sterile filter at the conclusion of the final formulation operation to prevent loss. Therefore, multiple methodical filtration operations may be incorporated throughout the manufacturing process to alleviate sterility risks while avoiding disruption of the final ANP drug product. FIG. 18 schematically illustrates various times at which filtration may be applied. Prior to the IVT reaction, both the purified template and the individual IVT reagents (including dNTPs, enzyme, etc.) may be filtered through 0.22 um filters (FIG. 18 A, B). After mRNA production is completed in the IVT microfluidic path device, all input materials will be filtered prior to the final formulation process (in which ANPs are formed). These include drug substance (mRNA, e.g., FIG. 18, C), adjuvant (CpG), amphipathic peptoid delivery vehicle components and buffers (FIG. e.g., FIG. 18, D), DMG-PEG2000 delivery vehicle component (e.g., FIG. 18, D), and buffer (e.g., FIG. 18, E). In addition to 0.22 micron filtration of the input materials, the final amphipathic nanoparticle drug product may be filtered through a 0.45 micron filter to remove any particulate or aggregated material (FIG. 18, F). This larger filtration operation may help prevent disruption of ANPs and maintain efficacy of the final drug product.

To supplement the discreet filtration operations described above, the microfluidic path device control systems described herein may be designed to operate with a sealed, sterile fluid path which will ensure the safety and sterility of the final drug product, e.g., as descried herein, using one or more sealed microfluidic path devices that conduct the required operations for drug product manufacturing including template preparation, *in vitro* transcription (IVT), formulation with delivery vehicle into amphipathic nanoparticles (ANPs), and buffer exchange and concentration operations. These microfluidic path devices may reside within a temperature-controlled class 5 laminar hood which is further housed, e.g., in 6 x 6 class 7 clean room. The mRNA reactor(s) may be an automated piece of equipment that is protected from human and external environment. The reagent and product fluid path may be delivered to core of the reactor from pressurized, sterile containers using a single use sterile nuclease free tube. The final product formulation and drug product may be manufactured within the multilayer microfluidic path device(s) as described above in a fully closed system.

### PERMEABLE INSERTS

Any of the microfluidic path devices described herein may include one or more permeable inserts for processing the solution of therapeutic material (or the solution in which the therapeutic material is being formed). The permeable insert may be inserted into the fluid-contacting side of a chamber in the microfluidic path device. And may be configured so that fluid entering or passing through the fluid-contacting side of the chamber must pass through, and therefore be modified by, the permeable insert. Any appropriate permeable insert may be used. For example, the permeable insert may include a material that is configured to remove an undesirable material; in some examples the permeable insert includes a cellulose material that is configured to remove double stranded RNA (dsRNA) from a therapeutic solution of single stranded RNA (ssRNA).

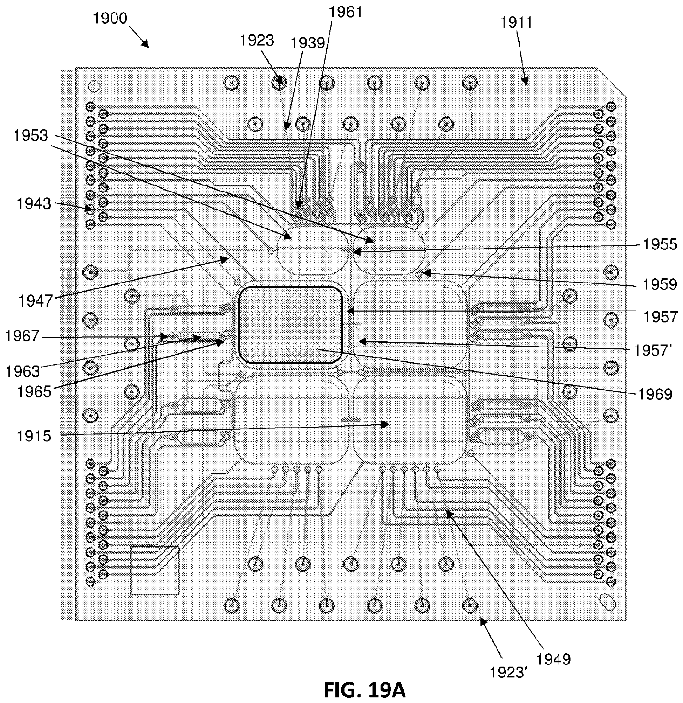

FIG. 19A shows one example of a microfluidic path device 1900 including a permeable insert 1969 within the fluid-contacting side of a chamber 1957. In FIG. 19A a microfluidic path device 1900 may include at least one pair of chambers 1953, 1957, 1957', each of which may include a fluid-contacting side 1917, a pressure (e.g., gas) side 1919, fluidic connections, pressure connections and fluidic/pressure lines that may be formed in the thickness of the microfluidic path device. In some examples the chambers are paired, and each chamber of the pair of chambers may be connected to each other by a fluidic connector 1955. The fluidic connector 1955 may be used in coordination with positive and/or negative pressure applied to the pressure side of the chamber(s) to drive liquid in the liquid side between the two chambers to mix this liquid within each of the chambers. The chamber may be bifurcated by an elastic material (e.g., an elastic layer or membrane) and deflecting an elastic material within the fixed volume of a chamber may drive any liquid within the liquid in/out of the fluid-contacting side of the chamber (e.g., between the two chambers).

The microfluidic path device 1900 may include more than one pair of chambers, any of which may include a permeable insert. Each pair of chambers may be used for different processes. For example, a first pair of chambers 1953 may be used for synthesis of the RNA. A second pair of chambers 1957, 1957' may be used for purification of the synthesized polynucleotides. Fluid from a first pair of chambers 1953 may be driven to a second pair of chambers upon application of pressure to the pressure-receiving side 1919 of the respective chambers and opening a valve 1959 between the first pair of chambers 1953 and the second pair of chambers 1957. The valve chamber 1959 may be formed by the elastic layer 1907 within a connector channel between the two pairs of chambers.

The microfluidic path device 1900 as shown in FIGS. 19A and 19B may have a plurality of pressure ports 1943 and fluid ports 1923, 1923'. The plurality of pressure ports and fluid ports may be disposed adjacent to a periphery of the microfluidic path device, and are configured to be connected to the fluid interface assembly 109 as described above.

Ports (e.g., sealing valves) may be formed from the elastic layer, along the length of a connecting channel 1939 (either pressure channel or fluid channel), such as is shown in FIG. 19A, for valve 1961, which may control timing of delivery of a reagent driven from fluid port 1923, but when placed in series with one or more similarly constructed valves, may also permit metering to the chambers of the device. For example, in FIG. 19A, three valve chambers are shown (described in greater detail below); the first of these three valves may act as a peristaltic pump, while the middle valve may be a metering chamber that meters small (e.g., having a metering volume of about 10 nL, about 20 nL, about 25 nL, about 50 nL, about 75 nL, about 100 nL, etc.). The size of the channels, and particularly the size of the chambers connected to the channels) can meter out the volume dispensed along fluidic connecting channel 1939, 1921 and delivered into the chamber 1953 that is connected to the fluidic connecting channel 1939, 1921. In some examples, a metered volume may be as little as 50 nL. Metered volumes of about 100 nL, about 1 microliter, about 5 microliters or more may be imported. A variety of valve sizes may be pre-selected for incorporation within the microfluidic path device 1900, and reagents may be connected to appropriate metering sizes by user choice.

Additionally, more than one valve body 1961 may be included in a row along fluidic connecting channel 1939. A series of valves 1961 may act as a peristaltic pump to move fluid, including (but not limited to) viscus fluids. The ability to function as a peristaltic pump for fluids generally, may have particular advantage for moving fluid that may be viscous or contain suspended particles such as purification or capture beads.

As mentioned, a microfluidic path device 1900 may also include a delivery or export reservoir or depot 1963. In FIG. 19A, a pre-selected volume may be formed similarly to the chamber construction described above, or may contain only a metering side, as desired. In either case, valves may be used to meter desired volumes into the reservoir 1963. Valve 1965 can control delivery of fluid from reservoir 1963. If larger volumes are desired, the delivery may be repeated. Alternatively, if reservoir 1963 was pre-selected to be an export reservoir, valve 1965 may open, and deliver fluid from chamber 1957, while retaining valve 1967 shut, which permits only the measured volume of fluid to be exported to reservoir 1963. This fluid may then be exported to a fluid vial on the reagent storage frame for further processing or testing. In some examples, a chamber, reservoir or depot (e.g., 1963) may be configured as a metering section of, e.g., a 1 µL pump formed by three valve structures (1967, 1965, 1967). A chamber may be configured for export of waste, for example, from a mixing chamber 1957.

The microfluidic path device 1900 can be a sealed path construction. While fluid vials, fluidic lines and the microfluidic path device are connected, operation of the apparatus may be performed without any exchange of materials in or out of the system, and in particularly in/out of the fluid path of the microfluidic path device for processing, including synthesizing a polynucleotide (RNA) and preparing it for biological delivery (as a therapeutic, such as drug, vaccine, etc.). Thus the entire system may operate as a closed path and/or individual microfluidic path devices may operate in the system as a closed path (protected from the atmosphere).

In general, these microfluidic path devices can include incorporating one or more permeable inserts 1969 within the fluid side 1917 of a chamber or channel. The permeable insert may be configured to absorb selected moieties (e.g., selected material) from the fluidic mixture in a chamber or channel. The absorbed material may be an unwanted material that is purified out of the solution, or it may be a desired material that is removed from the solution to be later eluted and further processed. In one example, the permeable material of the insert may include a cellulose material, which can selectively absorb double-stranded mRNA from a mixture. The cellulose material may be inserted in only one chamber of a pair of chambers, such that upon mixing or passing the fluid through the permeable insert in the first chamber, dsRNA may be effectively removed from the fluidic mixture, which can then be transferred to another pair of chambers further downstream for further processing or export.

Some examples of the microfluidic device 1900 may further include a concentrator within a chamber, which may be disposed within the thickness of the second plate and may be in fluid communication with an exit channel such as 1949. The polynucleotides may be concentrated by driving off excess fluidic medium, and the concentrated polynucleotide mixture exported out of the microfluidic path device 1900 for further handling or use. In some examples, the concentrator may be a dialysis chamber. For example, a dialysis membrane may be present within or between the plates of a microfluidic path device.

The microfluidic path device 1900 may be formed of materials that are at least substantially translucent to visible and/or ultraviolet light. By substantially translucent is meant that at least about 90% of light is transmitted through the material compared to a translucent material. In some examples, the microfluidic path device 1900 may be formed of materials that are substantially transparent to visible and/or ultraviolet light. By substantially translucent is meant that at least 90% of light is transmitted through the material compared to a completely transparent material.

The microfluidic path device may be formed of two or more plates that are layered atop each other with the chambers and/or channels formed between the plates; an elastic material may be sandwiched between the first and second plates. The first plate and/or the second plate may be formed from a rigid material. The plates may be formed of the same material, or a different material(s). For example, the rigid material may be a polymer or glass. The polymer or glass may be biocompatible, e.g., does not leach any monomers or soluble small molecules that are toxic to living cells. Any suitable biocompatible polymer may be used, including medical grade polycarbonate-urethane, silicone polycarbonate urethane, polyether urethane, amongst others. In some examples, the polymer may be a cycloolefin copolymer.

FIG. 19B shows a section through a portion of a microfluidic path device, showing a permeable insert 1969 within the fluid-contacting side 1917 of a chamber 1920 that is bifurcated by an elastic material 1907 into a fluid-contacting side and a pressure-receiving side 1919. Thus, a microfluidic path device may be configured as multilayered structure composed of two more rigid layers 1903, 1905 with a flexible membrane 1907 sandwiched between the two ridged layers. FIG. 19B shows a portion of a sectional view (transverse to the plane of the microfluidic path device) through one example of a microfluidic path device having multiple layers that form reactors for processing a therapeutic as described herein. The reactors may include seals, channels, valves, and chambers, including pumping chambers formed from the multiple layers. For example, a microfluidic path device may be formed of two or more rigid or semi-rigid plates 1903, 1905 and at least one elastic layer 1907. The elastic layer 1907 may be a sheet of elastic material that is liquid-impermeable. The elastic layer maybe somewhat gas permeable or may be treated to be more or less gas permeable, including in various regions. Although a single continuous sheet of elastic material may be used, in some examples multiple elastic materials sheets may be used, or the 'sheet' may be formed of sections of multiple sheets. The layers and the elastic sheet may be laminated together. In general, chambers for holding, valving and/or pumping fluid may be formed in the plates on either side of the elastic layer so that the elastic layer bisects the chambers into a liquid containing side and a pressure (e.g., gas) applying side. The overall volume of chamber(s) may be constant and may be formed into both the first (e.g., upper) plate and the second (e.g., lower) plate, but this volume may be divided into the pressure side and the liquid side. By applying positive or negative pressure into the pressure side, the elastic sheet may be deformed to make reduce (down to zero, closing the chamber off) the volume of the liquid containing side or to increase the volume of the liquid containing side (to a predetermined maximum). The pressure applying side of the chamber may be connected, e.g., via a pressure port 1943 in the upper plate 1903 connecting to a pressure channel 1947, for applying negative or positive pressure to the pressure-receiving side 1919 of one or more chambers. The liquid containing side 1917 opposite the pressure-applying side of each chamber may be connected via a fluid channel 1921 to a fluid port 1923. Both the fluid port and the pressure port may be formed by an opening into the upper plate 1903 and the elastic layer 1907, allowing a sealed connection that is isolated from the atmosphere even when there are multiple different input lines as the pressure line is pushed into the elastic layer 1907 that is supported on the underside of the port by the opposite rigid or semi-rigid layer(s), 1905, 1909.

In FIG. 19B, the microfluidic path device 1900 includes a first (e.g., upper) plate 1903 having a first (e.g., top or upper) surface 1911 and a second (bottom or lower) surface 1929 and a thickness between the two. The first surface 1911 may form an exposed outer surface. The microfluidic path device also includes a second plate 1905 having a first (e.g., upper or top) surface 1931 and a second (e.g., lower or bottom) surface 1933 and a thickness therebetween. An elastic layer 1907 is sandwiched between the second surface 1929 of the first plate 1903 and the first surface 1931 of the second plate 1905. In this example, a third plate 1909 is coupled to the second plate on the second surface 1933 of the second plate, either directly or indirectly. The third plate 1909 also has a first (e.g., upper or top) surface and a second (lower or bottom) surface and a thickness therebetween. The second surface of the third plate may form a bottom surface of the microfluidic path device. Any of the plates may be formed of multiple layers, which may be laminated or otherwise connected together. For example, in FIG. 19B, the third plate 1909 includes an optional second elastic layer 1913 which may help couple the third plate to the second plate; the second elastic layer 1913 in this example forms the first surface 1935 of the third plate 1909. The layers and plates shown in FIG. 19B may not be to scale (e.g., the elastic layer 1907 may be thinner relative to the plates).

The microfluidic path device 1900 shown in FIG. 19B may also include a plurality of chambers 1915, 1916, 1918, 1920 each having a fixed volume. These chambers are formed by cut-out regions (e.g., rounded/curved cuts) into the second (bottom) surface 1929 of the first plate 1903 and the first (upper) surface 1931 of the second plate 1905; the elastic layer 1907 bifurcates these chambers 1915 so that each includes a liquid containing side 1917 and a pressure-receiving (e.g., gas containing) side 1919. The microfluidic path device 1900 may also include multiple liquid (e.g., fluid) channels. In FIG. 19B, a single fluid channel 1921 is shown extending from a fluid port 1923 passing through the thickness of the first plate 1903, to a fluid channel opening 1925 through the elastic layer 1907 and through much of the thickness of the second plate 1905 down to the bottom surface 1933 of the second plate where a length of the liquid channel 1921 running parallel to the bottom surface of the third plate is formed in the bottom surface 1933 of the second plate, and bounded by the upper surface of the third plate 1909.

In regard to the fluid port 1923, the diameter of the opening into the first plate 1903 forming the fluid port 1923, which extends through the thickness of the first plate, may be larger than the diameter of the fluid channel opening 1925 which extends through the elastic layer 1907 and into the liquid (e.g., fluid) channel 1921. The fluid channel opening 1925 may be centered relative to the bottom of the fluid port opening and may be offset from the walls of the fluid port opening by at least the expected wall thickness of the fluid line or fluid line coupling interface that will connect to the fluid port.

The fluid channel 1921 connects to the liquid containing side 1917 of a first chamber 1915. This first chamber may be configured as a valve, which has a relatively low retaining volume (fixed volume) but can be fully opened or closed by the movement of the elastic layer 1907.

The microfluidic path device 1900 also includes a plurality of pressure channels that may be independently controlled to apply positive and/or negative pressure. In FIG. 19B, a single pressure port 1943 is shown, connected to the fourth chamber 1920, although each of the chambers 1915, 1916, 1918 may be connected to a separate pressure port and pressure channel for independently operating and controlling the movement of the portion of the elastic layer 1907 bifurcating these chambers, to valve, and/or pump each chamber independently. In some examples the pressure ports may be shared between multiple chambers. In FIG. 19B the pressure (e.g., gas) port 1943 is similar to the fluid (e.g., liquid) port 1925, and includes an opening completely through the first plate 1903, down to the exposed elastic layer 1907, to an opening through the elastic layer forming a pressure (e.g., gas) channel opening 1945. The pressure channel opening 1945 is continuous with a pressure (e.g., gas) channel 1947 that extends from the pressure port 1943, passing through much of the thickness of the first plate 1903, and in a cut-out channel along the bottom of the second plate (or alternatively into a cut-out region in the top of the third plate) and back up through the second plate and the elastic layer 1907, to a region of the pressure channel within the first plate that connects to the pressure (e.g., gas) containing portion 1919 of the fourth chamber 1920. As described for the similar fluid (e.g., liquid) port, the diameter of the pressure port 1943 passing through the thickness of the first plate 1903 may be larger than the diameter of the pressure channel opening 1945 through the elastic layer 1907 and may be centered or offset by greater than the wall thickness of a pressure line or pressure line coupling interface that will connect to the pressure port.

In the section through a microfluidic path device 1900 shown in FIG. 19B, there are multiple connections to other fluid (e.g., liquid) lines, fluid ports, pressure lines and pressure ports that are not shown, as they may be out of the plane shown. For example, in FIG. 19B the liquid containing side or portion 1917 of the fourth chamber may be connected to additional valves (chambers) and/or channels, including, e.g., an exit channel extending from the liquid containing side 1917. An additional chamber (e.g. configured as a valve), no shown may be formed as described above. In some examples, an exit channel may deliver fluid from the one or more chamber through another fluid port (not shown) to a fluid receiving depot, e.g., a vial, tube, etc. This receiving depot may be held in the reagent storage frame.

As mentioned above, the permeable insert 1969 may be inserted into the fluid-contacting side of the separation chamber and may be configured to be compressed by the elastic material separating the fluid-contacting chamber from the pressure-receiving side of the chamber. In this example, the positive or negative pressure applied to the receiving side (e.g., via a pressure port that is addressed to this chamber) may deflect the elastic material to change the volume of the fluid-contacting side. Fluid may be driven into the chamber 1920 with the permeable insert 1969, and the fluid may be passed through the insert to modify the solution. In examples in which the permeable insert is compressible it may be compressed to remove and eject the fluid from the chamber; in some examples the permeable insert may then be expanded (or allowed to expand) back to the expanded configuration and fluid may then be passed through it again, or further processing may be performed.

The methods and apparatuses described herein may also be used with or without a permeable insert for making a synthetic product comprising a synthetic DNA template suitable for in vitro transcription. For example, FIG. 19B shows one example of a microfluidic path device (e.g., "chip" which may be or may be part of a cartridge) 1980 that does not necessarily include a permeable insert within the fluid-contacting side of a chamber. In FIG. 19C the microfluidic path device 1980 includes four PCR chambers 1983, 1983', 1983", 1983‴, each of which may include a fluid-contacting side, a pressure (e.g., gas) side, and which are fluidically connected to adjacent PCR chambers. Each PCR chamber has a fixed volume, and, as described above for general chambers, are formed between a first surface of a first plate and the second surface of a second plate; the first and second plates may be joined together with an elastically deformable membrane (e.g., elastic layer) between them, dividing the chamber. The elastic layer divides each chamber into a fluid-contacting side in the second surface and a pressure-receiving side in the first surface.

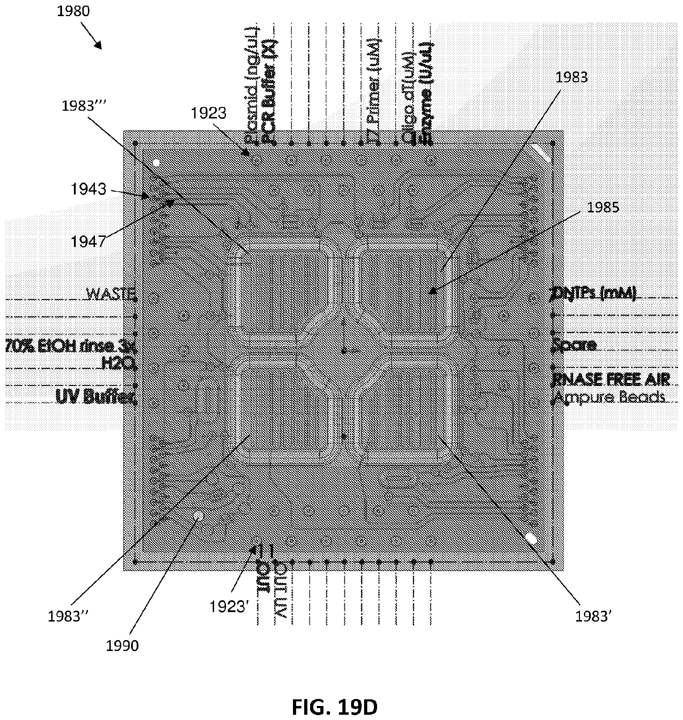

In the microfluidic path device shown in FIG. 19D, the pressure-receiving sides 1919 of each chamber is further partitioned up by one or more fluidly-connected serpentine pathways 1985. These serpentine pathways in the pressure receiving surface to distribute the positive and negative pressure applied through the channels (and in particular, the negative pressure)more evenly across the surface of the relatively large chamber. The sub-division of the pressure-receiving surface in each PCR chamber (e.g., in some examples by one or more serpentine pathways) may support the deflectable membrane when negative pressure is applied to pull it away from the fluid-containing chamber. This may also prevent the formation of bubbles and may maintain a fixed, predictable volume.

The microfluidic path device shown in FIG. 19D also includes a plurality of fluid channels each extending from a fluid port 1923, 1923' through the first plate region and into the second plate region to fluidly connect with the fluid-contacting side of one or more of the plurality of chambers (similar to the configuration shown in FIGS. 19A and 19B. In FIG. 19D, a subset of fluid ports are labeled, including those providing fluidic connection to a source of (off-device) plasmid 1923, PCR buffer, primer(s) (e.g., T7 primer), oligo dTs, enzyme (e.g., polymerase), purification substrate (e.g., Ampure^{™} beads), RNASE-free air, dNTPs, product output ("OUT") 1923, output from a UV yield detection channel ("OUT UV"), buffer for UV detection ("UV buffer"), water, Ethanol (e.g., 70% Ethanol rinse), and waste ("Waste"). Additional fluid ports are also included and may be redundant or may not be used. The microfluidic path device also includes a plurality of pressure ports 1943. As described, the pressure ports may provide communication for the application of positive and/or negative pressure to the pressure-receiving sides of each chamber, channel, vacuum cap, valve, etc. Thus, a controller may control movement of fluid within the device, including mixing, pumping, valving, etc., by applying positive or negative pressure to specific pressure ports 1943 or combinations of pressure ports. The pressure ports and fluid ports may be arranged on an upper side of the first plate, typically around a periphery of the plate, as shown in FIGS. 19A and 19D.

In FIG. 19D, the device also includes a plurality of pressure channels 1947 each extending from one or more pressure ports, through the first plate region and elastic layer, into the second plate region, and back through the elastic layer and into the first plate region (similar to that shown in FIG. 19B), wherein each pressure channel of the plurality of pressure channels extends within the first plate region and fluidly connects with one or more pressure-receiving sides of one or more of the plurality of chambers. As shown in FIG. 19B, the application of positive or negative pressure through the pressure channels (from the pressure port) by the controller of the system including a fluid depot and pneumatic drive may open/close valve 1915, 1918, and may pump fluid through the chambers 1916, 1920, 1983.

Any of the microfluidic path devices described herein may also include one or more UV yield detection chambers 1990 in fluid communication with one or more of the PCR chambers. The UV yield detection chamber may include a UV yield detection window that is configured to pass UV light therethrough for quantification of a polynucleotide within the UV yield detection chamber. The UV yield detection chamber 1990 may also be connected to a source of buffer for performing the UV detection. UV detection may be measured the absorbance of the buffer that the DNA is in. The apparatus (system), including a controller coordinating the operations on the microfluidic path device may be configured to control operation of the UV yield detection chambers, as will be described in detail below. For example, the controller of the system for which the microfluidic path device is configured to be used may first examine the absorbance of the UV buffer without the product, and may then add a predetermined amount of the (e.g., purified) product for comparison. The system (e.g., controller) may then automatically or semi-automatically use the determined concentration to alert the user and/or to dilute the product before it is exported from the microfluidic path device or moved to another microfluidic path device.

In general, the device shown in FIG. 19D is similar and may include any of the features of that shown in FIG. 19A (and 19B-19C). For example the ports may be formed from the elastic layer, along the length of a connecting channel 1939 (either pressure channel or fluid channel), such as is shown in FIG. 19A. One or more valve bodies 1961 may be included in a row along fluidic connecting channel 1939.

The PCR chambers may be configured to optimize the PCR processes described herein. For example, the microfluidic path device may include PCR chambers having a much larger area than height of the fluid-contacting side. For example, the fluid-contacting side of each PCR chamber may have a thickness that is 1.5 cm or less, such as in particular 1.3 cm or less, 1.2 cm or less, 1.1 cm or less 1.0 cm or less 0.9 cm or less, 0.8 cm or less, 0.7 cm or less, 0.6 cm or less, or 0.5 cm or less. Typically, the lower the height (e.g., the "thickness" of the chamber), the more efficient the heat transfer due to thermal cycling may be, however the lover the overall volume. The microfluidic path devices described herein may be used for PCR within the PCR chambers without requiring the addition of an oil/hydrophobic material, as evaporation may be limited by the closed (or closable) configuration of the chamber.

In general, any of these microfluidic path devices may include a purification chamber in fluid communication with a purification substrate (e.g., Ampure beads).

The microfluidic path devices described herein may also be configured to provide mixing (e.g., bubble mixing) by applying air (e.g., RNAse-free air) through the fluid-contacting side of the apparatus from the microfluidic path device, and out of a fluid port into a reservoir coupled to the fluid port. In FIG. 19D, for example, the microfluidic path device may be controlled by a controller (of a system to which the microfluidic path device is coupled) to mix and resuspend substrate beads (e.g., Ampure^{™} beads) within a reservoir coupled to a fluid port by driving RNAse-free air through the fluid channels in communication with the fluid port. This may result in bubbling and mixing of the substrate beads within the reservoir. The controller may control (using positive and/or negative pressure applied through the pressure ports of the microfluidic path device) the mixing by application of the RNAse-free air and after mixing, may direct resuspended substrate out of the reservoir and into the purification chamber(s) for purifying template product.

As mentioned, any of these apparatuses may be configured as a removable cartridge configured to engage with a fluid depot and pneumatic drive, and may be coupled to system, e.g., a microfluidic path device control system that includes a controller for coordinating the operation of the microfluidic path device to produce template.

In general, a microfluidic path device may be any appropriate size/volume. For example, the microfluidic path devices may be configured to have a total PCR reactor size of between about 3 mL and about 10 mL (e.g., between about 4 mL and about 8 mL, between about 5 mL and 7 mL, etc.). In the example shown in FIG. 19D the total PCR reactor volume (the combination of all four PCR chambers) is about 6.03mL. Thus, in FIG. 19D the PCR reaction volume is about 3 mL, which ahs been found to produce about 130 ng/uL of template product (within the 3 mL).

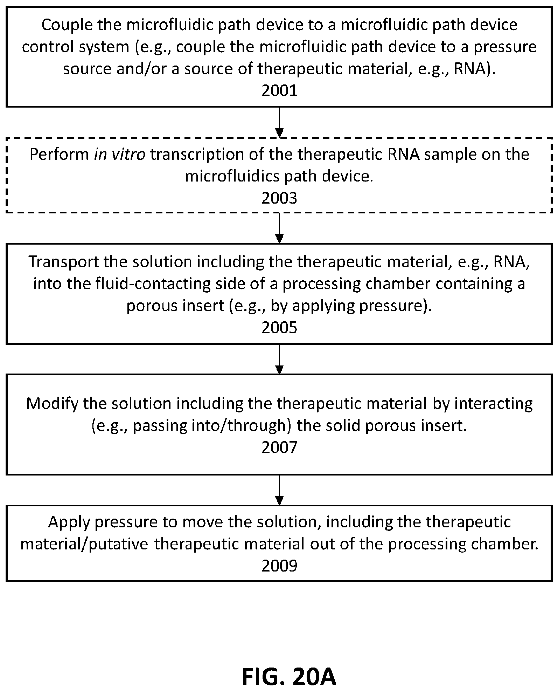

FIG. 20A schematically illustrates one example of a method of processing a therapeutic material in a fluid (e.g., an RNA sample) using any of the apparatuses described herein. For example, the method may include first attaching the microfluidic path device (or more than one microfluidic path device) to a microfluidic path device control system 2001. This may include coupling a microfluidic path device to a pressure source. In some examples, this operation (or an additional operation) may include coupling the microfluidic path device to a source of the therapeutic material, such as RNA. Optionally, in some examples the method may include synthesizing, in the microfluidic path device, the therapeutic material, such as generating the therapeutic RNA by *in vitro* transcription 2003. As mentioned, any of these devices and methods may optionally include permeable inserts as described herein to modify a solution including the therapeutic material (or in which the therapeutic material is being formed).

The method may further include transporting the sample with the therapeutic material (e.g., RNA) to a fluid-contacting portion of a processing chamber containing a permeable insert 2005. For example, this may include applying pressure to transport the sample to the fluid-contacting side of a separation chamber of the microfluidic path device. In some examples, pressure may be applied by deflecting an elastic material (e.g., an elastic membrane) in within the microfluidic path device to drive the fluid including the therapeutic (or putative therapeutic material) into the fluid-contacting side of the chamber. As part of this operation, the fluid (including the therapeutic/putative therapeutic) sample may be passed into the permeable insert within the fluid-contacting side of the separation chamber, to modify the sample 2007. For example, material from the therapeutic/putative therapeutic may be added or removed by interacting with the permeable insert.

Finally, pressure may be applied to transport the sample out of the fluid-contacting side of the separation chamber, e.g., by deflecting an elastic material, such as an elastic membrane, separating the fluid-contacting side of the chamber from the pressure-receiving side of the chamber 2009.

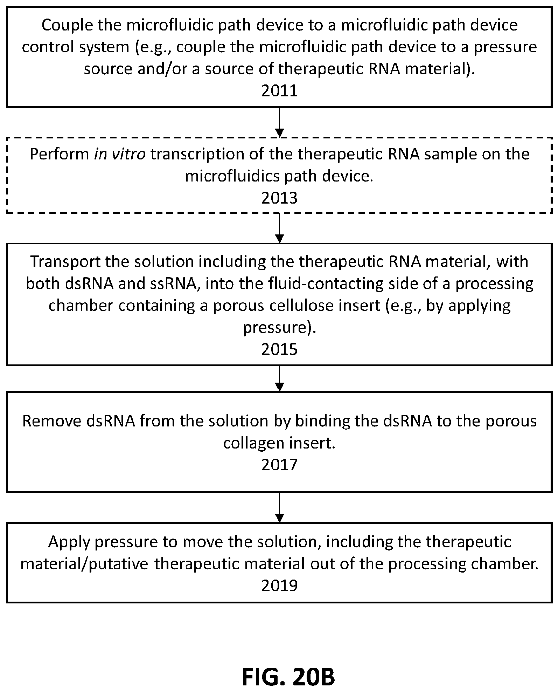

FIG. 20B illustrates a specific example of a method of processing a therapeutic material in a fluid (e.g., an RNA sample) using any of the apparatuses described herein. For example, in FIG. 20B, the method may be a method of removing double-stranded RNA (dsRNA) from an RNA sample containing both dsRNA and single-stranded RNA (ssRNA). In this example, the method may include: coupling a microfluidic path device to a pressure source 2011. As mentioned, in some examples this may include coupling the microfluidic path device to a source of the therapeutic RNA, and/or performing *in vitro* transcription of the therapeutic RNA in the microfluidic path device, as described above 2013. The method may then include applying pressure to transport the RNA sample to a fluid-contacting side of a separation chamber of the microfluidic path device 2015. The RNA sample may then be passed through/into a solid and permeable insert, which comprises collagen, within the fluid-contacting side of the separation chamber 2017, wherein the cellulose binds the dsRNA, so that dsRNA is retained by the insert. Pressure may then be applied to transport the RNA sample out of the fluid-contacting side of the separation chamber, leaving the ssRNA in the therapeutic solution 2019. The processes may be repeated as necessary to remove all or substantially all of the dsRNA.

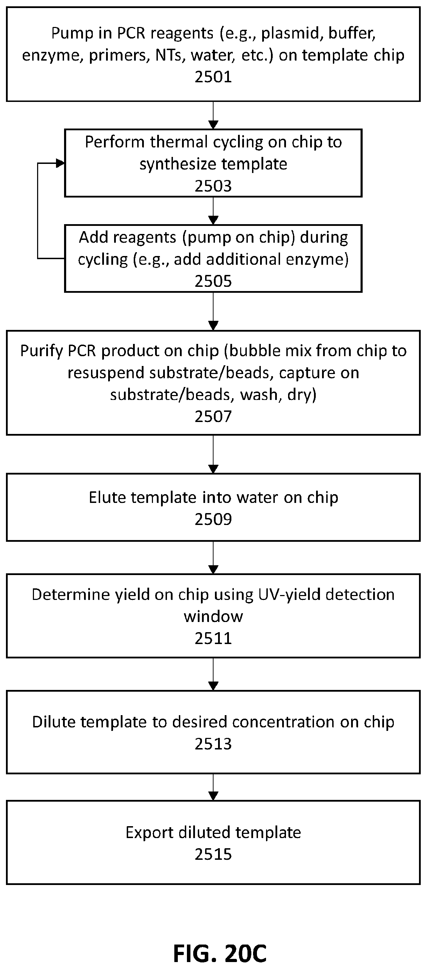

FIG. 20C illustrates another example of a method of making a synthetic product comprising a synthetic DNA template suitable for in vitro transcription. Any of the features or steps (or parts of steps) described above may be included and/or combined with the method shown in FIG. 20C. For example, any of these methods may include a UV yield detection chamber (having a UV yield detecting window) for detection of product. Any of these methods and apparatuses may also be configured to apply bubble mixing (e.g., to resuspend substrate beads in vial) through the device, as described above. The method may generally include PCR amplification using one or more reactors (e.g., a reactor divided up into four connected chambers, a shown in FIG. 19D). The methods and apparatuses descried herein may be configured for purification of the template product (e.g., using a purification substrate) and/or may be configured to diluting the resulting product in a controlled manner and eluting the product. Any of these steps may be performed automatically by the controller and/or semiautomatically under the guidance of a user.

For example, any of these methods may include pumping the reagents for the PCR reaction for forming the template into the PCR chamber(s) 2501, under the control of the controller. For example, the method may (e.g., FIG. 20C) may include delivering the PCR reagents (e.g., plasmid, buffer, enzyme, primers, NTs, water, etc.) to the PCR chamber(s) o the template microfluidic path device. The controller may then coordinate the amplification of the template, e.g., by thermal cycling the PCR chambers to synthesize the template 2503. At any point during the thermal cycling, the apparatus may manipulate the liquid flow to add a desired amount of additional component (e.g., enzyme, dNTPs, etc.) before or during any step of the PCR reaction 255. In particular, the methods described herein may include adding additional enzyme (polymerase) during the PCR reaction, such as, e.g., at the 15th thermal cycle (e.g., at the 12^{th} cycle, at the 13^{th} cycle, at the 14^{th} cycle, at the 15^{th} cycle, at the 16^{th} cycle, at the 17^{th} cycle, etc.). This may increase the yield; in some cases by as much as 20% when added at the 15^{th} cycle.

Once the thermal cycling is complete, the apparatus may purify the PCR product in the microfluidic path device. For example, the microfluidic path device may first mix the purification substrate, such as beads (e.g., Ampure^{™} beads) by applying bubble mixing as described above through the microfluidic path device, and may combine the suspended substate with the product 2507. Product may be bound onto the substrate, washed (e.g., using wash buffer) and dried (e.g., using 70% EtOH) all within the microfluidic path device, including in one or more purification chambers. The purified product may then be eluted from the substrate within the microfluidic path device and a small fraction of the eluted product may then be passed to the UV yield detection chamber so that the concentration may be determined 2511.

For example, the UV yield detection chamber may include a UV yield detection window through which UV light may be passed to measure absorbance using a spectrophotometer forming part of the system. UV detection buffer may be applied into the UV yield detection chamber so that a concentration measurement may be taken. For example, UV detection buffer may be applied into the UV yield detection chamber and a baseline level taken. Thereafter, eluted template product (e.g., approximately 5 uL) may be transferred to the UV yield detection chamber and combined with UV detection buffer; UV light may then be applied and the concentration of the yield determined by spectrophotometry. The system may then either elute the product or otherwise pass it on to another microfluidic path device and/or transfer it off of the microfluidic path device, or it may dilute it to a desired concentration 2513, and eluted 2515. Multiple concentration detections steps may be performed using the same or different UV yield detection chambers.

As mentioned above, further processing (combining with a delivery vehicle, dialysis, concentration, etc.) may then be performed.

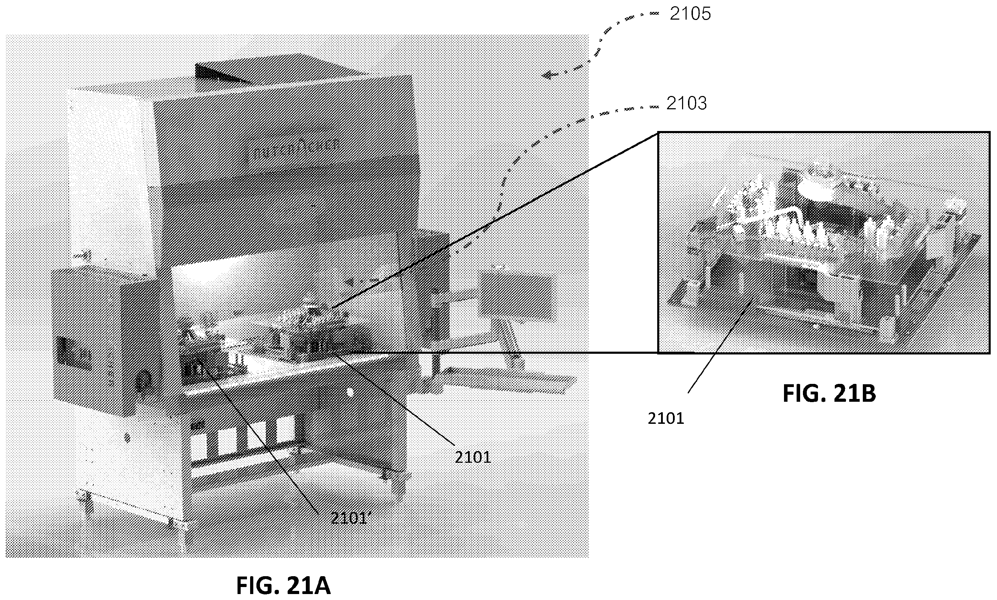

The apparatuses described herein may include and/or may be used with one or more isolation chambers. For example, in some examples the apparatuses described herein may be part of a therapeutic polynucleotide manufacturing 'factory' that may produce therapeutic polynucleotides, e.g., for delivery to a subject. The therapeutic polynucleotide may be, e.g., a therapeutic mRNA. FIGS. 21A-21B illustrate one example of a system 2105 that may include an apparatus to be used by itself as a factory apparatus or that may be used as part of a parallel manufacturing unit. In FIG. 21A the apparatus(s) 2101, 2101' may include or may be held in a class 5 isolation cabinet 2103; the isolation cabinet may itself be held within a class 7 isolation space. In FIG. 21A the cabinet includes two microfluidic control apparatuses 2101, 2101'. The apparatuses may be part of an assembly factory providing copy-exact GMP units that may automatically manufacture therapeutic polynucleotides, such as therapeutic mRNA rapidly for patient use. These apparatuses may be highly reconfigurable and allow for rapid deployment and low-cost production. In some examples they may be deployed on-demand manufacturing "factory" units. In some examples these apparatuses may be set up as part of a mobile unit that may be deployed to a remote site temporarily or for a longer time period.

It should be appreciated that all combinations of the foregoing concepts and additional concepts discussed in greater detail below (provided such concepts are not mutually inconsistent) are contemplated as being part of the inventive subject matter disclosed herein and may be used to achieve the benefits described herein.

The process parameters and sequence of steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like. For example, any of the methods described herein may be performed, at least in part, by an apparatus including one or more processors having a memory storing a non-transitory computer-readable storage medium storing a set of instructions for the processes(s) of the method.

While various embodiments have been described and/or illustrated herein in the context of fully functional computing systems, one or more of these example embodiments may be distributed as a program product in a variety of forms, regardless of the particular type of computer-readable media used to actually carry out the distribution. The embodiments disclosed herein may also be implemented using software modules that perform certain tasks. These software modules may include script, batch, or other executable files that may be stored on a computer-readable storage medium or in a computing system. In some embodiments, these software modules may configure a computing system to perform one or more of the example embodiments disclosed herein.

As described herein, the computing devices and systems described and/or illustrated herein broadly represent any type or form of computing device or system capable of executing computer-readable instructions, such as those contained within the modules described herein. In their most basic configuration, these computing device(s) may each comprise at least one memory device and at least one physical processor.

The term "memory" or "memory device," as used herein, generally represents any type or form of volatile or non-volatile storage device or medium capable of storing data and/or computer-readable instructions. In one example, a memory device may store, load, and/or maintain one or more of the modules described herein. Examples of memory devices comprise, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In addition, the term "processor" or "physical processor," as used herein, generally refers to any type or form of hardware-implemented processing unit capable of interpreting and/or executing computer-readable instructions. In one example, a physical processor may access and/or modify one or more modules stored in the above-described memory device. Examples of physical processors comprise, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the method steps described and/or illustrated herein may represent portions of a single application. In addition, in some embodiments one or more of these steps may represent or correspond to one or more software applications or programs that, when executed by a computing device, may cause the computing device to perform one or more tasks, such as the method step.

In addition, one or more of the devices described herein may transform data, physical devices, and/or representations of physical devices from one form to another. Additionally or alternatively, one or more of the modules recited herein may transform a processor, volatile memory, non-volatile memory, and/or any other portion of a physical computing device from one form of computing device to another form of computing device by executing on the computing device, storing data on the computing device, and/or otherwise interacting with the computing device.

The term "computer-readable medium," as used herein, generally refers to any form of device, carrier, or medium capable of storing or carrying computer-readable instructions. Examples of computer-readable media comprise, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

A person of ordinary skill in the art will recognize that any process or method disclosed herein can be modified in many ways. The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed.

The various exemplary methods described and/or illustrated herein may also omit one or more of the steps described or illustrated herein or comprise additional steps in addition to those disclosed. Further, a step of any method as disclosed herein can be combined with any one or more steps of any other method as disclosed herein.

The processor as described herein can be configured to perform one or more steps of any method disclosed herein. Alternatively or in combination, the processor can be configured to combine one or more steps of one or more methods as disclosed herein.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. Aspects of the invention are disclosed in the following numbered clauses:
1. A method of making a synthetic product comprising a synthetic DNA template suitable for *in vitro* transcription, the method comprising:
   transporting reagents to a first reactor in a microfluidic path device, wherein the reagents include a synthetic gene of interest, a polymerase, a buffer, a first primer having a first region specific to the synthetic gene of interest, and a second primer, wherein the second primer comprises a poly-T sequence of 150 base pairs (bp) or longer or a poly-A sequence of 150 bp or longer and a second region that is specific to the synthetic gene of interest;
   controlling a temperature of the first reactor of the microfluidic path device to perform a polymerase chain reaction (PCR) within the microfluidic path device to amplify the synthetic gene of interest using the first primer and the second primer to form a synthetic product including the poly-A sequence of 150 bp or longer; and
   transporting the synthetic product out of the first reactor, wherein the synthetic product comprises a synthetic DNA template suitable for *in vitro* transcription.
2. The method of clause 1, wherein the first primer includes an end that is complementary to or includes a sequence of a 3' end region of the synthetic gene of interest.
3. The method of clause 2, wherein the end that is complementary to or includes the sequence of a 3' end region of the synthetic gene of interest is complimentary to or includes between 20 and 40 bp of the synthetic gene of interest.
4. The method of any of clauses 1-3, wherein the second region of the second primer comprises an end region that includes or that is complimentary to a 5' end region of the synthetic gene of interest.
5. The method of clause 4, wherein the end region that includes or that is complimentary to the 5' end region of the synthetic gene of interest is between about 20 and about 40 bp long.
6. The method of any of clauses 1-5, wherein controlling the temperature to amplify the synthetic gene of interest by PCR comprises generating greater than 1 µM of an amplified DNA template.
7. The method of any of clauses 1-6, wherein the synthetic DNA template is free of bacterial DNA and free of endotoxin.
8. The method of clause 7, further comprising treating the synthetic gene of interest, the synthetic product, or both, with a methylation sensitive restriction enzyme to remove bacterial DNA.
9. The method of any of _{clauses} 1-8, wherein the first primer includes a promoter region.
10. The method of any of clauses 1-9, wherein controlling the temperature to amplify the synthetic gene of interest by PCR comprises amplifying for between 20 and 25 annealing and extension cycles.
11. The method of any of clauses 1-10, wherein transporting the first primer comprises transporting the first primer having a T7 promotor.
12. The method of any of clauses 1-11, wherein transporting the second primer comprises transporting the second primer comprising a poly-T sequence of 200 bp or longer or a poly-A sequence of 200 bp or longer.
13. The method of any of clauses 1-12, further comprising receiving, in a controller, optical sensor data from one or more sensors of the microfluidic path device, wherein the controller controls the operation of the microfluidic path device using at least the optical sensor data.
14. The method of any of clauses 1-13, further comprising purifying the synthetic product by one-dimensional (1D) or two-dimensional (2D) purification in the microfluidic path device.
15. The method of clause 14, wherein purifying comprises removing polynucleotides below a minimum length threshold.
16. The method of clause 15, wherein the minimum length threshold is below 500 bp.
17. The method of any of clauses 1-16, wherein transporting comprises using one or more fluid power circuits to move the reagents between a plurality of fluid depots into and the microfluidic path device or within the microfluidic path device.
18. The method of any of clause 1-16 further comprising performing an *in vitro* transcription using the synthetic DNA template to form a therapeutic polynucleotide.
19. The method of any of clauses 1-18, further comprising a determining yield of the synthetic product using a UV yield detection window on the microfluidic path device.
20. The method of clause 19, further comprising automatically diluting the synthetic product in the microfluidic path device based on the determined yield.
21. The method of any of clauses 1-20 wherein controlling the temperature comprises added additional enzyme during the polymerase chain reaction within the microfluidic path device.
22. A method of making a synthetic product comprising a DNA template, the method comprising:
   transporting reagents to a first reactor in a microfluidic path device, wherein the reagents include a synthetic gene of interest, a polymerase, a buffer, a forward primer including a 5' end that hybridizes to a first region of a polynucleotide that is complimentary to the synthetic gene of interest, and a reverse primer, wherein the reverse primer comprises a poly-T sequence of 150 bp or longer and a 5' region that is complimentary to a 5' end of the synthetic gene of interest;
   controlling a temperature of the first reactor of the microfluidic path device to perform a polymerase chain reaction (PCR) within the microfluidic path device to amplify the synthetic gene of interest using the forward primer and the reverse primer to form a synthetic product including a poly-A sequence of 150 bp or longer; and
   transporting the synthetic product out of the first reactor, wherein the synthetic product comprises the synthetic DNA template.
23. A method of making a product comprising a synthetic DNA template, the method comprising:
   transporting reagents to a first reactor in a microfluidic path device, wherein the reagents include a synthetic gene of interest, a polymerase, a buffer, nucleotides, a reverse primer including a 3' end that is complimentary to a first region of the synthetic gene of interest, and a forward primer, wherein the forward primer comprises a poly-A sequence of 150 bp or longer and a 3' region that that hybridizes to a second region of a polynucleotide that is complimentary to the synthetic gene of interest;
   controlling a temperature of the first reactor of the microfluidic path device to perform a polymerase chain reaction (PCR) within the microfluidic path device to amplify the synthetic gene of interest using the forward primer and the reverse primer to form a synthetic product including the poly-A sequence of 150 bp or longer; and
   transporting the synthetic product out of the first reactor, wherein the synthetic product comprises the synthetic DNA template.
24. A method of making a product comprising a synthetic DNA template, the method comprising:
   transporting reagents to a first reactor in a microfluidic path device, wherein the reagents include a synthetic gene of interest, a polymerase, a buffer, nucleotides, a first primer including an end that is complementary to or that includes a sequence of a 3' end region of the synthetic gene of interest, and a second primer, wherein the second primer comprises a poly-T sequence of 150 bp or longer, or a poly-A sequence of 150 bp or longer, and an end region that includes or that is complimentary to a 5' end region of the synthetic gene of interest;
   controlling a temperature of the first reactor of the microfluidic path device to thermocycle at least a first fluid reactor of the microfluidic path device to perform a polymerase chain reaction (PCR) within the microfluidic path device to amplify the synthetic gene of interest to generate 1 µM or more of synthetic product using the first primer and the second primer to form a synthetic product including a promoter region and a poly-A sequence of 150 bp or longer; and
   transporting the synthetic product to a second one or more reactors in the microfluidic path device, wherein the synthetic product comprises the synthetic DNA template.
25. A microfluidic path device for making a product comprising a synthetic DNA template, the microfluidic path device comprising:
   an elastic layer sandwiched between a first plate region having a first surface and a second plate region having a second surface;
   a plurality of PCR chambers each having a fixed volume connected by one or more channels, wherein each PCR chamber is formed between the first surface and the second surface, wherein a portion of the elastic layer divides each chamber into a fluid-contacting side in the second surface and a pressure-receiving side in the first surface, wherein the pressure receiving side is further partitioned by one or more fluidly-connected serpentine pathways;
   a plurality of fluid channels each extending from a fluid port through the first plate region and into the second plate region to fluidly connect with the fluid-contacting side of one or more of the plurality of chambers;
   a plurality of pressure channels each extending from one or more pressure ports, through the first plate region and elastic layer, into the second plate region, and back through the elastic layer and into the first plate region, wherein each pressure channel of the plurality of pressure channels extends within the first plate region and fluidly connects with one or more pressure-receiving sides of one or more of the plurality of chambers; and
   a UV yield detection chamber in fluid communication with one or more of the PCR chambers, wherein the UV yield detection chamber comprises a UV yield detection window configured to pass UV light therethrough for quantification of a polynucleotide within the UV yield detection chamber.
26. The microfluidic path device of clause 25, wherein the fluid-contacting side of each PCR chamber has a thickness of 1.5 cm or less.
27. The microfluidic path device of clause 25, further comprising a purification chamber in fluid communication with a purification substrate.
28. The microfluidic path device of clause 25, wherein the microfluidic path device is configured as a removable cartridge configured to engage with a fluid depot and pneumatic drive.
29. The microfluidic path device of clause 25, further comprising a vacuum cap, wherein the vacuum cap comprises a bubble-removing chamber formed between the first surface and the second surface, wherein a gas-permeable elastic layer divides the bubble-removing chamber into a fluid-contacting side of the bubble-removing chamber in the second surface and a vacuum receiving side in the first surface, further wherein the fluid-contacting side of the bubble-removing chamber is in fluid communication with the fluid-contacting sides of at least one of the PCR chambers.
30. The microfluidic path device of clause 25, wherein the fluid-contacting side in the second surface and the pressure-receiving side are concave and configured so that the elastic layer seats flush and without gaps to the fluid-contacting side in the second surface when a positive pressure in the pressure-receiving side drives the elastic layer against the fluid-contacting side.
31. The microfluidic path device of clause 25, further comprising the one or more pressure ports and fluid ports disposed adjacent to a periphery of the microfluidic path device on an upper surface of the first plate.
32. The microfluidic path device of clause 25, further comprising a material inserted into the fluid-contacting side of the channel.
33. The microfluidic path device of clause 32, wherein the material comprises a cellulose material configured to selectively absorb double-stranded mRNA.
34. The microfluidic path device of clause 25, wherein the first plate and the second plate are formed from a rigid material, wherein the rigid material is a polymer or glass.
35. The microfluidic path device of clause 34, wherein the polymer is cycloolefin copolymer.

## Claims

1. A method of making a synthetic product comprising a synthetic DNA template suitable for in vitro transcription, the method comprising:
transporting reagents to a first reactor in a closed-path microfluidic path device, wherein the reagents include a synthetic gene of interest, a polymerase, a buffer, a first primer having a first region specific to the synthetic gene of interest, and a second primer, wherein the second primer comprises a poly-T sequence of 150 base pairs (bp) or longer or a poly-A sequence of 150 bp or longer and a second region that is specific to the synthetic gene of interest, wherein the second primer is about four or more fold in length of the first primer;
controlling a temperature of the first reactor of the closed-path microfluidic path device to perform a polymerase chain reaction (PCR) within the microfluidic path device to amplify the synthetic gene of interest using the first primer and the second primer to form a synthetic product including the poly-A sequence of 150 bp or longer; and
transporting the synthetic product out of the first reactor of the closed-path microfluidic path device, wherein the synthetic product comprises a synthetic DNA template suitable for in vitro transcription.

2. The method of claim 1, wherein the second primer is about five or more fold, or about six or more fold, or about seven or more fold, or about eight or more fold, in length of the first primer.

3. The method of any of claims 1-2, wherein the first primer includes an end that is complementary to or includes a sequence of a 3' end region of the synthetic gene of interest, in particular wherein the end that is complementary to or includes the sequence of a 3' end region of the synthetic gene of interest is complimentary to or includes between 20 and 40 bp of the synthetic gene of interest.

4. The method of any of claims 1-3, wherein the second region of the second primer comprises an end region that includes or that is complimentary to a 5' end region of the synthetic gene of interest.

5. The method of claim 4, wherein the end region that includes or that is complimentary to the 5' end region of the synthetic gene of interest is between about 20 and about 40 bp long.

6. The method of any of claims 1-5, wherein controlling the temperature to amplify the synthetic gene of interest by PCR comprises generating greater than 1 µM of an amplified DNA template.

7. The method of any of claims 1-6, wherein the synthetic DNA template is free of bacterial DNA and free of endotoxin.

8. The method of any of claims 1-7, wherein the first primer includes a promoter region.

9. The method of any of claims 1-8, wherein controlling the temperature to amplify the synthetic gene of interest by PCR comprises amplifying for between 20 and 25 annealing and extension cycles.

10. The method of any of claims 1-9, wherein transporting the first primer comprises transporting the first primer having a T7 promotor.

11. The method of any of claims 1-10, wherein transporting the second primer comprises transporting the second primer comprising a poly-T sequence of 200 bp or longer or a poly-A sequence of 200 bp or longer.

12. The method of any of claims 1-11, further comprising receiving, in a controller, optical sensor data from one or more sensors of the closed-path microfluidic path device, wherein the controller controls the operation of the microfluidic path device using at least the optical sensor data.

13. The method of any of claim 1-12 further comprising performing an in vitro transcription reaction using the synthetic DNA template to form a therapeutic polynucleotide.

14. The method of any of claims 1-13, further comprising: determining yield of the synthetic product using a UV yield detection window on the closed-path microfluidic path device.

15. The method of any of claims 1-14 wherein controlling the temperature comprises added additional enzyme during the polymerase chain reaction within the closed-path microfluidic path device.
